# EUROPEAN PATENT APPLICATION

(11) **EP 4 706 687 A2**
(43) Date of publication of application: **11.03.2026**
(21) Application number: 26151115.8
(22) Date of filing: 27.06.2018
(51) Int. Cl.: A61K 48/00

(54) **TROPISM-MODIFIED RECOMBINANT VIRAL PARTICLES AND USES THEREOF FOR THE TARGETED INTRODUCTION OF GENETIC MATERIAL INTO HUMAN CELLS**

(30) Priority: 27.06.2017 US 201762525708 P
(62) Divisional of application: 23153644.2
(71) Applicant: Regeneron Pharmaceuticals, Inc., Tarrytown, NY 10591 (US)
(72) Inventor: SABIN, Leah, Tarrytown, 10591 (US); SCHOENHERR, Christopher, Tarrytown, 10591 (US); ECONOMIDES, Aris N., Tarrytown, 10591 (US); KYRATSOUS, Christos, Tarrytown, 10591 (US); MURPHY, Andrew J., Tarrytown, 10591 (US)
(74) Representative: Carpmaels & Ransford LLP

(57) **Abstract**

Provided herein are compositions and methods for redirecting recombinant viral capsid particles via a specific protein:protein binding pair that forms an covalent, e.g., isopeptide, bond to display a targeting ligand on the capsid protein, wherein the targeting ligand specifically binds a cell surface marker expressed on the cell of interest.

## Description

### REFERENCE TO A SEQUENCE LISTING

### SUBMITTED AS A TEXT FILE VIA EFS WEB

The Sequence Listing written in file 10359WO01_ST25.txt is 183 kilobytes, was created on June 27, 2018, and is hereby incorporated by reference.

### TECHNICAL FIELD

The disclosure herein relates generally to tropism modified recombinant viral particles, and compositions comprising same, useful for the targeted introduction of genetic material into cells.

### BACKGROUND OF THE INVENTION

The delivery of genes into particular target cells has become one of the most important technologies in modern medicine for the potential treatment of a variety of chronic and genetic diseases. So far, progress in the clinical application of gene therapy has been limited by the lack of ideal gene delivery vehicles. In order to achieve therapeutic success, gene delivery vehicles must be capable of transducing target cells while avoiding transduction of non-target cells. Specifically, when the native tropism of the virus does not meet the desired therapeutic target tissues or cell types, there is a need for recombinant viral particles wherein the natural tropism is ablated or diminished and the desired tropism is successfully engineered. (Buchholz et al.,)

In recent years, most progress in vector development has been achieved using non-enveloped viruses (e.g., viruses comprising a capsid formed by viral capsid proteins without an envelope (e.g., lipid bilayer)) such as adeno-associated viruses (AAVs) and adenoviruses (Ads), as well as enveloped viruses (e.g., viruses for which the capsid is surrounded by a lipid bilayer) such as retroviruses, lentiviruses, and herpes simplex virus. AAV based vectors have been the focus of much research, since AAVs are only mildly immunogenic and are capable of transducing a wide range of species and tissues *in vivo* with no evidence of toxicity.

AAVs are small, non-enveloped, single-stranded DNA viruses. The AAV genome is 4.7 kb and is characterized by two inverted terminal repeats (ITR) and two open reading frames which encode the Rep proteins and Cap proteins, respectively. The two ITRs are the only cis elements essential for AAV replication and encapsidation. The Rep reading frame encodes four proteins of molecular weight 78 kD, 68 kD, 52 kD and 40 kD. These proteins function mainly in regulating the transcription and replication of the AAV genome. The Cap reading frame encodes three structural (capsid) viral proteins (VPs) having molecular weights of 83-85 kD (VP 1), 72-73 kD (VP2) and 61-62 kD (VP3). More than 80% of total proteins in an AAV virion comprise VP3; in mature virions VP1, VP2 and VP3 are found at relative abundance of approximately 1:1:10. *In vitro,* the three proteins assemble spontaneously into virion-like structures, e.g., viral capsids. It appears, therefore, that viral capsid formation in infected cells proceeds independent of viral DNA synthesis (reviewed by Kotin et al. (1994) Hum. Gene Ther. 5:793).

Among all known AAV serotypes, AAV2 is perhaps the most well-characterized serotype, because its infectious clone was the first made. (Samulski et al. (1982) Proc. Natl. Acad. Sci. USA, 79:2077-2081). Subsequently, the full sequences for several AAV serotypes have also been determined. (see, e.g., Rutledge et al. (1998) J. Virol., 72:309-319; Gao et al. (2005) Curr. Gen. Ther. 5(3)285-97; Chiorini et al. (1997) J. Virol., 71:6823-6833; S. Muramatsu et al., (1996) Virol., 221:208-217). Generally, all AAVs share more than 80% identifying nucleotide sequence.

AAV is a promising vector for human gene therapy since, unlike other viral vectors, AAVs have not been shown to be associated with any known human disease and are generally not considered pathogenic. (Muzyczka, et al. (1992) Current Topics in Microbiology and Immunology, 158:97-129). Moreover, AAV safely transduces postmitotic tissues with relatively low immunogenicity, and although the virus can occasionally integrate into host chromosomes, it does so very infrequently into a safe-harbor locus in human chromosome 19, and only when the Rep proteins are supplied in trans. AAV genomes rapidly circularize and concatemerize in infected cells, and exist in a stable, episomal state in infected cells to provide long-term stable expression of their payloads.

A number of viruses, including AAVs, infect cells via a virus/ligand:cell/receptor interaction ultimately resulting in endocytosis of the virus by the infected cell. This ligand: receptor interaction is the focus of much of the research in viral vectors, and may be manipulated to redirect a virus' natural tropism from a cell naturally permissive to infection by the wildtype virus to a non-native target cell, e.g., via a receptor expressed by the target cell.

Theoretically, retargeting a vector toward any cell surface protein or marker should result in infection, since most cell surface receptors are involved in pathways of endocytosis, either constitutive (e.g., for recycling) or ligand induced (e.g., receptor-mediated). These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. As such, platforms for retargeting viral vectors often aim to ablate the natural tropism of the viral vector and redirect the viral vector to a receptor or marker expressed solely or primarily by the target cell. Many of the advances in targeted gene therapy using viral vectors may be summarized as non-recombinatorial (non-genetic) or recombinatorial (genetic) modification of the viral vector, which result in the pseudotyping, expanding, and/or retargeting of the natural tropism of the viral vector. (Reviewed in Nicklin and Baker (2002) Curr. Gene Ther. 2:273-93; Verheiji and Rottier (2012) Advances Virol 2012:1-15).

The most popular approach is a recombinatorial genetic modification of viral capsid proteins, and thus, the surface of the viral capsid. On the other hand, in indirect recombinatorial approaches, a viral capsid is modified with a heterologous "scaffold", which then links to an adaptor. The adaptor binds to both the scaffold and the target cell. In the direct recombinatorial targeting approach, a targeting ligand is directly inserted into, or coupled to, a viral capsid, i.e., protein viral capsids are modified to express a heterologous ligand. The ligand than redirects, e.g., binds, a receptor or marker preferentially or exclusively expressed on a target cell.

Each of the approaches has advantages and disadvantages. The ability to genetically modify the virus requires the structure of the capsid be maintained, and the targeting ligand or scaffold be placed in a position within the capsid protein that will tolerate and appropriately display the targeting ligand or scaffold. For example, the targeting ligand or scaffold introduced into the viral protein will have to meet size limitations as to not interfere with the structure of the modified capsid, which opens the possibility that the direct ligand or scaffold may not be presented correctly by the capsid and/or limits the spectrum of naturally existing molecules available for use as targeting ligands or scaffolds. Additionally, the use of targeting ligands inserted directly into the virus capsid is not modular and must be re-engineered for every target. Although the scaffold platform is advantageous in the flexibility and modular nature of the adaptor used, the scaffold on the virus particle and the adaptor interact ionically and remain two separate entities, and the inherent instability of their interactions may limit their utility *in vivo.* Optimal transduction efficiencies may be difficult to achieve with such two component systems.

Clearly, there remains a need for viral vector systems that maintain the integrity of the modified viral structure while remaining adaptable for the targeted transfer of nucleic acids of interest to a variety of target cells.

### SUMMARY OF THE INVENTION

Described herein is a viral retargeting strategy that solves the problems inherent in previous retargeting strategies by utilizing a first member and a second cognate member of a specific binding pair, which first member and second cognate member specifically interact to form a chemical, preferably covalent, bond. The first member, when displayed on a capsid protein, acts as a scaffold for any targeting ligand fused to the second cognate member, but upon binding of the first member and second cognate member, an isopeptide bond forms, and the recombinant viral particle acts as a one-component targeting vector.

Provided herein is a recombinant viral particle (e.g., a recombinant viral capsid protein, a recombinant viral capsid comprising the recombinant viral capsid protein, and/or a recombinant viral vector comprising a recombinant viral capsid that encapsulates a nucleotide of interest) that is genetically modified to display a heterologous amino acid sequence comprising a first member of a specific binding pair, e.g., a peptide tag, wherein the amino acid sequence is less than 50 amino acids in length, and wherein the recombinant viral capsid/particle protein exhibits reduced to abolished natural tropism. The tropism of a recombinant viral capsid protein/capsid/vector may be restored and/or redirected upon formation of an isopeptide bond with the cognate second member of the specific binding pair, which cognate second member is fused with a targeting ligand that specifically binds a target cell. Such bonding results in the recombinant viral capsid protein/capsid/vector displaying the targeting ligand. Transduction efficiencies and specificities are surprisingly enhanced when the targeting ligand is displayed by a recombinant viral capsid/vector via a linker and/or in limited amounts on the surface of the viral capsid. Such viral particles, compositions comprising same, and methods of making and using same are provided herein.

Accordingly, described herein is a recombinant viral capsid protein comprising a peptide tag operably linked (e.g., covalently linked) to the capsid protein, wherein the viral capsid protein is derived from a capsid gene of a virus that infects eukaryotic cells, and wherein the peptide tag is a first member of a specific binding pair that forms an isopeptide bond with a second cognate member of the specific binding pair. In some embodiments, a recombinant capsid protein (which may be derived from a capsid gene of a virus that infects eukaryotic cells, e.g., is a genetically modified capsid protein of a virus that infects eurkaryotic cells) as described herein comprises a first member of a specific binding pair (i.e., a peptide tag) operably linked to the capsid protein, and further comprises a second cognate member of the specific binding pair, wherein the first and second members of the specific binding pair are bound by a covalent (isopeptide) bond, e.g., the capsid protein comprises a first member operably linked to the capsid protein and further comprises a second cognate member of the specific binding pair covalently bound to the first member. In some embodiments, a recombinant capsid protein (which may be derived from a capsid gene of a virus that infects eurkaryotic cells, e.g., is a genetically modified capsid protein of a virus that infects eurkaryotic cells) as described herein comprises a first member of a specific binding pair (i.e., a peptide tag), operably linked to the capsid protein, and further comprises a second cognate member of the specific binding pair fused with a targeting ligand, wherein the first and second members of the specific binding pairs are bound by a covalent (isopeptide) bond, e.g., the capsid protein comprises a first member of a specific binding pair operably linked to the capsid protein and further comprises a second cognate member of the specific binding pair covalently bound to the first member, and wherein the second cognate member of the specific binding pair is operably linked to a targeting ligand that specifically binds a cell surface marker (e.g., a cell surface oligosaccharide, a cell surface receptor and/or cell surface marker, etc.) on a target cell. Also described herein are viral capsids comprising the recombinant viral capsid proteins, and viral vectors comprising a nucleotide of interest encapsulated by the viral capsids described herein. Also described are compositions comprising the recombinant viral particles described herein (e.g., the recombinant viral capsid proteins, recombinant viral capsids, and/or recombinant viral vectors), methods of using same for the targeted delivery of a nucleotide of interest, and methods of making same.

In some embodiments, the peptide tag (first member of a specific binding pair) is operably linked to (translated in frame with, chemically attached to, and/or displayed by) the capsid protein via a first or second linker, e.g., an amino acid spacer that is at least one amino acid in length. In some embodiments, the peptide tag (first member) is flanked by a first and/or second linker, e.g., a first and/or second amino acid spacer, each of which spacer is at least one amino acid in length.

In some embodiments, the first and/or second linkers are not identical. In some embodiments, the first and/or second linker is each independently one or two amino acids in length. In some embodiments, the first and/or second linker is each independently one, two or three amino acids in length. In some embodiments, the first and/or second linker is each independently one, two, three, or four amino acids in length. In some embodiments, the first and/or second linker is each independently one, two, three, four, or five amino acids in length. In some embodiments, the first and/or second linker are each independently one, two, three, four, or five amino acids in length. In some embodiments, the first and/or second linker is each independently one, two, three, four, five, or six amino acids in length. In some embodiments, the first and/or second linker is each independently one, two, three, four, five, six, or seven amino acids in length. In some embodiments, the first and/or second linker is each independently one, two, three, four, five, six, seven, or eight amino acids in length. In some embodiments, the first and/or second linker is each independently one, two, three, four, five, six, seven, eight or nine amino acids in length. In some embodiments, the first and or second linker is each independently one, two, three, four, five, six, seven, eight, nine, or ten amino acids in length. In some embodiments, the first and or second linker is each independently one, two, three, four, five, six, seven, eight, nine, ten, or more amino acids in length.

In some embodiments, the first and second linkers are identical in sequence and/or in length, and are each one amino acid in length. In some embodiments, the first and second linkers are identical in length, and are each one amino acid in length. In some embodiments, the first and second linkers are identical in length, and are each two amino acids in length. In some embodiments, the first and second linkers are identical in length, and are each three amino acids in length. In some embodiments, the first and second linkers are identical in length, and are each four amino acids in length, e.g., the linker is GLSG (SEQ ID NO:40). In some embodiments, the first and second linkers are identical in length, and are each five amino acids in length. In some embodiments, the first and second linkers are identical in length, and are each six amino acids in length, e.g., the first and second linkers each comprise a sequence of GLSGSG (SEQ ID NO:41). In some embodiments, the first and second linkers are identical in length, and are each seven amino acids in length. In some embodiments, the first and second linkers are identical in length, and are each eight amino acids in length, e.g., the first and second linkers each comprise a sequence of GLSGLSGS (SEQ ID NO:42). In some embodiments, the first and second linkers are identical in length, and are each nine amino acids in length. In some embodiments, the first and second linkers are identical in length, and are each ten amino acids in length, e.g., the first and second linkers each comprise a sequence of GLSGLSGLSG (SEQ ID NO:43) or GLSGGSGLSG (SEQ ID NO:44). In some embodiments, the first and second linkers are identical in length, and are each more than ten amino acids in length.

Generally, a peptide tag and optionally one or more linker as described herein, e.g., peptide tag by itself or in combination with one or more linkers, is between about 5 amino acids to about 50 amino acids in length. In some embodiments, the peptide tag by itself or in combination with one or more linkers is at least 5 amino acids in length. In some embodiments, the peptide tag by itself or in combination with one or more linkers is 6 amino acids in length. In some embodiments, the peptide tag by itself or in combination with one or more linkers is 7 amino acids in length. In some embodiments, peptide tag by itself or in combination with one or more linkers is 8 amino acids in length. In some embodiments, the peptide tag by itself or in combination with one or more linkers is 9 amino acids in length. In some embodiments, the peptide tag by itself or in combination with one or more linkers is 10 amino acids in length. In some embodiments, the peptide tag by itself or in combination with one or more linkers is 11 amino acids in length. In some embodiments, the peptide tag by itself or in combination with one or more linkers is 12 amino acids in length. In some embodiments, the peptide tag by itself or in combination with one or more linkers is 13 amino acids in length. In some embodiments, the peptide tag by itself or in combination with one or more linkers is 14 amino acids in length. In some embodiments, peptide tag by itself or in combination with one or more linkers is 15 amino acids in length. In some embodiments, the peptide tag by itself or in combination with one or more linkers is 16 amino acids in length. In some embodiments, the peptide tag by itself or in combination with one or more linkers is 17 amino acids in length. In some embodiments, the peptide tag by itself or in combination with one or more linkers is 18 amino acids in length. In some embodiments, the peptide tag by itself or in combination with one or more linkers is 19 amino acids in length. In some embodiments, the peptide tag by itself or in combination with one or more linkers is 20 amino acids in length. In some embodiments, the peptide tag by itself or in combination with one or more linkers is 21 amino acids in length. In some embodiments, the peptide tag by itself or in combination with one or more linkers is 22 amino acids in length. In some embodiments, the peptide tag by itself or in combination with one or more linkers is 23 amino acids in length. In some embodiments, the peptide tag by itself or in combination with one or more linkers is 24 amino acids in length. In some embodiments, the peptide tag by itself or in combination with one or more linkers is 25 amino acids in length. In some embodiments, the peptide tag by itself or in combination with one or more linkers is 26 amino acids in length. In some embodiments, peptide tag by itself or in combination with one or more linkers is 27 amino acids in length. In some embodiments, the peptide tag by itself or in combination with one or more linkers is 28 amino acids in length. In some embodiments, the peptide tag by itself or in combination with one or more linkers is 29 amino acids in length. In some embodiments, the peptide tag by itself or in combination with one or more linkers is 30 amino acids in length. In some embodiments, the peptide tag by itself or in combination with one or more linkers is 31 amino acids in length. In some embodiments, the peptide tag by itself or in combination with one or more linkers is 32 amino acids in length. In some embodiments, the peptide tag by itself or in combination with one or more linkers is 33 amino acids in length. In some embodiments, the peptide tag by itself or in combination with one or more linkers is 34 amino acids in length. In some embodiments, peptide tag by itself or in combination with one or more linkers is 35 amino acids in length. In some embodiments, the peptide tag by itself or in combination with one or more linkers is 36 amino acids in length. In some embodiments, the peptide tag by itself or in combination with one or more linkers is 37 amino acids in length. In some embodiments, peptide tag by itself or in combination with one or more linkers is 38 amino acids in length. In some embodiments, the peptide tag by itself or in combination with one or more linkers is 39 amino acids in length. In some embodiments, the peptide tag by itself or in combination with one or more linkers is 40 amino acids in length. In some embodiments, the peptide tag by itself or in combination with one or more linkers is more than 40 amino acids in length. In some embodiments, a peptide tag by itself or in combination with one or more linkers is no more than 50 amino acids in length.

Generally, the recombinant viral capsid proteins described herein may be derived from a capsid gene of a non-enveloped virus, e.g., is encoded by a *cap* gene modified to express a genetically modified capsid protein of a non-enveloped virus, wherein the non-enveloped virus infects human cells, or serotypes of non-enveloped viruses that generally infect human cells, e.g., adenovirus, adeno-associated virus, etc. In some embodiments, a recombinant viral capsid protein described herein is derived from an AAV capsid gene that encodes the VP1, VP2, and/or VP3 capsid proteins of the AAV (or portions of the VP1, VP2, and/or VP3 capsid proteins), e.g., is encoded by a *cap* gene modified to encode a genetically modified adeno-associated virus (AAV) VP1, VP2 and/or VP3 capsid protein, e.g., a genetically modified capsid protein of a AAV serotype that infects humans selected from the group consisting of AAV1, AAV2, AAV3, AAV4, AAV5, AAV6, AAV7, AAV8 and AAV9. In some embodiments, the recombinant viral capsid protein is derived from an AAV2 or AAV9 capsid gene that respectively encodes an AAV2 VP1, VP2, and/or VP3 capsid protein or AAV9 VP1, VP2, and/or VP3 capsid protein e.g., is encoded by an AAV2 or AAV9 *cap* gene modified to respectively encode a genetically modified AAV2 VP1, VP2 and/or VP3 capsid protein or a genetically modified AAV9 VP1, VP2 and/or VP3 capsid protein. In some embodiments, the recombinant viral capsid protein is a derived from an AAV6 capsid gene, e.g., is encoded by an AAV6 *cap* gene modified to encode a genetically modified AAV6 VP1, VP2, and/or VP3 capsid protein, the wildtype amino acid sequence of which AAV6 VP1 capsid protein is set forth respectively as SEQ ID NO:51. In some embodiments, the recombinant viral capsid protein is a derived from an AAV2 capsid gene, e.g., is encoded by an AAV2 *cap* gene modified to encode a genetically modified AAV2 VP1, VP2 and/or VP3 capsid protein, the wildtype amino acid sequence of which AAV2 VP1 capsid protein is set forth respectively as SEQ ID NO:9. In some embodiments the recombinant viral capsid protein is derived from an AAV9 capsid gene, e.g., is encoded by an AAV9 *cap* gene modified to encode a genetically modified AAV9 VP1, VP2, and/or VP3 capsid protein, the wildtype amino acid sequence of which AAV9 VP1 capsid protein is set forth respectively as SEQ ID NO:31.

In some embodiments, the recombinant viral capsid protein is derived from (encoded by) a chimeric AAV capsid gene, wherein the chimeric capsid gene comprises a plurality of nucleic acid sequences, wherein each of the plurality of nucleic acid sequences encodes a portion of a capsid protein of a different AAV serotype, and wherein the plurality of nucleic acid sequences together encodes a chimeric AAV capsid protein. In some embodiments, the recombinant viral capsid protein is derived from a chimeric AAV2 capsid gene. In some embodiments, the recombinant viral capsid protein is derived from a chimeric AAV6 capsid gene. In some embodiments, the recombinant viral capsid protein is derived from a chimeric AAV9 capsid gene.

Generally, a recombinant viral capsid protein as described herein is modified to comprise a peptide tag (first member of a protein:protein binding pair) operably linked (e.g., inserted into and/or displayed by), optionally via a linker, to the recombinant capsid protein such that the peptide tag (first member of a protein:protein binding pair) and optional linker itself reduces and/or abolishes the natural tropism of the recombinant capsid protein or capsid comprising same, as compared to a reference capsid protein lacking the peptide tag (first member of a protein:protein binding pair) and optional linker or capsid comprising the reference capsid capsid, respectively. In some embodiments, the peptide tag (first member of a protein:protein binding pair) is operably linked (e.g., inserted into and/or displayed by), optionally via a linker, to a region of the capsid protein involved with the natural tropism of the wildtype reference capsid protein, e.g., a region of the capsid protein involved with cell targeting. In some embodiments, the peptide tag (first member of a protein:protein binding pair) and optional linker is operably linked (e.g., inserted into and/or displayed by), optionally via a linker, to a knob domain of an Ad fiber protein. In some embodiments, the peptide tag (first member of a protein:protein binding pair) is operably linked (e.g., inserted into and/or displayed by), optionally via a linker, to the HI loop of an Ad fiber protein. In some embodiments, the peptide tag (first member of a protein:protein binding pair) is operably linked (e.g., inserted into and/or displayed by), optionally via a linker, to an exposed variable loop in an AAV capsid protein. In some embodiments, the peptide tag (first member of a protein:protein binding pair) is operably linked (e.g., inserted into and/or displayed by), optionally via a linker, to an exposed variable loop of an AAV2 capsid protein. In some embodiments, the peptide tag (first member of a protein:protein binding pair) is operably linked (e.g., inserted into and/or displayed by), optionally via a linker, to an exposed variable loop of an AAV9 capsid protein.

In some embodiments (i) the viral capsid protein is derived from an AAV2 capsid gene that encodes an AAV2 VP1, VP2, and/or VP3 capsid protein and the peptide tag is operably linked to (e.g., inserted into and/or displayed by), optionally via a linker, an amino acid at position I453 or I587 of the AAV2 VP1 capsid protein (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV3, AAV4, AAV5, AAV6, AAV7, AAV8, and AAV9); (ii) the viral capsid protein is derived from an AAV6 capsid gene and the peptide tag (first member of a protein:protein binding pair) is operably linked to (e.g., inserted into and/or displayed by), optionally via a linker, an amino acid at position I585 of the AAV6 VP1 capsid protein (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV7, AAV8, and AAV9); or (iii) the viral capsid protein is derived from an AAV9 capsid gene that encodes an AAV9 VP1, VP2 and/or VP3 capsid protein and the peptide tag is operably linked to (e.g., inserted into and/or displayed by), optionally via a linker, an amino acid at position I453 or I589 AAV9 VP1 capsid (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7 and AAV8).

In some embodiments, the peptide tag (first member of a protein:protein binding pair) is operably linked, optionally via a linker, to an amino acid a position selected from the group consisting of 453 of AAV2 capsid protein VP1, 587 of AAV2 capsid protein VP1, 585 of AAV6 capsid protein VP1, 453 of AAV9 capsid protein VP1, and 589 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, AAV8, and AAV9), e.g., is fused to the C-terminus of an amino acid at a position selected from the group consisting of 453 of AAV2 capsid protein VP1, 587 of AAV2 capsid protein VP1, 585 of AAV6 capsid protein VP1, 453 of AAV9 capsid protein VP1, and 589 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, AAV8, and AAV9). In some embodiments, the peptide tag (first member of a protein:protein binding pair) and optional linker is inserted immediately after (e.g., is fused to the C-terminus of) an amino acid at position 453 of AAV2 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV3, AAV4, AAV5, AAV6, AAV7, AAV8, and AAV9). In some embodiments, the peptide tag (first member of a protein:protein binding pair) and optional linker is inserted immediately after (e.g., is fused to the C-terminus of) an amino acid at position 587 of AAV2 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV3, AAV4, AAV5, AAV6, AAV7, AAV8, and AAV9). In some embodiments, the peptide tag (first member of a protein:protein binding pair) and optional linker is inserted immediately after (e.g., is fused to the C-terminus of) an amino acid at position 585 of AAV6 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV7, AAV8, and AAV9). In some embodiments, the peptide tag (first member of a protein:protein binding pair) and optional linker is inserted immediately after (e.g., is fused to the C-terminus of) an amino acid at position 453 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV8). In some embodiments, the peptide tag (first member of a protein:protein binding pair) and optional linker is inserted immediately after (e.g., is fused to the C-terminus of) an amino acid at position 589 of AAV9 capsid protein VP1 (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2 AAV3, AAV4, AAV5, AAV6, AAV7, and AAV8). In some embodiments, the peptide tag (first member of a protein:protein binding pair) and optional linker is inserted and/or displayed between positions 587 and 588 of an AAV2 VP1 capsid protein (or corresponding positions of the VP2 and/or VP3 capsid proteins encoded from the same capsid gene, or the corresponding amino acids of VP1, VP2, and/or VP3 capsid proteins of a different AAV that infects humans, e.g., AAV1, AAV2, AAV3, AAV4, AAV5, AAV6, AAV7, and AAV8).

In some embodiments, a recombinant capsid protein as described herein comprises a (second and different) mutation, which may be in addition to the peptide tag (first member of a protein:protein binding pair) and optional linker. In some embodiments, the (second and different mutation) comprises an insertion of a heterologous peptide into the capsid protein, substitution of one or more amino acids of the capsid protein with one or more heterologous amino acids, deletion of one or more amino acids of the capsid protein, or a combination thereof. For example, in some embodiment, a recombinant viral capsid protein as described herein may be derived from an AAV2 capsid gene (e.g., is a genetically modified AAV2 VP1, VP2 and /or VP3 capsid protein), comprises a peptide tag (first member of a protein:protein binding pair) and optional linker, and may further comprise a mutation, e.g., a R585A and/or R588A mutation in the AAV2 VP1 capsid protein (or corresponding mutation in the VP2 and/or VP3 capsid proteins encoded from the same AAV2 capsid gene). In some embodiments, a recombinant viral capsid protein is derived from a AAV2 capsid gene, e.g., is a genetically modified AAV2 VP1, VP2 and/or VP3 capsid protein, comprises a peptide tag (first member of a protein:protein binding pair) and optional linker inserted immediately after (e.g., fused to the C-terminus of) an amino acid at position 453 of the AAV2 VP1 protein (or amino acids at corresponding positions of the AAV2 VP2 and/or VP3 capsid proteins encoded by the AAV2 capsid gene), and further comprises a mutation selected from the group consisting of R585A and/or R588A (or corresponding mutations in the VP2 and/or VP3 capsid proteins encoded from the same AAV2 capsid gene). In some embodiments, a recombinant viral capsid protein is derived from an AAV2 capsid gene, e.g., is a genetically modified AAV2 VP1, VP2 and/or VP3 capsid protein, comprises a peptide tag (first member of a protein:protein binding pair) and optional linker inserted immediately after (e.g., fused to the C-terminus of) an amino acid at position 587 of the AAV2 VP1 capsid protein (or amino acids at corresponding positions of the AAV2 VP2 and/or VP3 capsid proteins encoded from the same AAV2 capsid gene), and further comprises a mutation selected from the group consisting of R585A, R588A and/or corresponding mutations in the VP2 and/or VP3 capsid proteins encoded from the same AAV2 capsid gene.

In some embodiments, a recombinant viral capsid protein is derived from an AAV9 capsid gene, e.g., is a genetically modified AAV9 VP1, VP2, and/or VP3 capsid protein, comprises a peptide tag (first member of a protein:protein binding pair) and optional linker inserted immediately after (e.g., fused to the C-terminus of) an amino acid at position 453 of the AAV9 VP1 protein (or the amino acid at corresponding positions of the AAV9 VP2 or VP3 capsid proteins encoded from the same AAV9 capsid gene), and further comprises a W503A mutation (or a corresponding mutation in the VP2 and/or VP3 capsid proteins encoded from the same AAV2 capsid gene). In some embodiments, a recombinant viral capsid protein is derived from an AAV9 capsid gene, e.g., is a genetically modified AAV9 VP1, VP2, and/or VP3 capsid protein, comprises a peptide tag (first member of a protein:protein binding pair) and optional linker inserted immediately after (e.g., fused to the C-terminus of) an amino acid at position 589 of the AAV9 VP1 protein (or the amino acids at corresponding positions of the AAV9 VP2 and/or VP3 capsid proteins encoded from the same AAV9 capsid gene), and further comprises a W503A mutation (or a corresponding mutation in the VP2 and/or VP3 capsid proteins encoded from the same AAV2 capsid gene).

In some embodiments, the protein:protein binding pair may be selected from the group consisting of SpyTag:SpyCatcher, SpyTag002:SpyCatcher002, SpyTag:KTag, Isopeptag:pilin-C, and SnoopTag:SnoopCatcher. In some embodiments, wherein the peptide tag (first member) is SpyTag (or a biologically active portion thereof) and the protein (second cognate member) is SpyCatcher (or a biologically active portion thereof). In some embodiments, wherein the peptide tag (first member) is SpyTag (or a biologically active portion thereof) and the protein (second cognate member) is KTag (or a biologically active portion thereof). In some embodiments, wherein the peptide tag (first member) is KTag (or a biologically active portion thereof) and the protein (second cognate member) is SpyTag (or a biologically active portion thereof). In some embodiments, wherein the peptide tag (first member) is SnoopTag (or a biologically active portion thereof) and the protein (second cognate member) is SnoopCatcher (or a biologically active portion thereof). In some embodiments, wherein the peptide tag (first member) is Isopeptag (or a biologically active portion thereof) and the protein (second cognate member) is Pilin-C (or a biologically active portion thereof). In some embodiments, wherein the peptide tag (first member) is SpyTag002 (or a biologically active portion thereof) and the protein (second cognate member) is SpyCatcher002 (or a biologically active portion thereof).

In some embodiments, a recombinant viral capsid protein comprises a SpyTag. In some embodiments, a recombinant viral capsid, a recombinant viral vector comprising a recombinant viral capsid, and/or compositions comprising a recombinant viral capsid or viral vector comprises an amino acid sequence set forth as any SEQ ID NO listed in Table 1 as an amino acid sequence of a recombinant viral capsid protein. In some embodiments, a recombinant viral capsid, viral vector comprising a recombinant viral capsid, and/or compositions comprising a recombinant viral capsid comprises an amino acid sequence set forth as SEQ ID NO: 13. In some embodiments, a recombinant viral capsid, viral vector comprising a recombinant viral capsid, and/or compositions comprising a recombinant viral capsid comprises an amino acid sequence set forth as SEQ ID NO:15. In some embodiments, a recombinant viral capsid, viral vector comprising a recombinant viral capsid, and/or compositions comprising a recombinant viral capsid comprises an amino acid sequence set forth as SEQ ID NO: 17. In some embodiments, a recombinant viral capsid, viral vector comprising a recombinant viral capsid, and/or compositions comprising a recombinant viral capsid comprises an amino acid sequence set forth as SEQ ID NO: 19. In some embodiments, a recombinant viral capsid, viral vector comprising a recombinant viral capsid, and/or compositions comprising a recombinant viral capsid comprises an amino acid sequence set forth as SEQ ID NO: 21. In some embodiments, a recombinant viral capsid, viral vector comprising a recombinant viral capsid, and/or compositions comprising a recombinant viral capsid comprises an amino acid sequence set forth as SEQ ID NO:23. In some embodiments, a recombinant viral capsid, viral vector comprising a recombinant viral capsid, and/or compositions comprising a recombinant viral capsid comprises an amino acid sequence set forth as SEQ ID NO:25. In some embodiments, a recombinant viral capsid, viral vector comprising a recombinant viral capsid, and/or compositions comprising a recombinant viral capsid comprises an amino acid sequence set forth as SEQ ID NO:27. In some embodiments, a recombinant viral capsid, viral vector comprising a recombinant viral capsid, and/or compositions comprising a recombinant viral capsid comprises an amino acid sequence set forth as SEQ ID NO:29. In some embodiments, a recombinant viral capsid, viral vector comprising a recombinant viral capsid, and/or compositions comprising a recombinant viral capsid comprises an amino acid sequence set forth as SEQ ID NO:35. In some embodiments, a recombinant viral capsid, viral vector comprising a recombinant viral capsid, and/or compositions comprising a recombinant viral capsid comprises an amino acid sequence set forth as SEQ ID NO: 37. In some embodiments, a recombinant viral capsid, viral vector comprising a recombinant viral capsid, and/or compositions comprising a recombinant viral capsid comprises an amino acid sequence set forth as SEQ ID NO:39.

In some embodiments a recombinant viral capsid protein described herein comprises a first member of a specific binding pair (e.g., a peptide tag) covalently bound to a second cognate protein member of the specific binding pair. In some embodiments a recombinant viral capsid protein described herein comprises a peptide tag (first member) covalently bound to an adaptor polypeptide comprising a cognate protein (second member) operably linked to a targeting ligand. In some embodiments, the targeting ligand is operably linked to the protein (second member), e.g., fused to the protein, optionally via a linker. Generally, a targeting ligand may be a binding moiety, e.g., a natural ligand, antibody, a multispecific binding molecule, etc. In some embodiments, the targeting ligand is an antibody or portion thereof. In some embodiments, the targeting ligand is an antibody comprising a variable domain that binds a cell surface protein on a target cell and a heavy chain constant domain. In some embodiments, the targeting ligand is an antibody comprising a variable domain that binds a cell surface protein on a target cell and an IgG heavy chain constant domain. In some embodiments, the targeting ligand is an antibody comprising a variable domain that binds a cell surface protein on a target cell and an IgG heavy chain constant domain, wherein the IgG heavy chain constant domain is operably linked, e.g., via a linker, to a protein (e.g., second member of a protein:protein binding pair) that forms an isopeptide covalent bond with a peptide tag. In some embodiments, a recombinant capsid protein described herein comprises a SpyTag operably linked to the viral capsid protein, and covalently linked to the SpyTag, an adaptor polypeptide comprising SpyCatcher linked to a targeting ligand comprising an antibody variable domain and an IgG heavy chain domain, wherein SpyCatcher and the IgG heavy chain domain are linked via an amino acid linker, e.g., GSGESG (SEQ ID NO:48). In some embodiments, the adaptor polypeptide comprises the sequence set forth as SEQ ID NO:46, which comprises a portion of a human IgG4 heavy chain, said IgG4 portion having a sequence set forth as SEQ ID NO:49, linked via linker (SEQ ID NO:48) to SpyCatcher (SEQ ID NO:3).

In some embodiments, a recombinant viral capsid, viral vector comprising a recombinant viral capsid, and/or compositions comprising a recombinant viral capsid comprises SpyTag covalently linked to a SpyCatcher fused to a targeting ligand. In some embodiments, a recombinant viral capsid, viral vector comprising a recombinant viral capsid, and/or compositions comprising a recombinant viral capsid comprises an amino acid sequence set forth as any SEQ ID NO listed in Table 1 as encoding a recombinant viral capsid protein and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises a recombinant viral capsid comprises an amino acid sequence set forth as SEQ ID NO:13 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO: 15 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:17 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:19 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO: 21 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:23 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:25 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:27 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:29 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:35 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO: 37 and a polypeptide adaptor comprising an amino acid sequence set forth as SEQ ID NO:46. In some embodiments, a recombinant viral capsid, viral vector comprising a recombinant viral capsid, and/or compositions comprising a recombinant viral capsid comprises an amino acid sequence set forth as SEQ ID NO:39 and a polypeptide adaptor comprising an amino acid sequence set forth as SEQ ID NO:46.

Generally, a targeting ligand specifically binds a cell surface molecule, e.g., an oligosaccharide, a receptor, cell surface marker, etc., expressed on the surface of a mammalian (e.g., human) eukaryotic cell, e.g., a target cell. In some embodiments, a targeting ligand binds a (human) liver cell, a (human) brain cell, a (human) T cell, a (human) kidney cell, a (human) intestinal cell, a (human) lung cell, a (human) cancerous cell, or a (human) cell infected with heterologous pathogen.

In some embodiments, the targeting ligand binds a receptor expressed by a (human) liver cell, e.g., an asialoglycoprotein receptor, e.g., hASGR1. In some embodiments, the targeting ligand binds a molecule expressed by a (human) neuronal cell, e.g., GABA, transferrin, etc. In some embodiments, the targeting ligand binds a molecule expressed by a (human) T cell, e.g., CD3, e.g., CD3ε. In some embodiments, the targeting ligand binds CD63. In some embodiments, the targeting ligand binds a molecule expressed by a (human) hematopoietic stem cell, e.g, CD34. In some embodiments, the targeting ligand binds a molecule expressed by a (human) kidney cell. In some embodiments, the targeting ligand binds a molecule expressed by a (human) muscle cell, e.g., an integrin. In some embodiments, the r targeting ligand binds a molecule expressed by a (human) cancerous cell, e.g., a tumor associated antigen, e.g., adipophilin, AIM-2, ALDH1A1, alpha-actinin-4, alpha-fetoprotein ("AFP"), ARTC1, B-RAF, BAGE-1, BCLX (L), BCR-ABL fusion protein b3a2, beta-catenin, BING-4, CA-125, CALCA, carcinoembryonic antigen ("CEA"), CASP-5, CASP-8, CD274, CD45, Cdc27, CDK12, CDK4, CDKN2A, CEA, CLPP, COA-1, CPSF, CSNK1A1, CTAG1, CTAG2, cyclin D1, Cyclin-A1, dek-can fusion protein, DKK1, EFTUD2, Elongation factor 2, ENAH (hMena), Ep-CAM, EpCAM, EphA3, epithelial tumor antigen ("ETA"), ETV6-AML1 fusion protein, EZH2, E6, E7, FGF5, FLT3-ITD, FN1, G250/MN/CAIX, GAGE-1,2,8, GAGE-3,4,5,6,7, GAS7, glypican-3, GnTV, gp100/Pme117, GPNMB, HAUS3, Hepsin, HER-2/neu, HERV-K-MEL, HLA-A11, HLA-A2, HLA-DOB, hsp70-2, IDO1, IGF2B3, IL13Ralpha2, Intestinal carboxyl esterase, K-ras, Kallikrein 4, KIF20A, KK-LC-1, KKLC1, KM-HN-1, KMHN1 also known as CCDC110, LAGE-1, LDLR-fucosyltransferaseAS fusion protein, Lengsin, M-CSF, MAGE-A1, MAGE-A10, MAGE-A12, MAGE-A2, MAGE-A3, MAGE-A4, MAGE-A6, MAGE-A9, MAGE-C1, MAGE-C2, malic enzyme, mammaglobin-A, MART2, MATN, MC1R, MCSP, mdm-2, ME1, Melan-A/MART-1, Meloe, Midkine, MMP-2, MMP-7, MUC1, MUC5AC, mucin, MUM-1, MUM-2, MUM-3, Myosin, Myosin class I, N-raw, NA88-A, neo-PAP, NFYC, NY-BR-1, NY-ESO-1/LAGE-2, OA1, OGT, OS-9, P polypeptide, p53, PAP, PAX5, PBF, pml-RARalpha fusion protein, polymorphic epithelial mucin ("PEM"), PPP1R3B, PRAME, PRDX5, PSA, PSMA, PTPRK, RAB38/NY-MEL-1, RAGE-1, RBAF600, RGS5, RhoC, RNF43, RU2AS, SAGE, secernin 1, SIRT2, SNRPD1, SOX10, Sp17, SPA17, SSX-2, SSX-4, STEAP1, survivin, SYT-SSX1 or -SSX2 fusion protein, TAG-1, TAG-2, Telomerase, TGF-betaRII, TPBG, TRAG-3, Triosephosphate isomerase, TRP-1/gp75, TRP-2, TRP2-INT2, tyrosinase, tyrosinase ("TYR"), VEGF, WT1, XAGE-lb/GAGED2a, Kras, NY-ESO1, MAGE-A3, HPV E2, HPV E6, HPV E7, WT-1 antigen (in lymphoma and other solid tumors), ErbB receptors, Melan A [MART1], gp 100, tyrosinase, TRP-1/gp 75, and TRP-2 (in melanoma); MAGE-1 and MAGE-3 (in bladder, head and neck, and non-small cell carcinoma); HPV EG and E7 proteins (in cervical cancer); Mucin [MUC-1] (in breast, pancreas, colon, and prostate cancers); prostate-specific antigen [PSA] (in prostate cancer); carcinoembryonic antigen [CEA] (in colon, breast, and gastrointestinal cancers), and such shared tumor-specific antigens as MAGE-2, MAGE-4, MAGE-6, MAGE-10, MAGE-12, BAGE-1, CAGE-1,2,8, CAGE-3 TO 7, LAGE-1, NY-ESO-1/LAGE-2, NA-88, GnTV, TRP2-INT2, etc. In some embodiments, the targeting ligand binds E6 and/or E7. In some embodiments, the targeting ligand binds Her2. In some embodiments, the targeting ligand binds CD63. In some embodiments, the targeting ligand binds human glucagon receptor (hGCGR). In some embodiments, the retargeting ligand binds human ectonucleoside triphosphate diphosphohydrolase 3 (hENTPD3).

Generally, a viral capsid comprising the recombinant viral capsid protein described herein is unable to infect a target cell in the absence of a targeting ligand, e.g., the second member operably linked to a targeting ligand. Generally, in the absence of an appropriate targeting ligand, a viral capsid comprising a recombinant viral capsid protein as described herein has reduced to abolished natural tropism, e.g., has a reduced capacity or is unable to target and bind a reference cell naturally permissive to transduction compared to that of a reference viral capsid, e.g., a capsid comprising a reference viral capsid protein, e.g., a wild-type control viral capsid protein or a viral capsid protein that would be identical to the recombinant viral capsid protein but for the lack of targeting ligand, and optionally either or both members of the protein:protein binding pair. In some embodiments, the transduction efficiency of a recombinant viral capsid protein comprising SpyTag is reduced or abolished compared to a control wildtype viral capsid protein.

In some embodiments and in the absence of an appropriate targeting ligand, a viral capsid comprising a recombinant viral capsid protein as described here, e.g., listed in Table 1, exhibits at least 10% decrease in transduction efficiency compared to an appropriate control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 20% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 30% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 40% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 50% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 60% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 70% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 75% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 80% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 85% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 90% decrease in transduction efficiency compared to a control wildtype viral capsid, e.g., listed in Table 1. In some embodiments and in the absence of an appropriate targeting ligand, a viral capsid comprising a recombinant viral capsid protein as described herein, e.g., listed in Table 1, exhibits at least 95% decrease in transduction efficiency compared to a control wildtype viral capsid, e.g., listed in Table 1. In some embodiments and in the absence of an appropriate targeting ligand, viral capsid comprising a recombinant viral capsid protein as described herein, e.g., listed in Table 1, exhibits at least 99% decrease in transduction efficiency compared to a control wildtype viral capsid, e.g., listed in Table 1. In some embodiments and in the absence of an appropriate targeting ligand, transduction of a control cell by a viral capsid comprising a recombinant viral capsid protein as described herein is abolished, e.g., undetectable, e.g., via methods measuring expression of the nucleotide of interest, e.g., reporter assays, etc.

Conversely, a viral capsid comprising the recombinant viral capsid protein comprising a peptide tag covalently linked to an appropriate adaptor polypeptide, e.g., a cognate protein operably linked to a targeting ligand, is able to infect a target cell, e.g., has a partially or completely restored capacity to target and bind a reference cell naturally permissive to transduction compared to that of a reference viral capsid, e.g., a capsid comprising a reference viral capsid protein, e.g., a wild-type control viral capsid protein. In some embodiments, a viral capsid comprising a recombinant viral capsid protein as described here, e.g., listed in Table 1, covalently bound to an appropriate adaptor polypeptide exhibits a transduction efficiency that is at least 10% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 20% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 30% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 40% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 50% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 60% the transduction efficiency of an appropriate control wildtype viral capsid, e.g., listed in Table 1 In some embodiments, a viral capsid comprising a recombinant viral capsid protein as described here, e.g., listed in Table 1, covalently bound to an appropriate adaptor polypeptide exhibits a transduction efficiency that is at least 70% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 75% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 80% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 85% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 90% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 95% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is at least 99% the transduction efficiency of an appropriate control wildtype viral capsid, e.g., listed in Table 1. In some embodiments, a viral capsid comprising a recombinant viral capsid protein as described here, e.g., listed in Table 1, covalently bound to an appropriate adaptor polypeptide exhibits an identical transduction efficiency to that of an appropriate control wildtype viral capsid, e.g., listed in Table 1.

Similarly, a viral capsid comprising the recombinant viral capsid protein comprising a peptide tag covalently linked to an appropriate adaptor polypeptide, e.g., a cognate protein operably linked to a targeting ligand, is able to infect a target cell, e.g., has an enhanced capacity to target and bind a reference cell naturally permissive to transduction compared to that of a reference viral capsid that is identical to the recombinant viral capsid protein except that it lacks either or both members of the protein:protein binding pair, e.g., comprises a reference capsid protein. In some embodiments, a viral capsid comprising a recombinant viral capsid protein as described here, e.g., listed in Table 1, covalently bound to an appropriate adaptor polypeptide exhibits a transduction efficiency that is 10% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 20% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 30% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 40% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 50% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 60% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 70% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 75% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 80% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 85% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 90% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 95% greater than the transduction efficiency of an appropriate control reference viral capsid, e.g., listed in Table 1. In some embodiments, a viral capsid comprising a recombinant viral capsid protein as described here, e.g., listed in Table 1, covalently bound to an appropriate adaptor polypeptide exhibits a transduction efficiency that is 99% greater than the transduction efficiency of an appropriate control reference viral capsid, e.g., listed in Table 1.

In some embodiments a viral capsid comprising a recombinant viral capsid protein as described herein is a mosaic capsid, e.g., comprises at least two sets of VP1, VP2, and/or VP3 proteins, each set of which is encoded by a different *cap* gene, e.g., comprises a recombinant viral capsid protein comprising a peptide tag and a reference capsid protein that does not comprise the peptide tag at a certain ratio. In some embodiments, a reference capsid protein is a wildtype reference capsid protein in that it comprises an amino acid sequence of a wildtype capsid protein having the same serotype as the recombinant viral capsid protein. In some embodiments, a reference capsid protein is a control reference capsid protein in that it comprises an amino acid sequence of the recombinant viral capsid protein except that the control reference capsid protein lacks the peptide tag. In some embodiments, a reference capsid protein is a mutated wildtype reference protein in that it comprises an amino acid sequence substantially identical to that of a wildtype capsid protein having the same serotype as the recombinant viral capsid protein but for a mutation, (e.g., a deletion of an amino acid sequence, an insertion of an amino acid sequence, chimerization, etc.) that reduces the tropism of the wildtype capsid protein. In some embodiments, a composition described herein comprises, or a method described herein combines, a recombinant viral capsid protein and a reference capsid protein at a ratio that ranges from 1:1 to 1:15. In some embodiments, the ratio is 1:2. In some embodiments, the ratio is 1:3. In some embodiments, the ratio is 1:4. In some embodiments, the ratio is 1:5. In some embodiments, the ratio is 1:6. In some embodiments, the ratio is 1:7. In some embodiments, the ratio is 1:8. In some embodiments, the ratio is 1:9. In some embodiments, the ratio is 1:10. In some embodiments, the ratio is 1:11. In some embodiments, the ratio is 1:12. In some embodiments, the ratio is 1:13. In some embodiments, the ratio is 1:14. In some embodiments, the ratio is 1:15.

In some embodiments, a composition described herein comprises, or a method described herein combines a recombinant viral capsid protein listed in Table 1 and its appropriate reference capsid protein (or a combination of its reference capsid proteins), also listed in Table 1, at a ratio (recombinant capsid protein:reference capsid protein(s)) that ranges from 1:1 to 1:15. In some embodiments, the ratio is 1:2. In some embodiments, the ratio is 1:3. In some embodiments, the ratio is 1:4. In some embodiments, the ratio is 1:5. In some embodiments, the ratio is 1:6. In some embodiments, the ratio is 1:7. In some embodiments, the ratio is 1:8. In some embodiments, the ratio is 1:9. In some embodiments, the ratio is 1:10. In some embodiments, the ratio is 1:11. In some embodiments, the ratio is 1:12. In some embodiments, the ratio is 1:13. In some embodiments, the ratio is 1:14. In some embodiments, the ratio is 1:15.

Table 1 provides the sequence identification numbers (SEQ ID NOs) setting forth the amino acid sequences of (1) exemplary and nonlimiting recombinant viral capsid proteins comprising a peptide tag described herein, (2) exemplary and nonlimiting examples corresponding control (C) wildtype viral capsid proteins that may optionally be used as a reference for determining reduction in or abolished transduction efficiency of a recombinant capsid protein comprising a covalent protein tag in the absence of a targeting vector, and (3) exemplary and non-limiting examples of corresponding reference viral capsid proteins for producing mosaic capsids and/or use as a reference for determining restoration of transduction efficiencies of a recombinant capsid protein comprising a protein:protein binding pair and targeting ligand.

**Table 1**

| Recombinant viral capsid protein comprising SpyTag (SEQ ID NO:) | Reference viral capsid protein: |
|---|---|
| | Wildtype (W) reference capsid protein (SEQ ID NO) |
| | Control (C) reference capsid protein(s) (SEQ ID NO) |
| | Mutated (M) reference capsid protein(s) |
| AAV2-CAP N587 SpyTag HBM (SEQ ID NO:13) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP N587 Linker 1 SpyTag HBM (SEQ ID NO:15) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP N587 Linker 2 SpyTag HBM (SEQ ID NO:17) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP N587 Linker 4 SpyTag HBM (SEQ ID NO:19) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP N587 Linker 6 SpyTag HBM (SEQ ID NO:21) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP N587 Linker 8 SpyTag HBM (SEQ ID NO:23) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP N587 Linker 10 SpyTag HBM (SEQ ID NO:25) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP G453 SpyTag HBM (SEQ ID NO:27) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV2-CAP G453 Linker10 SpyTag HBM (SEQ ID NO:29) | (W) AAV2-CAP (SEQ ID NO:9) |
| | (C) AAV2-CAP R585A R588A HBM (SEQ ID NO:11) |
| | (M) AAV2-CAP N587 Myc (SEQ ID NO:53) |
| AAV9-CAP A589 SpyTag W503A (SEQ ID NO:35) | (W) AAV9-CAP wt (SEQ ID NO:31) |
| | (C) AAV9-CAP W503A (SEQ ID NO:33) |
| AAV9-CAP A589 Linker10 SpyTag W503A (SEQ ID NO:37) | (W) AAV9-CAP wt (SEQ ID NO:31) |
| | (C) AAV9-CAP W503A (SEQ ID NO:33) |
| AAV9-CAP G453 Linker10 SpyTag W503A (SEQ ID NO:39) | (W) AAV9-CAP wt (SEQ ID NO:31) |
| | (C) AAV9-CAP W503A (SEQ ID NO:33) |

Generally, recombinant viral vectors as described herein comprise a viral capsid comprising a recombinant viral capsid protein as described herein, including mosaic viral capsids, wherein the viral capsid encapsulates a nucleotide of interest. In some embodiments, the nucleotide of interest is under the control of a promoter selected from the group consisting of a viral promoter, a bacterial promoter, a mammalian promoter, an avian promoter, a fish promoter, an insect promoter, and any combination thereof. In some embodiments, the nucleotide of interest is under the control of a non-human promoter. In some embodiments, the promoter is a cytomegalovirus (CMV) promoter. In some embodiments, the promoter is an EF1α promoter. In some embodiments, the promoter is a CAGG promoter. In some embodiments, the promoter is a Ubiquitin C (UbC) promoter.

Generally, a nucleotide of interest may be one or more genes, which may encode a detectable marker, e.g., reporter, or a therapeutic polypeptide. In some embodiments, the nucleotide of interest is a reporter gene. In some embodiments, the nucleotide of interest is a reporter gene that encodes a detectable marker selected from the group consisting of green fluorescent protein, luciferase, β-galactosidase, etc. In some embodiments, the detectable marker is green fluorescent protein. In other embodiments, the nucleotide of interest is selected from the group consisting of a suicide gene, a nucleotide encoding an antibody or fragment thereof, a nucleotide encoding a CRISPR/Cas system or portion(s) thereof, a nucleotide encoding antisense RNA, a nucleotide encoding siRNA, a secreted enzyme, a gene encoding a therapeutic protein, etc. In one embodiment, the nucleotide of interest encodes a multidomain therapeutic, e.g., a protein that comprises at least two domains providing two distinct functions.

Compositions described herein generally comprise a viral vector that comprises a recombinant viral capsid protein as described herein, e.g., comprises a capsid comprising the recombinant viral capsid protein (including a mosaic capsid), wherein the capsid encapsulates a nucleotide of interest. In some embodiments, a composition described herein comprises (1) a viral vector having a capsid comprising a recombinant viral capsid protein described herein, and (2)) a pharmaceutically acceptable carrier.

Also described herein are methods of making and using the recombinant viral capsid proteins, viral vectors comprising same, compositions, etc. In some embodiments, a methods of redirecting a virus, e.g., an adenovirus, adeno-associated virus, etc.; delivering diagnostic/therapeutic cargo to a target cell, etc. comprises contacting a target cell (which may be *in vitro* or *in vivo, e.g.,* in a human) with a recombinant viral vector comprising a recombinant viral capsid protein as described herein, wherein the viral capsid or viral vector comprises a targeting ligand that specifically binds a protein expressed on the surface the target cell. Such methods may include as a first step producing a recombinant viral vector, e.g., culturing a packaging cell in conditions sufficient for the production of viral vectors, wherein the packaging cell comprises a plasmid encoding the capsid protein comprising the peptide tag (first member) in the absence or presence of a plasmid encoding a reference capsid protein, incubating the recombinant capsid protein with a second cognate member operably linked to a targeting ligand, etc. In some embodiments, the target cell is a (human) liver cell and the (mosaic) recombinant viral vector comprises a targeting ligand that specifically binds asialoglycoprotein receptor, e.g., (h)ASGR1. In some embodiments, the target cell is a (human) neuronal cell, and the (mosaic) recombinant viral vector comprises a targeting ligand that specifically binds GABA, transferrin receptor, etc. In some embodiments, the target cell is a (human) T cell, and the (mosaic) recombinant viral vector comprises a targeting ligand that specifically binds CD3, e.g., CD3ε. In some embodiments, the target cell is a (human) hematopoietic stem cell, and the (mosaic) recombinant viral vector comprises a targeting ligand that specifically binds CD34. In some embodiments, the target cell is a (human) kidney cell. In some embodiments, the target cell a (human) muscle cell, and the (mosaic) recombinant viral vector comprises a targeting ligand that specifically binds an integrin. In some embodiments, the target cell is a (human) cancerous cell, and the (mosaic) recombinant viral vector comprises a targeting ligand that specifically binds a tumor associated antigen, e.g., E6 and E7, Her2, etc. In some embodiments, the targeting ligand binds human glucagon receptor (hGCGR).

Also described herein are methods of inactivating a viral capsid and/or producing viral vectors, which methods generally comprise (a) inserting a nucleic acid encoding a heterologous protein into a nucleic acid sequence encoding an viral capsid protein to form a nucleotide sequence encoding a genetically modified capsid protein comprising the peptide tag (in the absence or presence of a plasmid encoding a reference capsid protein) and/or (b) culturing a packaging cell in conditions sufficient for the production of viral vectors, wherein the packaging cell comprises the nucleotide sequence. In some embodiments, the packaging cell further comprises a helper plasmid and/or a transfer plasmid comprising a nucleotide of interest. In some embodiments, the methods further comprise isolating self-complementary adeno-associated viral vectors from culture supernatant. In some embodiments, the methods further comprise lysing the packaging cell and isolating single-stranded adeno-associated viral vectors from the cell lysate. In some embodiments, the methods further comprise (a) clearing cell debris, (b) treating the supernatant containing viral vectors with nucleases, e.g., DNase I in the presence of MgCl₂, (c) concentrating viral vectors, (d) purifying the viral vectors, and (e) any combination of (a)-(d). Also provided herein are viral vector made according to the method described herein, and packaging cell useful for producing a viral vector as described herein, e.g., packaging cells comprising a plasmid encoding a recombinant capsid protein described.

### BRIEF DESCRIPTION OF THE DRAWINGS

The patent or application file contains at least one drawing executed in color. Copies of this patent or patent application publication with color drawing(s) will be provided to the Office upon request and payment of the necessary fee.

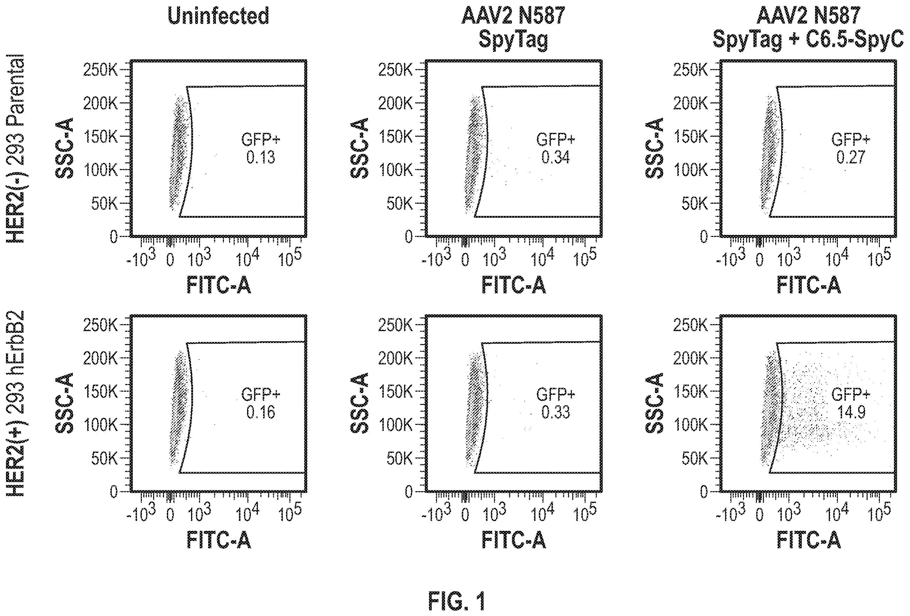

**Figure 1** provides scatter plots obtained from fluorescence-activated cell sorting (FACS) evaluating green fluorescent protein (GFP) expression by HER2-positive (+) 293 hErbB2 or HER2-negative (-) 293 parental cells either "Uninfected" or cells infected with "AAV2 N587-SpyTag" particles or cells infected with "AAV2 N587-SpyTag + C6.5-SpyC" particles. The capsids of both viruses contain the following mutations: R585A, delR588, and insertion of the SpyTag peptide (SEQ ID NO: 1) directly following residue N587 (SEQ ID NO:13). The C6.5-SpyC particles were conjugated to an anti-HER2 scFv (C6.5) fused with SpyCatcher (SEQ ID NO: 3) via SpyTag. Viruses express GFP as a marker of transduction.

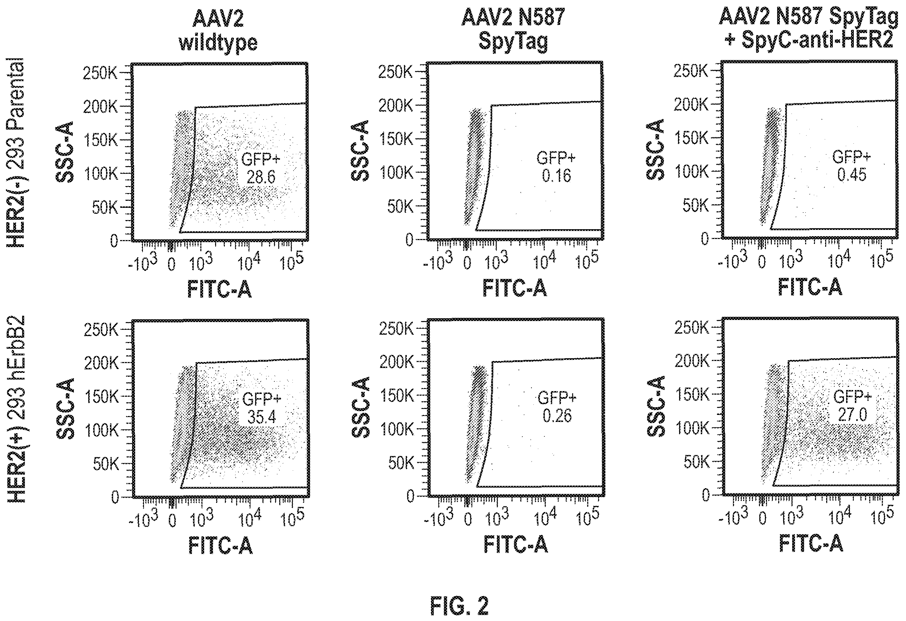

**Figure 2** provides scatter plots obtained from fluorescence-activated cell sorting (FACS) evaluating green fluorescent protein (GFP) expression by HER2-positive (+) 293 hErbB2 or HER2-negative (-) 293 parental cells either "Uninfected" or cells infected with "AAV2 N587-SpyTag" particles or cells infected with "AAV2 N587-SpyTag + SpyC-anti-HER2" particles. The capsids of both viruses contain the following mutations: R585A, delR588, and insertion of the SpyTag peptide (SEQ ID NO:1) directly following residue N587 (SEQ ID NO:13). The SpyC-anti-HER2 particles were conjugated to an anti-HER2 antibody (HERCEPTIN^{®}) fused with SpyCatcher (SEQ ID NO: 3) via SpyTag. Viruses express GFP as a marker of transduction.

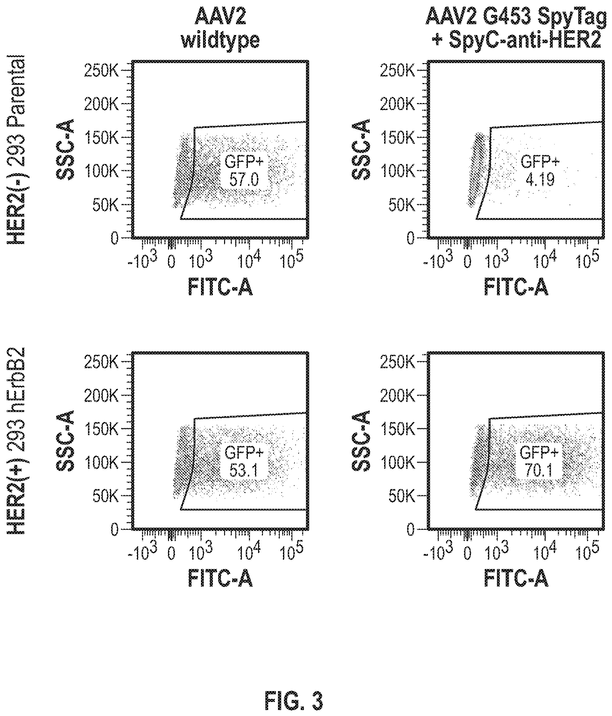

**Figure 3** provides scatter plots obtained from fluorescence-activated cell sorting (FACS) evaluating green fluorescent protein (GFP) expression by HER2-positive (+) 293 hErbB2 or HER2-negative (-) 293 parental cells infected with "AAV2 wildtype" particles or cells infected with "AAV2 G453-SpyTag + SpyC-anti-HER2" particles. "AAV2 wildtype" capsids have no mutations or modifications (SEQ ID NO: 9), while the "AAV2 G453-SpyTag" capsid is a mosaic viral particle comprised of a 1:7 ratio between "SpyTag" capsid proteins wherein the SpyTag is inserted directly following residue G453 flanked by a 10 amino acid linker (SEQ ID NO:29) and "AAV2 HBM" capsids without SpyTag but containing the mutations R585A and R588A (SEQ ID NO:11). The AAV2 G453-SpyTag mosaic particles were conjugated to an anti-HER2 antibody (HERCEPTIN^{®}) fused with SpyCatcher (SEQ ID NO:3) via SpyTag. Viruses express GFP as a marker of transduction.

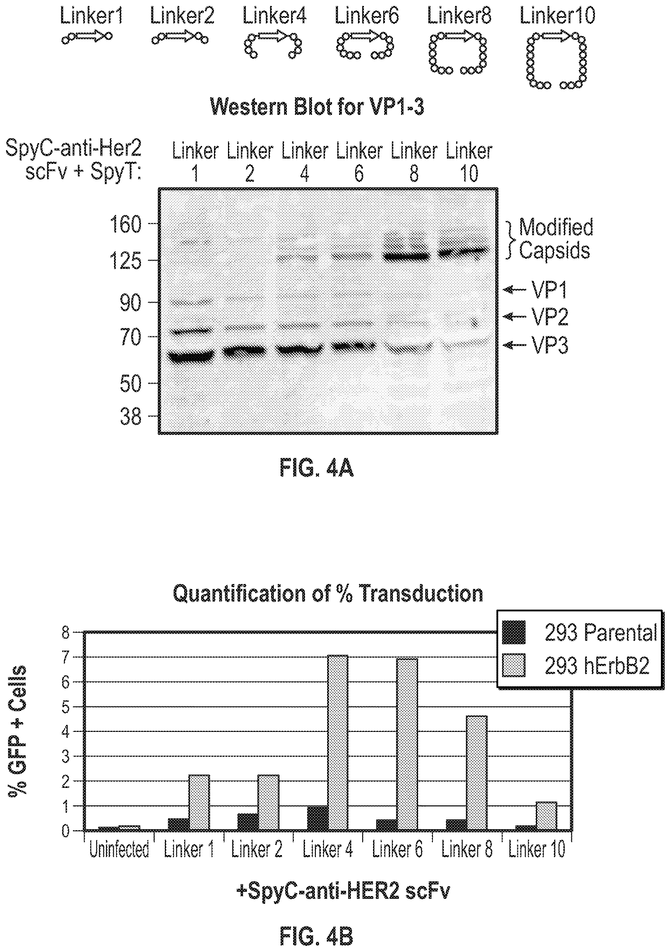

**Figure 4A** provides a western blot using B1 antibody, which recognizes a linear epitope shared by AAV2 VP1, VP2, and VP3 capsid proteins, analyzing the reaction between an anti-HER2 scFv fused with SpyCatcher "SpyC-anti-Her2 scFv", and a panel of AAV2 viral particles comprised of capsids with the following mutations: R585A, delR588, and insertion of the SpyTag peptide directly following residue N587, flanked by amino acid linkers of varying lengths (Linker 1, Linker 2, Linker 4, Linker 6, Linker 8 and Linker 10) (SEQ ID NO:13,15,17,19,21,23,25). **Figure 4B** provides the percentage of HER2+ cells (grey, y-axis) that express GFP versus the percentage of HER2- cells that express GFP (black, y-axis) 5 days post-infection with AAV2 viral particles comprised of capsids with the following mutations: R585A, delR588, and N587-SpyTag flanked by amino acid linkers of the indicated lengths (Linker 1, Linker 2, Linker 4, Linker 6, Linker 8 and Linker 10) (SEQ ID NOs:13, 15, 17, 19, 21, 23, and 25, respectively) (x-axis). The AAV2 N587 SpyTag particles were conjugated to an anti-HER2 scFv fused with SpyCatcher (SEQ ID NO:3).

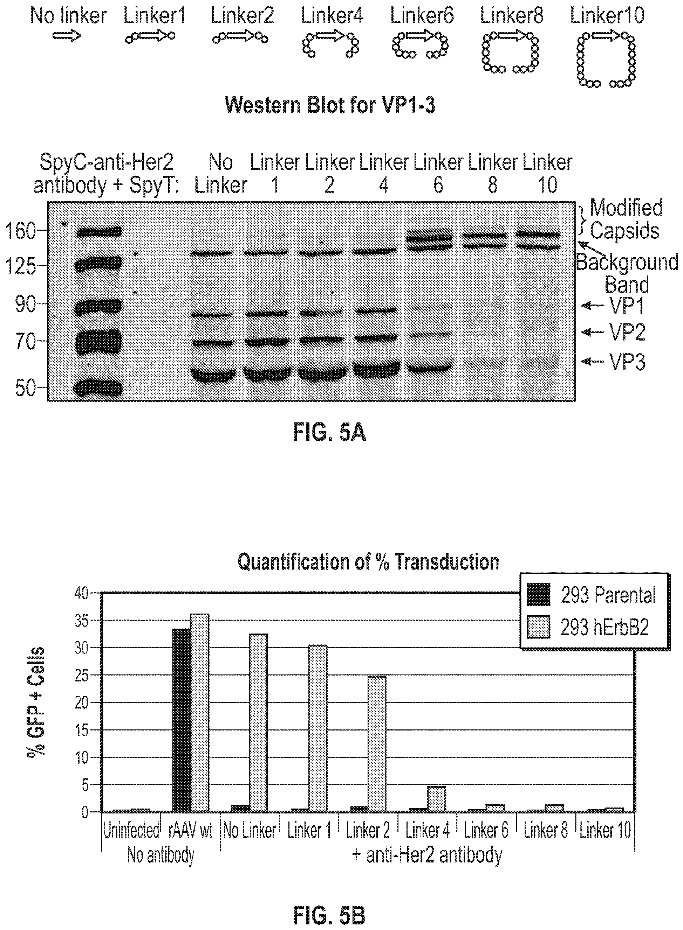

**Figure 5A** provides a western blot using B1 antibody, which recognizes a linear epitope shared by AAV2 VP1, VP2, and VP3 capsid proteins, analyzing the reaction between an anti-HER2 antibody (HERCEPTIN^{®}) fused with SpyCatcher "SpyC-anti-Her2 antibody", and a panel of AAV2 viral particles comprised of capsids with the following mutations: R585A, delR588, and insertion of the SpyTag peptide directly following residue N587, flanked by amino acid linkers of varying lengths (No linker, Linker 1, Linker 2, Linker 4, Linker 6, Linker 8 and Linker 10) (SEQ ID NOs:13, 15, 17, 19, 21, 23, and 25, respectively)**Figure 5B** provides the percentage of HER2+ cells (grey, y-axis) that express GFP versus the percentage of HER2- cells that express GFP (black, y-axis) 5 days post-infection with wildtype (wt) AAV2 particles or AAV2 viral particles comprised of capsids with the following mutations: R585A, delR588, and N587-SpyTag flanked by amino acid linkers of the indicated lengths (No linker, Linker 1, Linker 2, Linker 4, Linker 6, Linker 8 and Linker 10) (SEQ ID NOs: 13, 15, 17, 19, 21, 23, and 25, respectively) (x-axis). The AAV2 N587 SpyTag particles were conjugated to an anti-HER2 antibody (HERCEPTIN^{®}) fused with SpyCatcher (SEQ ID NO:3).

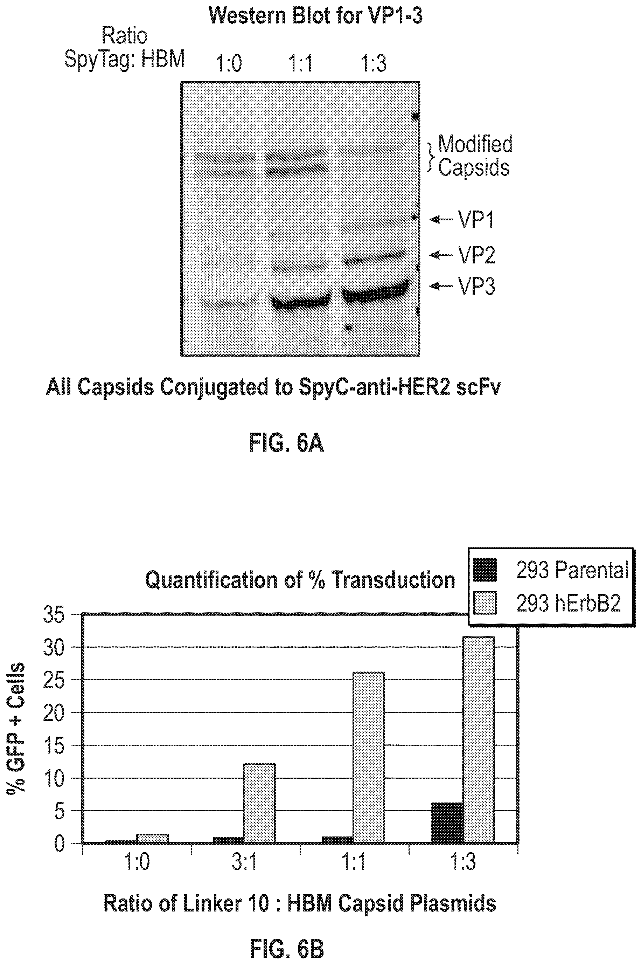

**Figure 6A** provides a western blot using B1 antibody, which recognizes a linear epitope shared by AAV2 VP1, VP2, and VP3 capsid proteins, analyzing the reaction between an anti-HER2 scFv C6.5 fused with SpyCatcher (SEQ ID NO:3) "SpyC-anti-HER2 scFv" and a panel of mosaic AAV2 viral particles comprised of mixtures between "SpyTag" capsid proteins containing mutations R585A, delR588, and N587-linker 10-SpyTag (SEQ ID NO:25), and "HBM" capsid proteins containing mutations R585A and R588A, but no SpyTag (SEQ ID NO:11). "SpyTag" and "HBM" capsid proteins were mixed at varying ratios (1:0, 1:1, and 1:3). **Figure 6B** provides the percentage of HER2+ 293 hErbB2 cells (grey bars) and HER2- 293 parental cells (black bars) that express GFP (y-axis) 5 days post-infection with mosaic AAV2 viral particles (x-axis) comprised of mixtures between "Linker 10" capsid proteins containing mutations R585A, delR588, and N587-linker 10-SpyTag (SEQ ID NO:25), and "HBM" capsid proteins containing mutations R585A and R588A, but no SpyTag (SEQ ID NO:11). "Linker 10" and "HBM" capsid proteins were mixed at varying ratios (1:0, 3:1, 1:1, and 1:3), and were conjugated to an anti-HER2 scFv fused with SpyCatcher (SEQ ID NO:3).

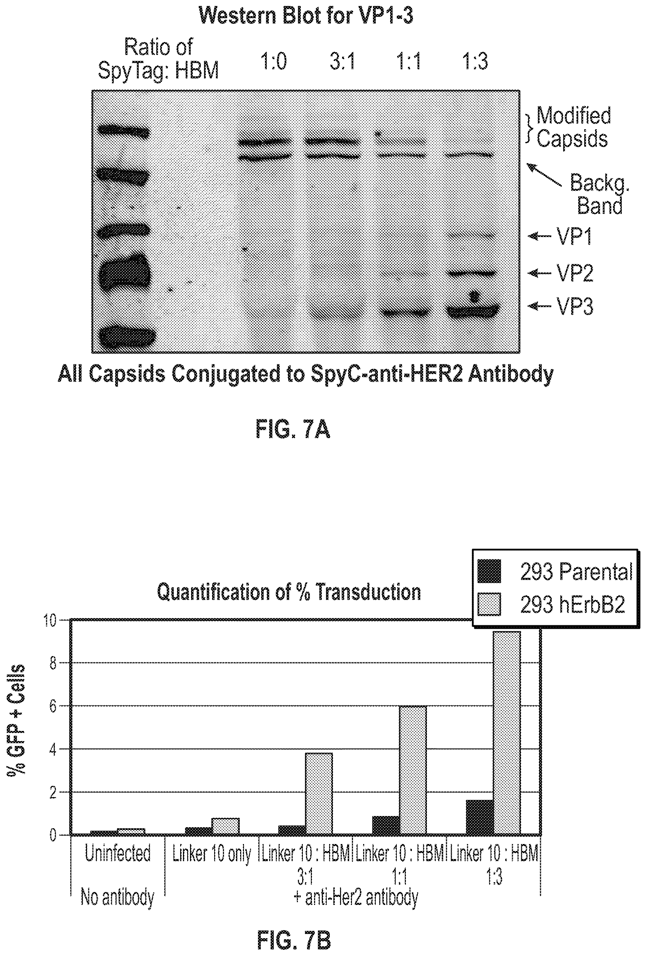

**Figure 7A** provides a western blot using B1 antibody, which recognizes a linear epitope shared by AAV2 VP1, VP2, and VP3 capsid proteins, analyzing the reaction between an anti-HER2 antibody (HERCEPTIN^{®}) fused with SpyCatcher (SEQ ID NO:3) "SpyC-anti-HER2 antibody" and a panel of mosaic AAV2 viral particles comprised of mixtures between "SpyTag" capsid proteins containing mutations R585A, delR588, and N587-linker 10-SpyTag (SEQ ID NO:25), and "HBM" capsid proteins containing mutations R585A and R588A, but no SpyTag (SEQ ID NO:11). "SpyTag" and "HBM" capsid proteins were mixed at varying ratios (1:0, 3:1, 1:1, and 1:3). **Figure 7B** provides the percentage of HER2+ 293 hErbB2 cells (grey bars) and HER2- 293 parental cells (black bars) that express GFP (y-axis) 5 days post-infection with mosaic AAV2 viral particles (x-axis) comprised of mixtures between "Linker 10" capsid proteins containing mutations R585A, delR588, and N587-linker 10-SpyTag (SEQ ID NO:25), and "HBM" capsid proteins containing mutations R585A and R588A, but no SpyTag (SEQ ID NO:11). "Linker 10" and "HBM" capsid proteins were mixed at varying ratios (1:0, 3:1, 1:1, and 1:3), and were conjugated to an anti-HER2 antibody (HERCEPTIN^{®}) fused with SpyCatcher (SEQ ID NO:3).

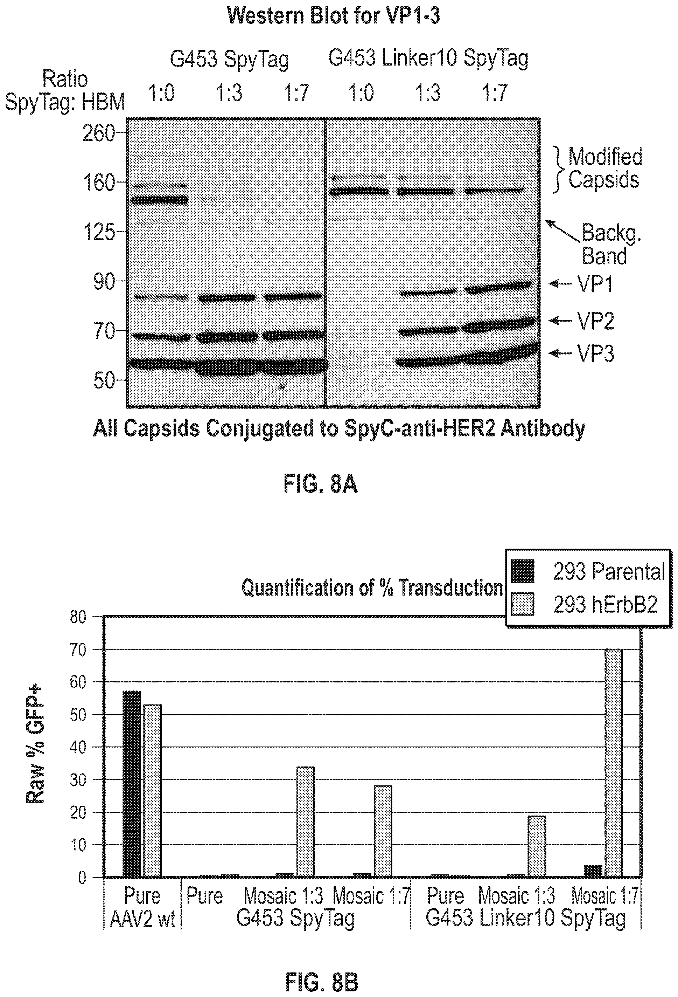

**Figure 8A** provides a western blot using B1 antibody, which recognizes a linear epitope shared by AAV2 VP1, VP2, and VP3 capsid proteins, analyzing the reaction between an anti-HER2 antibody (HERCEPTIN^{®}) fused with SpyCatcher (SEQ ID NO:3) "SpyC-anti-HER2 antibody" and a panel of mosaic AAV2 viral particles comprised of mixtures between "SpyTag" capsid proteins and "HBM" capsid proteins containing mutations R585A and R588A, but no SpyTag (SEQ ID NO:11). "SpyTag" capsid proteins include "G453 SpyTag", which contains mutations R585A, R588A and insertion of the SpyTag peptide directly following residue G453 (SEQ ID NO:27), and "G453 Linker10 SpyTag", which contains mutations R585A, R588A and insertion of the SpyTag peptide directly following residue G453 and flanked on either side by 10 linker amino acids (SEQ ID NO:29). The indicated "G453 SpyTag" and "G453 Linker10 SpyTag" capsids were mixed with "HBM" capsids at varying ratios (1:0, 1:3, and 1:7). **Figure 8B** provides the percentage of HER2+ 293 hErbB2 cells (grey bars) and HER2- 293 parental cells (black bars) that express GFP (y-axis) 5 days post-infection with wildtype "wt" or mosaic AAV2 viral particles (x-axis) comprised of mixtures between "G453 SpyTag" capsid proteins containing mutations R585A, R588A, and insertion of the SpyTag peptide directly following residue G453 (SEQ ID NO:27), or "G453 Linker 10 SpyTag" capsid proteins containing mutations R585A, R588A, and insertion of the SpyTag peptide directly following residue G453 and flanked on either side by 10 linker amino acids (SEQ ID NO:29), and "HBM" capsid proteins containing mutations R585A and R588A, but no SpyTag (SEQ ID NO:11). "G453 SpyTag" or "G453 Linker10 SpyTag" and "HBM" capsids were mixed at varying ratios (1:0 "pure", 1:3, and 1:7), and were conjugated to an anti-HER2 antibody (HERCEPTIN^{®}) fused with SpyCatcher (SEQ ID NO:3).

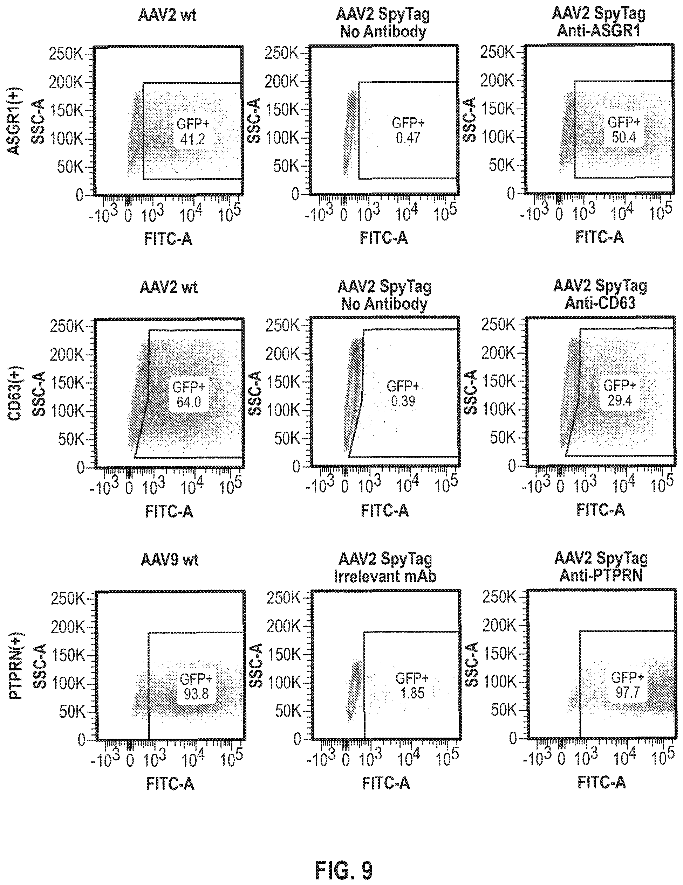

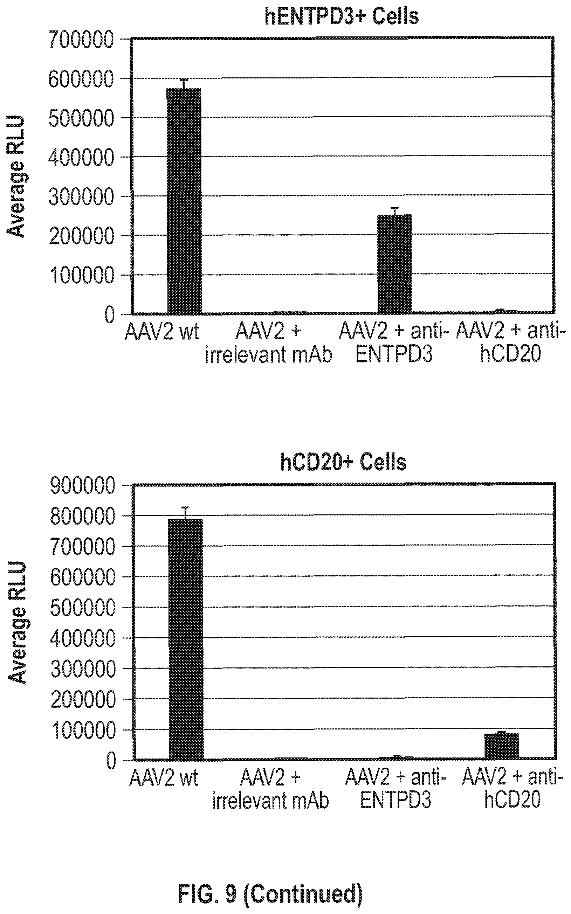

**Figure 9** provides scatter plots obtained from fluorescence-activated cell sorting (FACS) evaluating green fluorescent protein (GFP) expression by cells positive (+) for ASGR1 expression after infection with "AAV2 wt" particles, "AAV2 SpyTag no antibody" particles, or "AAV2 SpyTag Anti-ASGR1" particles. "AAV2 wt" capsids are wildtype with no mutations or modifications (SEQ ID NO:9), while the "AAV2 SpyTag" capsid contains the following mutations: R585A, delR588, and insertion of the SpyTag peptide directly following residue N587 (SEQ ID NO:13). The "AAV2 SpyTag Anti-ASGR1" particles were conjugated to a SpyCatcher-fused antibody that specifically binds ASGR1. Also shown are scatter plots obtained from fluorescence-activated cell sorting (FACS) evaluating green fluorescent protein (GFP) expression by cells positive (+) for CD63 expression after infection with "AAV2 wt" particles, "AAV2 SpyTag no antibody" particles, or "AAV2 SpyTag Anti-CD63" particles. "AAV2 wt" capsids are wildtype with no mutations or modifications (SEQ ID NO:9), while the "AAV2 SpyTag" capsid contains the following mutations: R585A, delR588, and insertion of the SpyTag peptide directly following residue N587 (SEQ ID NO:13). The "AAV2 SpyTag Anti-CD63" particles were conjugated to a SpyCatcher-fused antibody that specifically binds CD63. Viruses express GFP as a marker of transduction. Also shown are scatter plots obtained from fluorescence-activated cell sorting (FACS) evaluating green fluorescent protein (GFP) expression by cells positive (+) for PTPRN expression after infection with "AAV9 wt" particles, "AAV2 SpyTag Irrelevant Antibody" particles, or "AAV2 SpyTag Anti-PTPRN" particles. "AAV9 wt" capsids are wildtype with no mutations or modifications (SEQ ID NO:31), while the "AAV2 SpyTag" capsids are mosaic viral particles comprised of a 1:7 ratio between "SpyTag" capsid proteins wherein the SpyTag is inserted directly following residue G453 flanked by a 10 amino acid linker (SEQ ID NO:29) and between capsids without SpyTag but containing a Myc tag amino acid sequence inserted directly following residue N587 (SEQ ID NO:53) which reduces natural receptor binding. The "AAV2 SpyTag Irrelevant Antibody" particles were conjugated to a SpyCatcher-fused antibody that does not bind PTPRN. The "AAV2 SpyTag Anti-PTPRN" particles were conjugated to a SpyCatcher-fused antibody that specifically binds PTPRN. Viruses express GFP as a marker of transduction. Also shown are results of a Luciferase assay evaluating Firefly Luciferase expression by cells positive (+) for hENTPD3 after infection with "AAV2 wt" particles, "AAV2 + irrelevant mAb" particles, "AAV2 + Anti-ENTPD3" particles, and "AAV2 + Anti-hCD20" particles. "AAV2 wt" capsids are wildtype with no mutations or modifications (SEQ ID NO:9), while the "AAV2 + antibody" capsids contain the following mutations: R585A, delR588, and insertion of the SpyTag peptide directly following residue N587 (SEQ ID NO: 13). The "AAV2 + irrelevant mAb" particles were conjugated to a SpyCatcher-fused antibody that does not bind hENTPD3. The "AAV2 + anti-hCD20" particles were conjugated to a SpyCatcher-fused antibody that specifically binds hCD20, which is not expressed on hENTPD3+ cells and serves as an additional negative control. The "AAV2 + anti-ENTPD3" particles were conjugated to a SpyCatcher-fused antibody that specifically binds hENTPD3. Viruses express Firefly Luciferase as a marker of transduction. Also shown are results of a Luciferase assay evaluating Firefly Luciferase expression by cells positive (+) for hCD20 after infection with "AAV2 wt" particles, "AAV2 + irrelevant mAb" particles, "AAV2 + Anti-ENTPD3" particles, and "AAV2 + Anti-hCD20" particles. "AAV2 wt" capsids are wildtype with no mutations or modifications (SEQ ID NO:9), while the "AAV2 + antibody" capsids contain the following mutations: R585A, delR588, and insertion of the SpyTag peptide directly following residue N587 (SEQ ID NO: 13). The "AAV2 + irrelevant mAb" particles were conjugated to a SpyCatcher-fused antibody that does not bind hENTPD3. The "AAV2 + anti-ENTPD3" particles were conjugated to a SpyCatcher-fused antibody that specifically binds hENTPD3, which is not expressed on hCD20+ cells and serves as an additional negative control. The "AAV2 + anti-hCD20" particles were conjugated to a SpyCatcher-fused antibody that specifically binds hCD20. Viruses express Firefly Luciferase as a marker of transduction.

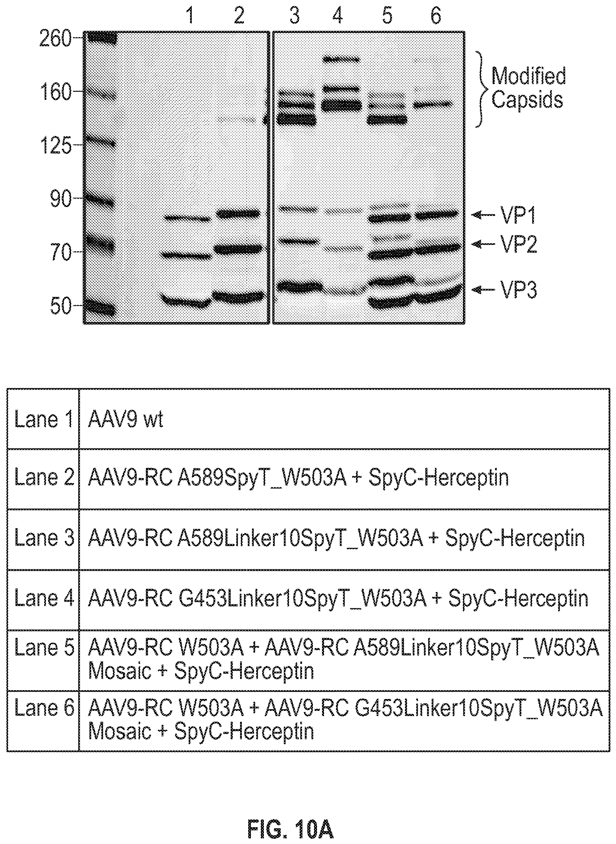

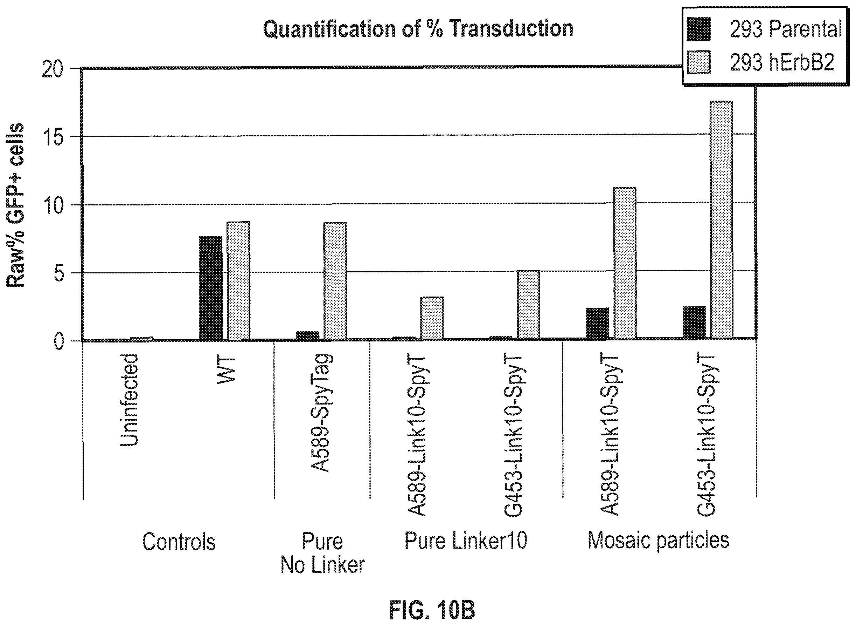

**Figure 10A** provides a western blot using B1 antibody, which recognizes a linear epitope shared by AAV9 VP1, VP2, and VP3 capsid proteins, analyzing the reaction between an anti-HER2 antibody (HERCEPTIN^{®}) fused with SpyCatcher (SEQ ID NO:3) "SpyC-Herceptin" and a panel of AAV9 viral particles. These AAV9 viral particles are comprised of capsids containing the mutation W503A, which reduces receptor binding, and insertion of the SpyTag peptide directly following residue A589 or G453 without a linker or flanked by a 10 amino acid linker, or mosaic AAV9 viral particles comprised of a 1:7 ratio between "SpyTag" capsid proteins containing mutations W503A and either A589-Linker10-SpyTag or G453-Linker10-SpyTag, with "W503A" capsid proteins containing mutation W503A, but no SpyTag. **Figure 10B** provides the percentage of HER2+ 293 hErbB2 cells (grey bars) and HER2- 293 parental cells (black bars) (y-axis) that express GFP five days post-infection with AAV9 viral particles conjugated to an anti-HER2 antibody (HERCEPTIN^{®}) fused with SpyCatcher (x-axis). These AAV9 viral particles are comprised of capsids containing the mutation W503A, which reduces receptor binding, and insertion of the SpyTag peptide directly following residue A589 (or G453) without a linker or flanked by a 10 amino acid linker, or mosaic AAV9 viral particles comprised of a 1:7 ratio between "SpyTag" capsid proteins containing mutations W503A and either A589-Linker10-SpyTag or G453-Linker10-SpyTag, with "W503A" capsid proteins containing mutation W503A, but no SpyTag. Viruses express GFP as a marker of transduction.

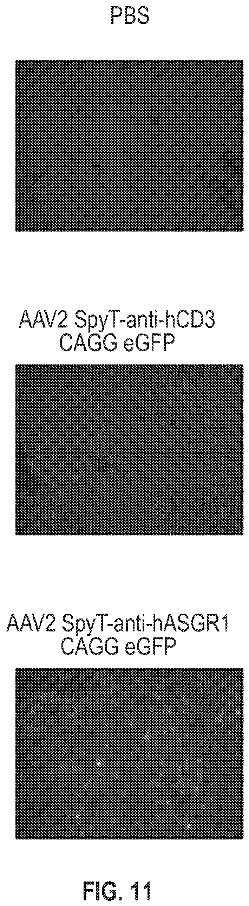

**Figure 11** provides immunofluorescence microscopy images of liver samples taken from C57BL/6 mice transgenically modified to express human ASGR1 on liver cells. Samples were collected ten days post intravenous injection with phosphate buffered saline (PBS) or with 2.5x10¹¹ viral genome (vg)/ animal of SpyTagged AAV2 particles carrying a CAGG eGFP nucleotide of interest and modified by (1) SpyCatcher-anti-human CD3 antibody (AAV SpyT-anti-hCD3 CAGG eGFP) or (2) SpyCatcher-anti-human ASGR1 antibody (AAV SpyT-anti-hASGR1 CAGG eGFP). Mice were sacrificed and transcardially perfused with 4% PFA. Organs of livers, kidney and heart were collected and dehydrated in 15% sucrose followed by 30% sucrose. Then organs were cyro-sectioned on slides and stained with chicken anti-EGFP antibody (Jackson ImmunoResearch Labs, Inc. West Grove, PA) and Alexa-488 conjugated anti-chicken secondary antibody (Jackson ImmunoResearch Labs, Inc. West Grove, PA). Each image represents one mouse. The "SpyTagged AAV2" capsid contains the following mutations: R585A, delR588, and insertion of the SpyTag peptide directly following residue N587 (SEQ ID NO:13). Viruses express eGFP as a marker of transduction.

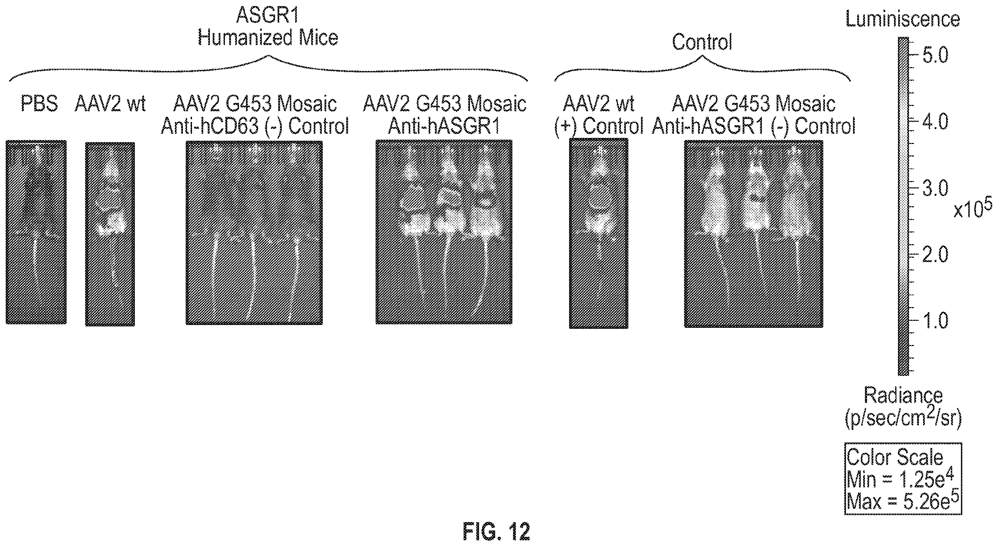

**Figure 12** provides luminescence images of individual mice that do not express human ASGR1 on liver cells (Control) and genetically modified mice that express human ASGR1 on liver cells (ASGR1 Humanized mice) 14 days post intravenous injection with phosphate buffered saline (PBS) or with 3.0x10¹¹ viral genomes (vg)/ animal of wildtype (wt) AAV2 particles, or SpyTagged AAV2 particles carrying firefly luciferase nucleotide of interest and modified by (1) SpyCatcher-anti-human CD63 antibody or (2) SpyCatcher-anti-human ASGR1 antibody. These AAV2 viral particles are mosaic viral particles comprised of a 1:7 ratio between "SpyTag" capsids proteins wherein the SpyTag is inserted directly following residue G453 flanked by a 10 amino acid linker (SEQ ID NO:29) and between capsids without SpyTag but containing a Myc tag amino acid sequence inserted directly following residue N587 (SEQ ID NO:53) which reduces natural receptor binding. Viruses express Firefly luciferase as a marker of transduction. Mice were anesthetized using isoflurane, injected with a Luciferin substrate and imaged 10 minutes later using the IVIS Spectrum In Vivo Imaging System (PerkinElmer).

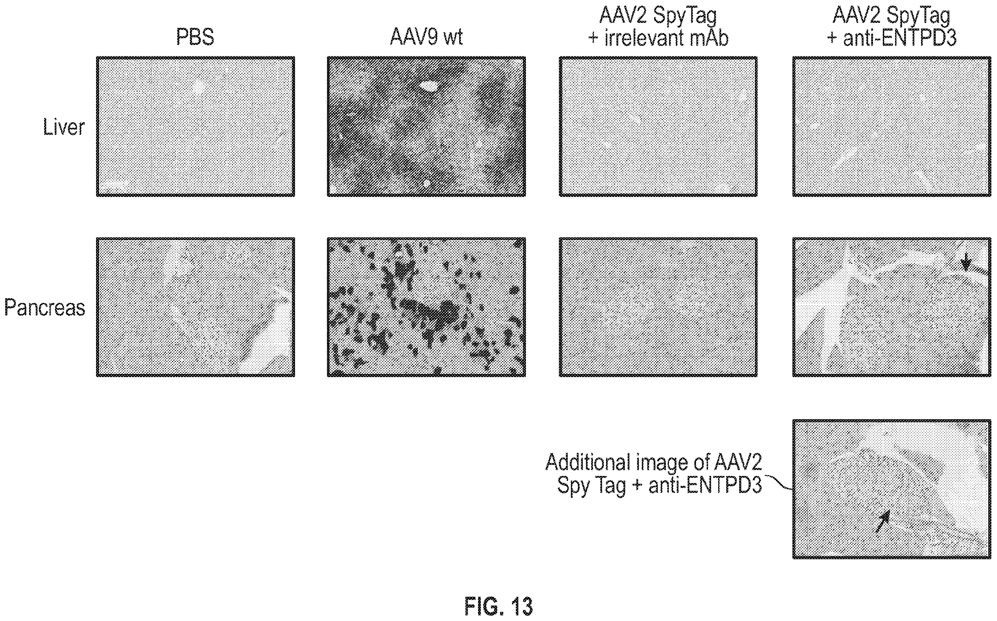

**Figure 13** provides immunohistochemistry images of liver and pancreas samples taken from C57BL/6 mice 4 weeks post intravenous injection with phosphate buffered saline (PBS) or with 1.0x10¹² viral genome (vg)/ animal of wildtype (wt) AAV9 particles, or with 1.0x10¹³ viral genome (vg)/ animal of SpyTagged AAV2 particles carrying a CMV eGFP nucleotide of interest and modified by (1) SpyCatcher-anti-human ASGR1 antibody (AAV2 SpyTag + irrelevant mAb) or (2) SpyCatcher-anti-human ENTPD3 antibody antibody (AAV2 SpyTag + anti-ENTPD3). Mice were sacrificed and liver and pancreas were collected and fixed in 10% neutral buffered formalin. Then organs were embedded and cyro-sectioned on slides and stained with anti-GFP antibodies. Each image represents one mouse. These AAV2 viral particles are mosaic viral particles comprised of a 1:7 ratio between "SpyTag" capsids proteins wherein the SpyTag is inserted directly following residue G453 flanked by a 10 amino acid linker (SEQ ID NO:29) and between capsids without SpyTag but containing a Myc tag amino acid sequence inserted directly following residue N587 (SEQ ID NO:53) which reduces natural receptor binding. Viruses express eGFP as a marker of transduction. ENTPD3 is reported to be expressed in pancreatic islet cells and in the tongue, among other cell types, but not in the liver. SpyTagged AAV2 particles were detargeted from liver; eGFP expression was not observed in the livers of mice injected with "AAV2 SpyTag + irrelevant mAb" or "AAV2 SpyTag + anti-ENTPD3" particles. Positive staining in pancreatic islets was detected in the pancreas sample of one of the mice injected with "AAV2 SpyTag + anti-ENTPD3" particles.

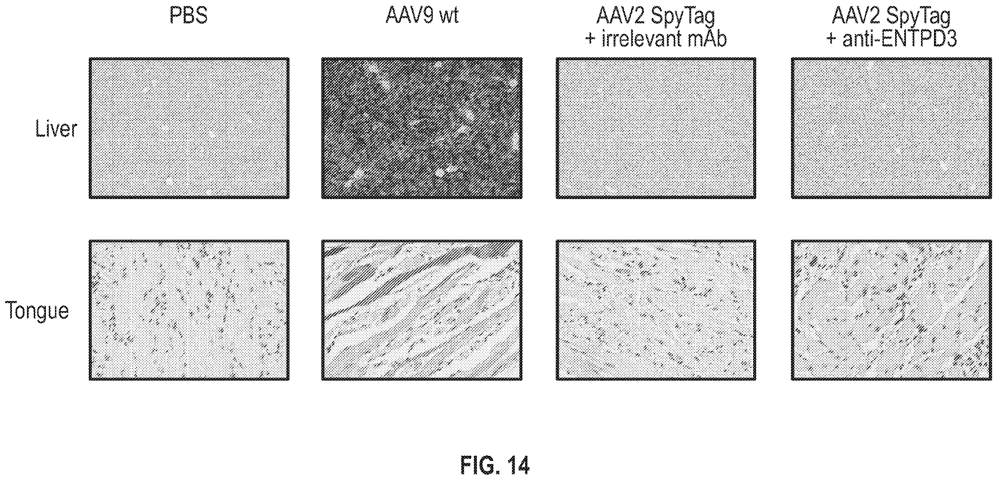

**Figure 14** provides immunohistochemistry images of liver and tongue samples taken from C57BL/6 mice 14 days post intravenous injection with phosphate buffered saline (PBS) or with 2.0x10¹² viral genome (vg)/ animal of wildtype (wt) AAV9 particles, or SpyTagged AAV2 particles carrying a CMV eGFP nucleic acid of interest and modified by (1) SpyCatcher-anti-human ASGR1 antibody (AAV2 SpyTag + irrelevant mAb) or (2) SpyCatcher-anti-human ENTPD3 antibody, which binds to mouse ENTPD3 (AAV2 SpyTag + anti-ENTPD3). Mice were sacrificed and livers and tongues were collected and fixed in 10% neutral buffered formalin. Then organs were embedded and cyro-sectioned on slides and stained with anti-GFP antibodies. Each image represents one mouse; three mice were injected and analyzed from the "AAV2 SpyTag + irrelevant mAb" and "AAV2 SpyTag + anti-ENTPD3" groups and all showed similar GFP expression patterns. These AAV2 viral particles are mosaic viral particles comprised of a 1:7 ratio between "SpyTag" capsids proteins wherein the SpyTag is inserted directly following residue G453 flanked by a 10 amino acid linker (SEQ ID NO:29) and between capsids without SpyTag but containing a Myc tag amino acid sequence inserted directly following residue N587 (SEQ ID NO:52) which reduces natural receptor binding. Viruses express eGFP as a marker of transduction. ENTPD3 is reported to be expressed in the mouse tongue but not in the liver (data extracted from public databases GenePaint.org http://www.informatics.iax.org/assay/MGI:5423021 and Riken FANTOM5 project, adult mouse dataset). eGFP expression was not observed in the livers of mice injected with "AAV2 SpyTag + irrelevant mAb" or "AAV2 SpyTag + anti-ENTPD3" particles, but was detected in the tongue of all three mice injected with "AAV2 SpyTag + anti-ENTPD3" particles.

### DETAILED DESCRIPTION

WO201611291 describes the utilization of a specific binding pair (SpyCatcher: SpyTag) to generate virus like particles (VLP) from a modified bacteriophage AP205 displaying immunogenic antigens at a high density on a the AP205 capsid for the purposes of vaccination. Theoretically, for the purposes of retargeting a viral vector, such a high degree of modification of the viral capsid may be desirable to ensure that the natural tropism of the viral vector is substantially reduced or abolished. However, such displaying targeting ligands at a high density on the viral surface may interfere with transductions efficiencies. *See,* Examples 4 and 5. To achieve optimal transduction efficiencies, it was discovered that optimal transduction efficiencies occur when the degree of modification of the viral surface with the member is decreased. As such, provided herein are the genetically modified viral particles, compositions comprising same, and methods of making and using same.

Unless defined otherwise, all technical and scientific terms used herein have the same meaning as commonly understood by one of ordinary skill in the art to which this invention belongs.

Singular forms "a", "an", and "the" include plural references unless the context clearly dictates otherwise. Thus, for example, a reference to "a method" includes one or more methods, and/or steps of the type described herein and/or which will become apparent to those persons skilled in the art upon reading this disclosure.

The term "antibody" includes immunoglobulin molecules comprising four polypeptide chains, two heavy (H) chains and two light (L) chains inter-connected by disulfide bonds. Each heavy chain comprises a heavy chain variable domain (V_{H}) and a heavy chain constant region (C_{H}). The heavy chain constant region comprises at least three domains, C_{H}1, C_{H}2, C_{H}3 and optionally CH₄. Each light chain comprises a light chain variable domain (C_{H}) and a light chain constant region (C_{L}). The heavy chain and light chain variable domains can be further subdivided into regions of hypervariability, termed complementarity determining regions (CDR), interspersed with regions that are more conserved, termed framework regions (FR). Each heavy and light chain variable domain comprises three CDRs and four FRs, arranged from amino-terminus to carboxy-terminus in the following order: FR1, CDR1, FR2, CDR2, FR3, CDR3, FR4 (heavy chain CDRs may be abbreviated as HCDR1, HCDR2 and HCDR3; light chain CDRs may be abbreviated as LCDR1, LCDR2 and LCDR3. Typical tetrameric antibody structures comprise two identical antigen-binding domains, each of which formed by association of the V_{H} and V_{L} domains, and each of which together with respective C_{H} and C_{L} domains form the antibody Fv region. Single domain antibodies comprise a single antigen-binding domain, e.g., a V_{H} or a V_{L}. The antigen-binding domain of an antibody, e.g., the part of an antibody that recognizes and binds to the first member of a specific binding pair of an antigen, is also referred to as a "paratope." It is a small region (of 5 to 10 amino acids) of an antibody's Fv region, part of the fragment antigen-binding (Fab region), and may contains parts of the antibody's heavy and/or light chains. A paratope specifically binds a first member of a specific binding pair when the paratope binds the first member of a specific binding pair with a high affinity. The term "high affinity" antibody refers to an antibody that has a K_{D} with respect to its target first member of a specific binding pair about of 10⁻⁹ M or lower (*e.g*., about 1 x 10⁻⁹ M, 1 x 10⁻¹⁰ M, 1 x 10⁻¹¹ M, or about 1 x 10⁻¹² M). In one embodiment, K_{D} is measured by surface plasmon resonance, *e.g*., BIACORE^{™}; in another embodiment, K_{D} is measured by ELISA.

The phrase "complementarity determining region," or the term "CDR," includes an amino acid sequence encoded by a nucleic acid sequence of an organism's immunoglobulin genes that normally (i.e., in a wild-type animal) appears between two framework regions in a variable region of a light or a heavy chain of an immunoglobulin molecule (e.g., an antibody or a T cell receptor). A CDR can be encoded by, for example, a germ line sequence or a rearranged or unrearranged sequence, and, for example, by a naive or a mature B cell or a T cell. A CDR can be somatically mutated (e.g., vary from a sequence encoded in an animal's germ line), humanized, and/or modified with amino acid substitutions, additions, or deletions. In some circumstances (e.g., for a CDR3), CDRs can be encoded by two or more sequences (e.g., germ line sequences) that are not contiguous (e.g., in an unrearranged nucleic acid sequence) but are contiguous in a B cell nucleic acid sequence, e.g., as the result of splicing or connecting the sequences (e.g., V-D-J recombination to form a heavy chain CDR3).

The phrase "Inverted terminal repeat" or "ITR" includes symmetrical nucleic acid sequences in the genome of adeno-associated viruses required for efficient replication. ITR sequences are located at each end of the AAV DNA genome. The ITRs serve as the origins of replication for viral DNA synthesis and are essential cis components for generating AAV vectors.

The phrase "light chain" includes an immunoglobulin light chain sequence from any organism, and unless otherwise specified includes human κ and λ light chains and a VpreB, as well as surrogate light chains. Light chain variable domains typically include three light chain CDRs and four framework (FR) regions, unless otherwise specified. Generally, a full-length light chain includes, from amino terminus to carboxyl terminus, a variable domain that includes FR1-CDR1-FR2-CDR2-FR3-CDR3-FR4, and a light chain constant region. A light chain variable domain is encoded by a light chain variable region gene sequence, which generally comprises V_{L} and J_{L} segments, derived from a repertoire of V and J segments present in the germ line. Sequences, locations and nomenclature for V and J light chain segments for various organisms can be found in IMGT database, www.imgt.org. Light chains include those, e.g., that do not selectively bind either a first or a second first member of a specific binding pair selectively bound by the first member of a specific binding pair-binding protein in which they appear. Light chains also include those that bind and recognize, or assist the heavy chain or another light chain with binding and recognizing, one or more first member of a specific binding pairs selectively bound by the first member of a specific binding pair-binding protein in which they appear. Common or universal light chains include those derived from a human Vκ1-39Jκ gene or a human Vκ3-20Jκ gene, and include somatically mutated (e.g., affinity matured) versions of the same. Exemplary human V_{L} segments include a human Vκ1-39 gene segment, a human Vκ3-20 gene segment, a human Vλ1-40 gene segment, a human Vλ1-44 gene segment, a human Vλ2-8 gene segment, a human Vλ2-14 gene segment, and human Vλ3-21 gene segment, and include somatically mutated (e.g., affinity matured) versions of the same. Light chains can be made that comprise a variable domain from one organism (e.g., human or rodent, e.g., rat or mouse; or bird, e.g., chicken) and a constant region from the same or a different organism (e.g., human or rodent, e.g., rat or mouse; or bird, e.g., chicken).

The term "about" or "approximately" includes being within a statistically meaningful range of a value. Such a range can be within an order of magnitude, preferably within 50%, more preferably within 20%, still more preferably within 10%, and even more preferably within 5% of a given value or range. The allowable variation encompassed by the term "about" or "approximately" depends on the particular system under study, and can be readily appreciated by one of ordinary skill in the art.

The term "capsid protein" includes a protein that is part of the capsid of the virus. For adeno-associated viruses, the capsid proteins are generally referred to as VP1, VP2 and/or VP3, and are encoded by the single *cap* gene. For AAV, the three AAV capsid proteins are produced in an overlapping fashion from the *cap* open reading frame (ORF) via alternative mRNA splicing and/or alternative translational start codon usage, although all three proteins use a common stop codon. Warrington et al. (2004) J. Virol. 78:6595, incorporated herein by reference in its entirety. VP1 of AAV2 is generally translated from an ATG start codon (amino acid M1) on a 2.4-kb mRNA, while VP2 and VP3 of AAV2 arise from a smaller 2.3-kb mRNA, using a weaker ACG start codon for VP2 production (amino acid T138) and readthrough translation to the next available ATG codon (amino acid M203) for the production of the most abundant capsid protein, VP3. *Warrington, supra;* Rutledge et al. (1998) J. Virol. 72:309-19, incorporated herein by reference in its entirety. The amino acid sequences of capsid proteins of adeno-associated viruses are well-known in the art and generally conserved, particularly within the dependoparvoviruses. *See, Rutledge et al., supra.* For example, *Rutledge et al.* (1998), *supra,* provides at Figure 4B amino acid sequence alignments for VP1, VP2, and VP3 capsid proteins of AAV2, AAV3, AAV4 and AAV6, wherein the start sites for each of the VP1, VP2, and VP3 capsid proteins are indicated by arrows and the variable domains are boxed. Accordingly, although amino acid positions provided herein may be provided in relation to the VP1 capsid protein of the AAV, and amino acid positions provided herein that are not further specified refer to the AAV2 sequence of the major coat protein VP1 set forth as SEQ ID NO:1, a skilled artisan would be able to respectively and readily determine the position of that same amino acid within the VP2 and/or VP3 capsid protein of the AAV, and the corresponding position of amino acids among different serotypes. Accordingly, although amino acid positions provided herein may be provided in relation to the VP1 capsid protein of the AAV, and amino acid positions provided herein that are not further specified refer to the AAV-2 sequence of the major coat protein VP1 set forth as SEQ ID NO: 9, a skilled artisan would be able to respectively and readily determine the position of that same amino acid within the VP2 and/or VP3 capsid protein of the AAV, and the corresponding amino acid and position among different AAV serotypes. Additional, a skilled artisan would be able to swap domains between capsid proteins of different AAV serotypes for the formation of a "chimeric capsid protein."

Domain swapping between two AAV capsid protein constructs for the generation of a "chimeric AAV capsid protein" has been described, *see, e.g.,* Shen et al. (2007) Mol. Therapy 15(11):1955-1962, incorporated herein in its entirety by reference. A "chimeric AAV capsid protein" includes an AAV capsid protein that comprises amino acid sequences, e.g., domains, from two or more different AAV serotypes and that is capable of forming and/or forms an AAV-like viral capsid/viral particle. A chimeric AAV capsid protein is encoded by a chimeric AAV capsid gene, e.g., a nucleotide comprising a plurality, e.g., at least two, nucleic acid sequences, each of which plurality is identical to a portion of a capsid gene encoding a capsid protein of distinct AAV serotypes, and which plurality together encodes a functional chimeric AAV capsid protein. Reference to a chimeric capsid protein in relation to a specific AAV serotype indicates that the capsid protein comprises one or more domains from a capsid protein of that serotype and one or more domains from a capsid protein of a different serotype. For example, an AAV2 chimeric capsid protein includes a capsid protein comprising one or more domains of an AAV2 VP1, VP2, and/or VP3 capsid protein and one or more domains of a VP1, VP2, and/or VP3 capsid protein of a different AAV.

A "mosaic capsid" comprises at least two sets of VP1, VP2, and/or VP3 proteins, each set of which is encoded by a different *cap* gene.

In some embodiments, a mosaic capsid described herein comprises recombinant VP1, VP2, and/or VP3 proteins encoded by a *cap* gene genetically modified with an insertion of a nucleic acid sequence encoding a heterologous epitope, and further comprises VP1, VP2, and/or VP3 proteins encoded by a reference *cap* gene, e.g., a wildtype reference *cap* gene encoding the wildtype VP1, VP2, and/or VP3 proteins of the same AAV serotype as the recombinant VP1, VP2, and/or VP3 proteins, a control reference *cap* gene encoding VP1, VP2, and VP3 proteins identical to the recombinant VP1, VP2, and/or VP3 proteins but for the absence of the heterologous epitope, a mutated wildtype reference *cap* gene encoding substantially wildtype VP1, VP2, and/or VP3 proteins of the same AAV serotype as the recombinant VP1, VP2, and/or VP3 proteins but for a mutation (e.g., insertion, substitution, deletion), which mutation preferably reduces the tropism of the wildtype VP1, VP2, and VP3 proteins. In some embodiments, the reference *cap* gene encodes a chimeric VP1, VP2, and/or VP3 protein.

The phrase "heavy chain," or "immunoglobulin heavy chain" includes an immunoglobulin heavy chain sequence, including immunoglobulin heavy chain constant region sequence, from any organism. Heavy chain variable domains include three heavy chain CDRs and four FR regions, unless otherwise specified. Fragments of heavy chains include CDRs, CDRs and FRs, and combinations thereof. A typical heavy chain has, following the variable domain (from N-terminal to C-terminal), a C_{H}1 domain, a hinge, a C_{H}2 domain, and a C_{H}3 domain. A functional fragment of a heavy chain includes a fragment that is capable of specifically recognizing an first member of a specific binding pair (*e.g.*, recognizing the first member of a specific binding pair with a K_{D} in the micromolar, nanomolar, or picomolar range), that is capable of expressing and secreting from a cell, and that comprises at least one CDR. Heavy chain variable domains are encoded by variable region nucleotide sequence, which generally comprises V_{H}, D_{H}, and J_{H} segments derived from a repertoire of V_{H}, D_{H}, and J_{H} segments present in the germline. Sequences, locations and nomenclature for V, D, and J heavy chain segments for various organisms can be found in IMGT database, which is accessible via the internet on the world wide web (www) at the URL "imgt.org."

The term "heavy chain only antibody," "heavy chain only antigen binding protein," "single domain antigen binding protein," "single domain binding protein" or the like refers to a monomeric or homodimeric immunoglobulin molecule comprising an immunoglobulin-like chain comprising a variable domain operably linked to a heavy chain constant region, that is unable to associate with a light chain because the heavy chain constant region typically lacks a functional C_{H}1 domain. Accordingly, the term "heavy chain only antibody," "heavy chain only antigen binding protein," "single domain antigen binding protein," "single domain binding protein" or the like encompasses a both (i) a monomeric single domain antigen binding protein comprising one of the immunoglobulin-like chain comprising a variable domain operably linked to a heavy chain constant region lacking a functional C_{H}1 domain, or (ii) a homodimeric single domain antigen binding protein comprising two immunoglobulin-like chains, each of which comprising a variable domain operably linked to a heavy chain constant region lacking a functional C_{H}1 domain. In various aspects, a homodimeric single domain antigen binding protein comprises two identical immunoglobulin-like chains, each of which comprising an identical variable domain operably linked to an identical heavy chain constant region lacking a functional C_{H}1 domain. Additionally, each immunoglobulin-like chain of a single domain antigen binding protein comprises a variable domain, which may be derived from heavy chain variable region gene segments (e.g., V_{H}, D_{H}, J_{H}), light chain gene segments (e.g., V_{L}, J_{L}), or a combination thereof, linked to a heavy chain constant region (C_{H}) gene sequence comprising a deletion or inactivating mutation in a C_{H}1 encoding sequence (and, optionally, a hinge region) of a heavy chain constant region gene, e.g., IgG, IgA, IgE, IgD, or a combination thereof. A single domain antigen binding protein comprising a variable domain derived from heavy chain gene segments may be referred to as a "V_{H}- single domain antibody" or "V_{H}-single domain antigen binding protein", *see, e.g.,* U.S. Patent No. 8,754,287; U.S. Patent Publication Nos. 20140289876; 20150197553; 20150197554; 20150197555; 20150196015; 20150197556 and 20150197557, each of which is incorporated in its entirety by reference. A single domain antigen binding protein comprising a variable domain derived from light chain gene segments may be referred to as a or "V_{L}-single domain antigen binding protein," *see, e.g.,* U.S. Publication No. 20150289489, incorporated in its entirety by reference.

The phrase "light chain" includes an immunoglobulin light chain sequence from any organism, and unless otherwise specified includes human kappa (κ) and lambda (λ) light chains and a VpreB, as well as surrogate light chains. Light chain variable domains typically include three light chain CDRs and four framework (FR) regions, unless otherwise specified. Generally, a full-length light chain includes, from amino terminus to carboxyl terminus, a variable domain that includes FR1-CDR1-FR2-CDR2-FR3-CDR3-FR4, and a light chain constant region amino acid sequence. Light chain variable domains are encoded by the light chain variable region nucleotide sequence, which generally comprises light chain V_{L} and light chain J_{L} gene segments, derived from a repertoire of light chain V and J gene segments present in the germline. Sequences, locations and nomenclature for light chain V and J gene segments for various organisms can be found in IMGT database, which is accessible via the internet on the world wide web (www) at the URL "imgt.org." Light chains include those, *e.g.,* that do not selectively bind either a first or a second first member of a specific binding pair selectively bound by the first member of a specific binding pair-binding protein in which they appear. Light chains also include those that bind and recognize, or assist the heavy chain with binding and recognizing, one or more first member of a specific binding pairs selectively bound by the first member of a specific binding pair-binding protein in which they appear. Light chains also include those that bind and recognize, or assist the heavy chain with binding and recognizing, one or more first member of a specific binding pairs selectively bound by the first member of a specific binding pair-binding protein in which they appear. Common or universal light chains include those derived from a human Vκ1-39Jκ5 gene or a human Vκ3-20Jκ1 gene, and include somatically mutated (e.g., affinity matured) versions of the same.

The phrase "operably linked", as used herein, includes a physical juxtaposition (e.g., in three-dimensional space) of components or elements that interact, directly or indirectly with one another, or otherwise coordinate with each other to participate in a biological event, which juxtaposition achieves or permits such interaction and/or coordination. To give but one example, a control sequence (e.g., an expression control sequence) in a nucleic acid is said to be "operably linked" to a coding sequence when it is located relative to the coding sequence such that its presence or absence impacts expression and/or activity of the coding sequence. In many embodiments, "operable linkage" involves covalent linkage of relevant components or elements with one another. Those skilled in the art will readily appreciate that, in some embodiments, covalent linkage is not required to achieve effective operable linkage. For example, in some embodiments, nucleic acid control sequences that are operably linked with coding sequences that they control are contiguous with the nucleotide of interest. Alternatively or additionally, in some embodiments, one or more such control sequences acts in trans or at a distance to control a coding sequence of interest. In some embodiments, the term "expression control sequence" as used herein refers to polynucleotide sequences which are necessary and/or sufficient to effect the expression and processing of coding sequences to which they are ligated. In some embodiments, expression control sequences may be or comprise appropriate transcription initiation, termination, promoter and/or enhancer sequences; efficient RNA processing signals such as splicing and polyadenylation signals; sequences that stabilize cytoplasmic mRNA; sequences that enhance translation efficiency (e.g., Kozak consensus sequence); sequences that enhance protein stability; and/or, in some embodiments, sequences that enhance protein secretion. In some embodiments, one or more control sequences are preferentially or exclusively active in a particular host cell or organism, or type thereof. To give but one example, in prokaryotes, control sequences typically include promoter, ribosomal binding site, and transcription termination sequence; in eukaryotes, in many embodiments, control sequences typically include promoters, enhancers, and/or transcription termination sequences. Those of ordinary skill in the art will appreciate from context that, in many embodiments, the term "control sequences" refers to components whose presence is essential for expression and processing, and in some embodiments includes components whose presence is advantageous for expression (including, for example, leader sequences, targeting sequences, and/or fusion partner sequences).

The term "recombinant capsid protein" includes a capsid protein that has at least one mutation in comparison to the corresponding capsid protein of the wild-type virus, which wild-type may be a reference and/or control virus for comparative study. A recombinant capsid protein includes a capsid protein that comprises a heterologous amino acid sequence, which may be inserted into and/or displayed by the capsid protein. "Heterologous" in this context means heterologous as compared to the virus, from which the capsid protein is derived. The inserted amino acids can simply be inserted between two given amino acids of the capsid protein. An insertion of amino acids can also go along with a deletion of given amino acids of the capsid protein at the site of insertion, e.g. 1 or more capsid protein amino acids are substituted by 5 or more heterologous amino acids).

"Retargeting" or "redirecting" may include a scenario in which the wildtype vector targets several cells within a tissue and/or several organs within an organism, which general targeting of the tissue or organs is reduced to abolished by insertion of the heterologous epitope, and which retargeting to more a specific cell in the tissue or a specific organ in the organism is achieved with the targeting ligand that binds a marker expressed by the specific cell. Such retargeting or redirecting may also include a scenario in which the wildtype vector targets a tissue, which targeting of the tissue is reduced to abolished by insertion of the heterologous epitope, and which retargeting to a completely different tissue is achieved with the targeting ligand.

"Specific binding pair," "protein:protein binding pair" and the like includes two proteins (e.g., a first member (e.g., a first polypeptide) and a second cognate member (e.g., a second polypeptide)) that interact to form a covalent isopeptide bond bond under conditions that enable or facilitate isopeptide bond formation, wherein the term "cognate" refers to components that function together, i.e. to react together to form an isopeptide bond. Thus, two proteins that react together efficiently to form an isopeptide bond under conditions that enable or facilitate isopeptide bond formation can also be referred to as being a "complementary" pair of peptide linkers. Specific binding pairs capable of interacting to form a covalent isopeptide bond are reviewed in Veggiani et al. (2014) *Trends Biotechnol.* 32:506, and include peptide:peptide binding pairs such as SpyTag:SpyCatcher, SpyTag002:SpyCatcher002; SpyTag:KTag; isopeptag:pilin C, SnoopTag:SnoopCatcher, etc. Generally, a peptide tag refers to member of a protein:protein binding pair, which is generally less than 30 amino acids in length, and which forms a covalent isopeptide bond with the second cognate protein, wherein the second cognate protein is generally larger, but may also be less than 30 amino acids in length such as in the SpyTag:KTag sytem.

The term "isopeptide bond" refers to an amide bond between a carboxyl or carboxamide group and an amino group at least one of which is not derived from a protein main chain or alternatively viewed is not part of the protein backbone. An isopeptide bond may form within a single protein or may occur between two peptides or a peptide and a protein. Thus, an isopeptide bond may form intramolecularly within a single protein or intermolecularly i.e. between two peptide/protein molecules, e.g. between two peptide linkers. Typically, an isopeptide bond may occur between a lysine residue and an asparagine, aspartic acid, glutamine, or glutamic acid residue or the terminal carboxyl group of the protein or peptide chain or may occur between the alpha-amino terminus of the protein or peptide chain and an asparagine, aspartic acid, glutamine or glutamic acid. Each residue of the pair involved in the isopeptide bond is referred to herein as a reactive residue. In preferred embodiments of the invention, an isopeptide bond may form between a lysine residue and an asparagine residue or between a lysine residue and an aspartic acid residue. Particularly, isopeptide bonds can occur between the side chain amine of lysine and carboxamide group of asparagine or carboxyl group of an aspartate.

The SpyTag:SpyCatcher system is described in U.S. Patent No. 9,547,003 and Zakeri et al. (2012) PNAS 109:E690-E697, each of which is incorporated herein in its entirety by reference, and is derived from the CnaB2 domain of the *Streptococcus pyogenes* fibronecting-binding protein FbaB. By splitting the domain, Zakeri et al. obtained a peptide "SpyTag" having the sequence AHIVMVDAYKPTK (SEQ ID NO:1) which forms an amide bond to its cognate protein "SpyCatcher," an 112 amino acid polypeptide having the amino acid sequence set forth in SEQ ID NO:3. (Zakeri (2012), *supra).* An additional specific binding pair derived from CnaB2 domain is SpyTag:KTag, which forms an isopeptide bond in the presence of SpyLigase. (Fierer (2014) *PNAS* 111:E1176-1181) SpyLigase was engineered by excising the β strand from SpyCatcher that contains a reactive lysine, resulting in KTag, 10-residue peptide tag having the amino acid sequence ATHIKFSKRD (SEQ ID NO:2). The SpyTag002:SpyCatcher002 system is described in Keeble et al (2017) Angew Chem Int Ed Engl 56:16521-25, incorporated herein in its entirety by reference. SpyTag002 has the amino acid sequence VPTIVMVDAYKRYK, set forth as SEQ ID NO:54, and binds SpyCatcher002 (SEQ ID NO:55).

The SnoopTag: SnoopCatcher system is described in Veggiani (2016) PNAS 113:1202-07. The D4 Ig-like domain of RrgA, an adhesion from *Streptococcus pneumoniae,* was split to form SnoopTag (residues 734-745; SEQ ID NO:5) and SnoopCatcher (residues 749-860). Incubation of SnoopTag and SnoopCatcher results in a spontaneous isopeptide bond that is specific between the complementary proteins. Veggiani (2016)), *supra.*

The isopeptag:pilin-C specific binding pair was derived from the major pilin protein Spy0128 from *Streptococcus pyogenes.* (Zakeir and Howarth (2010) J. Am. Chem. Soc. 132:4526-27). Isopeptag has the amino acid sequence TDKDMTITFTNKKDAE, set forth as SEQ ID NO:7, and binds pilin-C (residues 18-299 of Spy0128). Incubation of SnoopTag and SnoopCatcher results in a spontaneous isopeptide bond that is specific between the complementary proteins. Zakeir and Howarth (2010), *supra.*

The term "peptide tag" includes polypeptides that are (1) heterologous to the protein which is tagged with the peptide tag, (2) a member of a specific protein:protein binding pair capable of forming an isopeptide bond, and (3) no more than 50 amino acids in length.

The term "target cells" includes any cells in which expression of a nucleotide of interest is desired. Preferably, target cells exhibit a receptor on their surface that allows the cell to be targeted with a targeting ligand, as described below.

The term "transduction" or "infection" or the like refers to the introduction of a nucleic acid into a target cell nucleus by a viral vector. The term efficiency in relation to transduction or the like, e.g., "transduction efficiency" refers to the fraction (e.g., percentage) of cells expressing a nucleotide of interest after incubation with a set number of viral vectors comprising the nucleotide of interest. Well-known methods of determining transduction efficiency include fluorescence activated cell sorting of cells transduced with a fluorescent reporter gene, PCR for expression of the nucleotide of interest, etc.

The term "wild-type", as used herein, includes an entity having a structure and/or activity as found in nature in a "normal" (as contrasted with mutant, diseased, altered, etc.) state or context. Those of ordinary skill in the art will appreciate that wildtype viral vectors, e.g.,wild-type capsid proteins, may be used as reference viral vector in comparative studies. Generally, a reference viral capsid protein/capsid/vector are identical to the test viral capsid protein/capsid/vector but for the change for which the effect is to be tested. For example, to determine the effect, e.g., on transduction efficiency, of inserting a first member of a specific binding pair into a test viral vector, the transduction efficiencies of the test viral vector (in the absence or presence of an appropriate targeting ligand) can be compared to the transduction efficiencies of a reference viral vector (in the absence or presence of an appropriate targeting ligan if necessary) which is identical to the test viral vector in every instance (e.g., additional mutations, nucleotide of interest, numbers of viral vectors and target cells, etc.) except for the presence of a first member of a specific binding pair.

The retargeting strategy described herein provides the advantages of both the scaffold and direct recombinatorial approaches described above, as well as resolves many of the disadvantages inherent in both. The strategy utilizes a specific binding pair, wherein the first member and second cognate member specifically bind to each other, and upon binding, form a covalent bond that permanently links the viral particle to any targeting ligand that is fused with the cognate member. With such a genetically modified viral particle, the tropism is maintained so long as the viral capsid remains intact, e.g., one advantage of the system provided herein compared to other scaffolding approaches is the permanence with which the "adaptor", e.g., targeting ligand, is bound to the recombinant viral particle similar to a direct recombinatorial approach. However, in contrast to the direct recombinatorial approach, the system described herein maintains the flexibility of the scaffolding adaptor approaches in that the recombinant viral particle can remain constant with the variability being found in the adaptor, e.g., the cognate member may be fused to differing targeting ligands and the different fusion proteins then coupled to the viral particle in accordance to the target cell.

### Recombinant virus capsid proteins and viral vectors and nucleic acids

Provided herein is a recombinant viral particle (e.g., a viral capsid protein, and a recombinant viral capsid and/or a recombinant viral vector that comprises the recombinant viral capsid protein) that is genetically modified to display a heterologous amino acid sequence comprising a first member of a specific binding pair, wherein the amino acid sequence is less than 50 amino acids in length, and wherein the recombinant viral capsid/particle protein exhibits reduced to abolished natural tropism. In some embodiments, the viral particle further comprises a second cognate member of the specific binding pair, wherein the first and second members are covalently bonded, and wherein the second member is fused to a targeting ligand.

In some embodiments, the heterologous amino acid sequence comprises a first member of a specific binding pair and one or more linkers. In some embodiments, the heterologous amino acid sequence comprises a first member of a specific binding pair flanked by a linker, e.g., the heterologous amino acid sequence comprises from N-terminus to C-terminus a first linker, a first member of a specific binding pair, and a second linker. In some embodiments, the first and second linkers are each independently at least one amino acid in length. In some embodiments, the first and second linkers are identical.

Generally, a heterologous amino acid sequence as described herein, e.g., comprising a first member of a specific binding pair by itself or in combination with one or more linkers, is between about 5 amino acids to about 50 amino acids in length. **In** some embodiments, the heterologous amino acid sequence is at least 5 amino acids in length. In some embodiments, the heterologous amino acid sequence is 6 amino acids in length. In some embodiments, the heterologous amino acid sequence is 7 amino acids in length. In some embodiments, the heterologous amino acid sequence is 8 amino acids in length. In some embodiments, the heterologous amino acid sequence is 9 amino acids in length. In some embodiments, the heterologous amino acid sequence is 10 amino acids in length. In some embodiments, the heterologous amino acid sequence is 11 amino acids in length. In some embodiments, the heterologous amino acid sequence is 12 amino acids in length. In some embodiments, the heterologous amino acid sequence is 13 amino acids in length. In some embodiments, the heterologous amino acid sequence is 14 amino acids in length. In some embodiments, the heterologous amino acid sequence is 15 amino acids in length. In some embodiments, the heterologous amino acid sequence is 16 amino acids in length. In some embodiments, the heterologous amino acid sequence is 17 amino acids in length. In some embodiments, the heterologous amino acid sequence is 18 amino acids in length. In some embodiments, the heterologous amino acid sequence is 19 amino acids in length. In some embodiments, the heterologous amino acid sequence is 20 amino acids in length. In some embodiments, the heterologous amino acid sequence is 21 amino acids in length. In some embodiments, the heterologous amino acid sequence is 22 amino acids in length. In some embodiments, the heterologous amino acid sequence is 23 amino acids in length. In some embodiments, the heterologous amino acid sequence is 24 amino acids in length. In some embodiments, the heterologous amino acid sequence is 25 amino acids in length. In some embodiments, the heterologous amino acid sequence is 26 amino acids in length. In some embodiments, the heterologous amino acid sequence is 27 amino acids in length. In some embodiments, the heterologous amino acid sequence is 28 amino acids in length. In some embodiments, the heterologous amino acid sequence is 29 amino acids in length. In some embodiments, the heterologous amino acid sequence is 30 amino acids in length. In some embodiments, the heterologous amino acid sequence is 31 amino acids in length. In some embodiments, the heterologous amino acid sequence is 32 amino acids in length. In some embodiments, the heterologous amino acid sequence is 33 amino acids in length. In some embodiments, the heterologous amino acid sequence is 34 amino acids in length. In some embodiments, the heterologous amino acid sequence is 35 amino acids in length. In some embodiments, the heterologous amino acid sequence is 36 amino acids in length. In some embodiments, the heterologous amino acid sequence is 37 amino acids in length. In some embodiments, the heterologous amino acid sequence is 38 amino acids in length. In some embodiments, the heterologous amino acid sequence is 39 amino acids in length. In some embodiments, the heterologous amino acid sequence is 40 amino acids in length. In some embodiments, the heterologous amino acid sequence is 41 amino acids in length. In some embodiments, the heterologous amino acid sequence is 42 amino acids in length. In some embodiments, the heterologous amino acid sequence is 43 amino acids in length. In some embodiments, the heterologous amino acid sequence is 44 amino acids in length. In some embodiments, the heterologous amino acid sequence is 45 amino acids in length. In some embodiments, the heterologous amino acid sequence is 46 amino acids in length. In some embodiments, the heterologous amino acid sequence is 47 amino acids in length. In some embodiments, the heterologous amino acid sequence is 48 amino acids in length. In some embodiments, the heterologous amino acid sequence is 49 amino acids in length. In some embodiments, the heterologous amino acid sequence is 50 amino acids in length.

In some embodiments, the specific binding pair is a SpyTag: SpyCatcher binding pair, wherein the first member is SpyTag, and wherein the second cognate member is SpyCatcher. In some embodiments, the specific binding pair is SpyTag:KTag, wherein the first member is SpyTag and wherein the second cognate member is KTag. In some embodiments, the specific binding pair is SpyTag:KTag, wherein the first member is KTag and wherein the second cognate member is SpyTag. In some embodiments, the specific binding pair is isopeptag:pilin-C, wherein the first member is isopeptag, and wherein the second cognate member is pilin-C, or a portion thereof. In some embodiments, the specific binding pair is SnoopTag:SnoopCatcher, and the first member is SnoopTag, and the second cognate member is SnoopCatcher.

In some embodiments, a recombinant viral capsid protein as described herein is an Ad capsid protein, e.g., a capsid protein of an Ad serotype selected from the group consisting of Ad1, Ad2, Ad3, Ad4, Ad5, Ad6, and Ad7. In some embodiments, a recombinant viral capsid protein is derived from an Ad2 capsid gene. In some embodiments, a recombinant viral capsid protein is derived from an Ad5 capsid gene. In some embodiments, a recombinant Ad viral capsid protein as described herein comprises a first member of a specific binding pair in a fiber protein domain, e.g., at the carboxy terminus of the fiber protein, fiber knob, and/or HI loop of the fiber knob.

In some embodiments, a recombinant viral capsid protein described herein is derived from an adeno-associated virus (AAV) capsid protein gene, e.g., a capsid gene of an AAV serotype selected from the group consisting of AAV1, AAV2, AAV3, AAV4, AAV5, AAV6, AAV7, AAV8 and AAV9. In some embodiments, the recombinant viral capsid protein is derived from an AAV2 capsid gene or an AAV9 capsid gene. In some embodiments, the recombinant viral capsid protein is a genetically modified AAV2 VP1 capsid protein, the wildtype amino acid sequence of which is set forth as SEQ ID NO:9. In some embodiments the recombinant viral capsid protein is a genetically modified AAV9 VP1 capsid protein, the wildtype amino acid sequence of which is set forth as SEQ ID NO:31.

Generally, a recombinant viral capsid protein as described herein comprises a first member of a specific binding pair inserted into and/or displayed by the capsid protein such that the first member of a specific binding pair reduces and/or abolishes the natural tropism of the capsid protein or capsid comprising same. In some embodiments, the first member of a specific binding pair is inserted into a region of the capsid protein involved with the natural tropism of the wildtype reference capsid protein, e.g., a region of the capsid protein involved with cell receptor. In some embodiments, the first member of a specific binding pair is inserted into and/or displayed by a knob domain of an Ad fiber protein. In some embodiments, the first member of a specific binding pair is inserted into and/or displayed by the HI loop of an Ad fiber protein. In some embodiments, the first member of a specific binding pair is inserted after an amino acid position selected from the group consisting of G453 of AAV2 capsid protein VP1, N587 of AAV2 capsid protein VP1, G453 of AAV9 capsid protein VP1, and A589 of AAV9 capsid protein VP1. In some embodiments, the first member of a specific binding pair is inserted and/or displayed between amino acids N587 and R588 of an AAV2 VP1 capsid. In some embodiments, a recombinant viral capsid, viral vector comprising a recombinant viral capsid, and/or compositions comprising a recombinant viral capsid comprises an amino acid sequence set forth as SEQ ID NO: 13, 15, 17, 19, 21, 23, 25, 27, 29, 35, 37, or 39. Additional suitable insertion sites identified by using AAV2 are well known in the art (Wu et al. (2000) J. Virol. 74:8635-8647) and include I-1, I-34, 1-138, 1-139, 1-161, 1-261, 1-266, I-381, I-447, I-448, I-459, 1-471, 1-520, 1-534, 1-570, 1-573, 1-584, 1-587, I-588, I-591, I-657, I-664, I-713 and I-716. A recombinant virus capsid protein as described herein may be an AAV2 capsid protein comprising a first member of a specific binding pair inserted into a position selected from the group consisting of I-1, I-34, I-138, I-139, I-161, I-261, I-266, I-381, I-447, I-448, I-459, I-471, I-520, I-534, I-570, I-573, I-584, I-587, I-588, I-591, I-657, I-664, I-713, I-716, and a combination thereof. Additional suitable insertion sites identified by using additional AAV serotypes are well-known and include I-587 (AAV1), I-589 (AAV1), I-585 (AAV3), I-585 (AAV4), and I-585 (AAV5). In some embodiments, a recombinant virus capsid protein as described herein may be an AAV2 capsid protein comprising a first member of a specific binding pair inserted into a position selected from the group consisting of I-587 (AAV1), I-589 (AAV1), I-585 (AAV3), I-585 (AAV4), I-585 (AAV5), and a combination thereof.

The used nomenclature I-### herein refers to the insertion site with ### naming the amino acid number relative to the VP1 protein of an AAV capsid protein, however such the insertion may be located directly N- or C-terminal, preferably C-terminal of one amino acid in the sequence of 5 amino acids N- or C-terminal of the given amino acid, preferably 3, more preferably 2, especially 1 amino acid(s) N- or C-terminal of the given amino acid. Additionally, the positions referred to herein are relative to the VP1 protein encoded by an AAV capsid gene, and corresponding positions (and mutations thereof) may be easily identified for the VP2 and VP3 capsid proteins encoding by the capsid gene by performing a sequence alignment of the VP1, VP2 and VP3 proteins encoding by the reference AAV capsid gene.

Accordingly, an insertion into the corresponding position of the coding nucleic acid of one of these sites of the cap gene leads to an insertion into VP1, VP2 and/or VP3, as the capsid proteins are encoded by overlapping reading frames of the same gene with staggered start codons. Therefore, for AAV2, for example, according to this nomenclature insertions between amino acids 1 and 138 are only inserted into VP1, insertions between 138 and 203 are inserted into VP1 and VP2, and insertions between 203 and the C-terminus are inserted into VP1, VP2 and VP3, which is of course also the case for the insertion site I-587. Therefore, the present invention encompasses structural genes of AAV with corresponding insertions in the VP1, VP2 and/or VP3 proteins.

Additionally, due to the high conservation of at least large stretches and the large member of closely related family member, the corresponding insertion sites for AAV other than the enumerated AAV can be identified by performing an amino acid alignment or by comparison of the capsid structures. *See, e.g.,* Rutledge et al. (1998) J. Virol. 72:309-19 and U.S. Patent No. 9,624,274 for exemplary alignments of different AAV capsid proteins, each of which reference is incorporated herein by reference in its entirety.

In some embodiments, insertion (display) of the first member of a specific binding pair reduces or abolishes the natural tropism of the viral vector, e.g., transduction of a cell naturally permissive to infection by wildtype reference viral vectors and/or a target cell is undetectable in the absence of a covalent bond with the second cognate member of the binding pair, which is fused to an appropriate targeting ligand. In some embodiments, insertion (display) of the first member of a specific binding pair reduces the natural tropism of the viral vector, e.g., compared to transduction of a cell naturally permissive to infection by wildtype reference viral vectors. In some embodiments, the insertion (display) of the first member of a specific binding pair reduces the natural tropism of the viral vector by at least 5%. In some embodiments, the insertion (display) of the first member of a specific binding pair reduces the natural tropism of the viral vector by at least 5%. In some embodiments, the insertion (display) of the first member of a specific binding pair reduces the natural tropism of the viral vector by at least 10%. In some embodiments, the insertion (display) of the first member of a specific binding pair reduces the natural tropism of the viral vector by at least 20%. In some embodiments, the insertion (display) of the first member of a specific binding pair reduces the natural tropism of the viral vector by at least 30%. In some embodiments, the insertion (display) of the first member of a specific binding pair reduces the natural tropism of the viral vector by at least 40%. In some embodiments, the insertion (display) of the first member of a specific binding pair reduces the natural tropism of the viral vector by at least 50%. In some embodiments, the insertion (display) of the first member of a specific binding pair reduces the natural tropism of the viral vector by at least 60%. In some embodiments, the insertion (display) of the first member of a specific binding pair reduces the natural tropism of the viral vector by at least 70%. In some embodiments, the insertion (display) of the first member of a specific binding pair reduces the natural tropism of the viral vector by at least 80%. In some embodiments, the insertion (display) of the first member of a specific binding pair reduces the natural tropism of the viral vector by at least 90%. In some embodiments, the insertion (display) of the first member of a specific binding pair reduces the natural tropism of the viral vector by at least 95%. In some embodiments, the insertion (display) of the first member of a specific binding pair reduces the natural tropism of the viral vector by at least 90%. In the embodiments wherein the insertion (display) of the first member of a specific binding pair does not completely abolish the natural tropism of the recombinant viral capsids, the natural tropism of such recombinant viral capsids may be further reduced by a second and different mutation. For example, in one embodiment, a recombinant viral capsid protein as described herein may be derived from an AAV9 serotype, and may comprise a first member of a specific binding pair, and may further comprise a mutation, e.g., a W503A mutation.

This detargeting of the virus from its natural host cell is important especially if systemic versus local or loco-regional administration of the viral vectors is intended, as uptake of the viral vectors by the natural host cells limits the effective dose of the viral vectors. In case of AAV2 and AAV6, HSPG is reported to be the primary receptor for viral uptake in a large number of cells, especially liver cells. For AAV2 HSPG-binding activity is dependent on a group of 5 basic amino acids, R484, R487, R585, R588 and K532 (Kern et al., (2003) J Virol. 77(20): 11072-81). Accordingly, preferred point mutations are those that reduce the transducing activity of the viral vector for a given target cell mediated by the natural receptor by at least 50%, preferably at least 80%, especially at least 95%, in case of HSPG as a primary receptor for the binding of the viral vectors to target cells.

Consequently, further mutations preferred for HSPG-binding viral vectors are those mutations that delete or replace a basic amino acid such as R, K or H, preferably R or K which is involved in HSPG binding of the respective virus, by a non-basic amino acid such as A, D, G, Q, S and T, preferably A or an amino acid that is present at the corresponding position of a different but highly conserved AAV serotype lacking such basic amino acid at this position. Consequently preferred amino acid substitutions are R484A, R487A, R487G, K532A, K532D, R585A, R585S, R585Q, R585A or R588T, especially R585A and/or R588A for AAV2, and K531A or K531E for AAV6. One especially preferred embodiment of the invention are such capsid protein mutants of AAV2 that additionally contain the two point mutations R585A and R588A as these two point mutations are sufficient to ablate HSPG binding activity to a large extent. These point mutations enable an efficient detargeting from HSPG-expressing cells which- -for targeting purposes--increases specificity of the respective mutant virus for its new target cell.

### Targeting Ligands

A viral particle described herein may further comprise a second member of the specific binding pair that specifically forms a covalent bond with the first member of the specific binding pair that is inserted into/displayed by a recombinant viral capsid protein, wherein the second member is fused to a targeting ligand. In some embodiments, the targeting ligand binds a receptor expressed on the surface of a cell, e.g., a cell surface protein on a (human) eukaryotic cell, e.g., a target cell. In some embodiments the targeting ligand binds a receptor expressed primarily (e.g., solely) by (human) liver cells. In some embodiments the targeting ligand binds a receptor expressed primarily (e.g., solely) by (human) brain cells. In some embodiments the targeting ligand binds a receptor expressed primarily (e.g., solely) by (human) lymphocytes. In some embodiments the targeting ligand binds a receptor expressed primarily (e.g., solely) by (human) T cells. In some embodiments the targeting ligand binds a receptor expressed primarily (e.g., solely) by (human) B cells. In some embodiments the targeting ligand binds a receptor expressed primarily (e.g., solely) by (human) dendritic cells. In some embodiments the targeting ligand binds a receptor expressed primarily (e.g., solely) by (human) macrophages. In some embodiments the targeting ligand binds a receptor expressed primarily (e.g., solely) by (human) NK cells. In some embodiments the targeting ligand binds a receptor expressed primarily (e.g., solely) by (human) kidney cells. In some embodiments the targeting ligand binds a receptor expressed primarily (e.g., solely) by a (human) cancerous cell. In some embodiments the targeting ligand binds a receptor expressed primarily (e.g., solely) by (human) cell infected with heterologous pathogen.

There are a large number of cell surface proteins, e.g., cell surface receptors, suitable which may be targeted by a targeting ligand, and for which a targeting ligand, e.g., antibodies or portions thereof, are already available. Such structures include, but are not limited to: the class I and class II Major Histocompatibility Antigens; receptors for a variety of cytokines (e.g., receptors for IL-1, IL-4, IL-6, IL-13, IL-22, IL-25, IL-33, etc.), cell-type specific growth hormones, brain derived neurotrophic factor (BDNF), ciliary neurotrophic factor (CTNF), colony stimulating growth factors, endothelial growth factors, epidermal growth factors, fibroblast growth factors, glially derived neurotrophic factor, glial growth factors, gro-beta/mip 2, hepatocyte growth factors, insulin-like growth factor, interferons (α-IFN, β-IFN, γIFN, consensus IFN), interleukins (IL-1, IL-2, IL-3, IL-4, IL-5, IL-6, IL-7, IL-8, IL-9, IL-10, IL-11, IL-12, IL-13, IL-14), keratinocyte growth factor, leukemia inhibitory factors, macrophage/monocyte chemotactic activating factor, nerve growth factor, neutrophil activating protein 2, platelet derived growth factor, stem cell factor, transforming growth factor, tumor necrosis factors. vascular endothial growth factor, lipoproteins, including additional or other type 1 transmembrane receptors such as PRLR, G-protein coupled receptors such as GCGR, ion channels such as Nav1.7, ASIC1 or ASIC2; cell adhesion molecules; transport molecules for metabolites such as amino acids; the antigen receptors of B- and T-lymphocytes (e.g., B cell receptors and associated proteins (e.g., CD19, CD20, etc.) and T cell receptors and associated proteins (e.g., CD3, CD4, CD8, etc.); a tetraspanin protein (e.g., CD63). A recombinant viral capsid described herein allows for the specific infection of a cell type by employing a targeting ligand that binds differentiation cell surface antigens as targets for the viral vector complex.

In some embodiments the targeting ligand binds a protein expressed primarily (e.g., solely) by (human) liver cells, i.e., a liver specific marker. In some embodiments the targeting ligand binds a protein expressed primarily (e.g., solely) by (human) brain cells, a brain cell specific marker. In some embodiments the targeting ligand binds a protein expressed primarily (e.g., solely) by (human) hematopoietic cells, i.e., a hematopoietic cell specific marker. In some embodiments the targeting ligand binds a protein expressed primarily (e.g., solely) by (human) T cells, i.e., a T-cell specific marker. In some embodiments the targeting ligand binds a protein expressed primarily (e.g., solely) by (human) B cells, i.e., a B-cell specific marker. In some embodiments the targeting ligand binds a protein expressed primarily (e.g., solely) by (human) dendritic cells, i.e., a dendritic cell specific marker. In some embodiments the targeting ligand binds a protein expressed primarily (e.g., solely) by (human) macrophages, i.e, a macrophage specific marker. In some embodiments the targeting ligand binds a protein expressed primarily (e.g., solely) by (human) NK cells, i.e., an NK cell specific marker. In some embodiments the targeting ligand binds a protein expressed primarily (e.g., solely) by (human) kidney cells, i.e., a kidney specific marker. In some embodiments, the targeting ligand binds a receptor expressed primarily (e.g., solely) by (human) pancreas cells, i.e., a pancreas specific marker. In some embodiments, the targeting ligand binds a receptor expressed primarily (e.g., solely) by (human) intestinal cells, i.e., a intestine specific marker. In some embodiments the targeting ligand binds a protein expressed primarily (e.g., solely) by a (human) cancerous cell, i.e., a tumor associated antigen. In some embodiments the targeting ligand binds a protein expressed primarily (e.g., solely) by (human) cell infected with heterologous pathogen. Proteins that (1) are specifically expressed by, or for which expression is enriched in, a cell/tissue/organ, and (2) recognized by an antigen-binding protein useful as a targeting ligand as described herein are well-known and may also be found at www.proteinatlas.org; *see also* Uhlen et al. (2010) Nat. Biotech. 28:1248-50, incorporated herein in its entirety by reference. Table 2 below provides exemplary and non-limiting organ specific markers for which antigen-binding proteins, which may be useful as targeting ligands, are available and the cells/tissue/organ expressing such markers.

**Table 2 : Exemplary tissue specific markers**

| Tissue | Tissue Specific Markers |
|---|---|
| Liver | ATP binding cassette subfamily B; members 11 (ABCB11) |
| | Alanine-glyoxylate aminotransferase (AGXT) |
| | Alcohol dehydrogenase 1A, class I (ADH1A) |
| | Alchohol dehydrogenase 4 (class II) pi polypeptide (ADH4) |
| | Amyloid P component, serum (APCS) |
| | Angiopoietin like 3 (ANGPTL3) |
| | Apolipoprotein; C1, C2 (APOC1, APOC2) |
| | APOC4-APOC2 |
| | Asialoglycoprotein receptor 1 (ASGR1) |
| | Asialoglycoprotein receptor 2 (ASGR2) |
| | Bile acid-CoA: amino acid N-aceyltransferase (BAAT) |
| | Complement C8 beta chain (C8B) |
| | Coagulation factor II, thrombin (F2) |
| | Cytochrome P450 family 1 subfamily A member 2 CYP1A2 |
| | Mannose binding lectin 2 (MBL2) |
| | Soluble carrier organic anion transporter family member 1B3 (SLCO1B3) |
| | Paraoxonase 3 (PON3) |
| | Transferrin receptor 2 (TFR2) |
| | Urocanate hydratase 1 (UROC 1) |
| Intestine | Fatty acid binding protein 6 (FABP6) |
| Pancreas | CUB and zona pellucida like domains 1 (CUZD1) |
| | Protease, serine 2 (PRSS2) |
| | Protease, serine 3 (PRSS3) |

In some embodiments, the targeting ligand binds a receptor expressed by a (human) liver cell, e.g., an asialoglycoprotein receptor, e.g., hASGR1. In some embodiments, the targeting ligand binds a receptor expressed by a (human) brain cell. In some embodiments, the targeting ligand binds a receptor expressed by a (human) T cell, e.g., CD3, e.g., CD3ε. In some embodiments, the targeting ligand binds a receptor expressed by a (human) kidney cell, e.g., . In some embodiments, the targeting ligand binds a receptor expressed by a (human) muscle cell, e.g., an integrin. In some embodiments, the targeting ligand binds a receptor expressed by a (human) cancerous cell, e.g., a tumor associated antigen, e.g., E6 and E7. In some embodiments, the targeting ligand binds human glucagon receptor (hGCGR).

In some embodiments, the targeting ligand binds a tumor-associated antigen expressed by a tumor cell. Non-limiting examples of specific tumor-associated antigens include, e.g., AFP, ALK, BAGE proteins, β-catenin, brc-abl, BRCA1, BORIS, CA9, carbonic anhydrase IX, caspase-8, CCR5, CD19, CD20, CD30, CD40, CDK4, CEA, CTLA4, cyclin-B1, CYP1B1, EGFR, EGFRvIII, ErbB2/Her2, ErbB3, ErbB4, ETV6-AML, EpCAM, EphA2, Fra-1, FOLR1, GAGE proteins (e.g., GAGE-1, -2), GD2, GD3, GloboH, glypican-3, GM3, gp100, Her2, HLA/B-raf, HLA/k-ras, HLA/MAGE-A3, hTERT, LMP2, MAGE proteins (e.g., MAGE-1, -2, - 3, -4, -6, and -12), MART-1, mesothelin, ML-IAP, Muc1, Muc2, Muc3, Muc4, Muc5, Muc16 (CA-125), MUM1, NA17, NY-BR1, NY-BR62, NY-BR85, NY-ESO1, OX40, p15, p53, PAP, PAX3, PAX5, PCTA-1, PLAC1, PRLR, PRAME, PSMA (FOLH1), RAGE proteins, Ras, RGS5, Rho, SART-1, SART-3, Steap-1, Steap-2, survivin, TAG-72, TGF-β, TMPRSS2, Tn, TRP-1, TRP-2, tyrosinase, and uroplakin-3.

In some embodiments, the targeting ligand binds to CD markers associated with the immune response, e.g., CD3, CD4, CD8, CD19, CD20, etc.

One embodiment of the present invention is a multimeric structure comprising a recombinant viral capsid protein of the present invention. A multimeric structure comprises at least 5, preferably at least 10, more preferably at least 30, most preferably at least 60 recombinant viral capsid proteins comprising a first member of a specific binding pair as described herein. They can form regular viral capsids (empty viral particles) or viral vectors (capsids encapsulating a nucleotide of interest). The formation of viral vectors capable of packaging a viral genome is a highly preferred feature for use of the recombinant viral capsids described herein as viral vectors.

One embodiment of the present invention is a nucleic acid encoding a capsid protein as described above. The nucleic acid is preferably a vector comprising the claimed nucleic. Nucleic acids, especially vectors are necessary to recombinantly express the capsid proteins of this invention.

A further embodiment of the present invention is the use of at least one recombinant viral capsid protein and/or a nucleic acid encoding same, preferably at least one multimeric structure (e.g., viral vector) for the manufacture of and use as a gene transfer vector.

### Methods of Use and Making

A further embodiment of the recombinant viral capsid proteins described herein is their use for delivering a nucleotide of interest, e.g., a reporter gene or a therapeutic gene, to a target cell. Generally, a nucleotide of interest may be a transfer plasmid, which may generally comprise 5' and 3' inverted terminal repeat (ITR) sequences flanking the reporter gene(s) or therapeutic gene(s) (which may be under the control of a viral or non-viral promoter, when encompassed within an AAV vector. In one embodiment, a nucleotide of interest is a transfer plasmid comprising from 5' to 3': a 5' ITR, a promoter, a gene (e.g., a reporter and/or therapeutic gene) and a 3'ITR.

Non-limiting examples of useful promoters include, e.g., cytomegalovirus (CMV)-promoter, the spleen focus forming virus (SFFV)-promoter, the elongation factor 1 alpha (EF 1a)-promoter (the 1.2 kb EFla-promoter or the 0.2 kb EFla-promoter), the chimeric EF 1 a/IF4-promoter, and the phospho-glycerate kinase (PGK)-promoter. An internal enhancer may also be present in the viral construct to increase expression of the gene of interest. For example, the CMV enhancer (Karasuyama et al. 1989. J. Exp. Med. 169:13, which is incorporated herein by reference in its entirety) may be used. In some embodiments, the CMV enhancer can be used in combination with the chicken β-actin promoter.

A variety of reporter genes (or detectable moieties) can be encapsulated in a multimeric structure comprising the recombinant viral capsid proteins described herein. Exemplary reporter genes include, for example, β-galactosidase (encoded *lacZ* gene), Green Fluorescent Protein (GFP), enhanced Green Fluorescent Protein (eGFP), MmGFP, blue fluorescent protein (BFP), enhanced blue fluorescent protein (eBFP), mPlum, mCherry, tdTomato, mStrawberry, J-Red, DsRed, mOrange, mKO, mCitrine, Venus, YPet, yellow fluorescent protein (YFP), enhanced yellow fluorescent protein (eYFP), Emerald, CyPet, cyan fluorescent protein (CFP), Cerulean, T-Sapphire, luciferase, alkaline phosphatase, or a combination thereof. The methods described herein demonstrate the construction of targeting vectors that employ the use of a reporter gene that encodes green fluorescent protein, however, persons of skill upon reading this disclosure will understand that non-human animals described herein can be generated in the absence of a reporter gene or with any reporter gene known in the art.

A variety of therapeutic genes can also be encapsulated in the can be encapsulated in a multimeric structure comprising the recombinant viral capsid proteins described herein, e.g., as part of a transfer vector. Non-limiting examples of a therapeutic gene include those that encode a toxin (e.g., a suicide gene), a therapeutic antibody or fragment thereof, a CRISPR/Cas system or portion(s) thereof, antisense RNA, siRNA, shRNA, etc.

A further embodiment of the present invention is a process for the preparation of a recombinant capsid protein, the method comprising the steps of:
a) expressing a nucleic acid encoding the recombinant capsid protein under suitable conditions, and
b) isolating the expressed capsid protein of step a).

In some embodiments, a viral particle as described herein comprises a mosaic capsid, e.g., a capsid comprising capsid proteins genetically modified as described herein (in the absence or presence of a covalent bond with a targeting ligand) in a certain ratio with reference capsid proteins. A method for making such a mosaic viral particle comprises
a) expressing a nucleic acid encoding the recombinant capsid protein and a nucleotide encoding a reference capsid protein at a ratio (wt/wt) of 1:1 and 10:1 under suitable conditions, and
b) isolating the expressed capsid protein of step a).

Generally speaking, a mosaic capsid formed according to the method will be considered to have a modified capsid protein:reference capsid protein ratio similar to the ratio (wt:wt) of nucleic acids encoding same used to produce the mosaic capsid. Accordingly, in some embodiments, a composition described herein comprises, or a method described herein combines, a recombinant viral capsid protein and a reference capsid protein (or combination of reference capsid proteins) at a ratio that ranges from 1:1 to 1:15. In some embodiments, the ratio is 1:2. In some embodiments, the ratio is 1:3. In some embodiments, the ratio is 1:4. In some embodiments, the ratio is 1:5. In some embodiments, the ratio is 1:6. In some embodiments, the ratio is 1:7. In some embodiments, the ratio is 1:8. In some embodiments, the ratio is 1:9. In some embodiments, the ratio is 1:10. In some embodiments, the ratio is 1:11. In some embodiments, the ratio is 1:12. In some embodiments, the ratio is 1:13. In some embodiments, the ratio is 1:14. In some embodiments, the ratio is 1:15.

Further embodiments of the present invention is a method for altering the tropism of a virus, the method comprising the steps of: (a) inserting a nucleic acid encoding a heterologous amino acid sequence into a nucleic acid sequence encoding an viral capsid protein to form a nucleotide sequence encoding a genetically modified capsid protein comprising the heterologous amino acid sequence and/or (b) culturing a packaging cell in conditions sufficient for the production of viral vectors, wherein the packaging cell comprises the nucleic acid. A further embodiment of the present invention is a method for displaying a targeting ligand on the surface of a capsid protein, the method comprising the steps of: (a) expressing a nucleic acid encoding a recombinant viral capsid protein as described herein (and optionally with a nucleotide encoding a reference capsid protein) under suitable conditions, wherein the nucleic acid encodes a capsid protein comprising a first member of a specific binding pair, (b) isolating the expressed capsid protein comprising a first member of a specific binding pair of step (a) or capsid comprising same, and (c) incubating the capsid protein or capsid with a second cognate member of the specific binding pair under conditions suitable for allowing the formation of an isopeptide bond between the first and second member, wherein the second cognate member of the specific binding pair is fused with a targeting ligand.

In some embodiments, the packaging cell further comprises a helper plasmid and/or a transfer plasmid comprising a nucleotide of interest. In some embodiments, the methods further comprise isolating self-complementary adeno-associated viral vectors from culture supernatant. In some embodiments, the methods further comprise lysing the packaging cell and isolating single-stranded adeno-associated viral vectors from the cell lysate. In some embodiments, the methods further comprise (a) clearing cell debris, (b) treating the supernatant containing viral vectors with nucleases, e.g., DNase I and MgCl2, (c) concentrating viral vectors, (d) purifying the viral vectors, and (e) any combination of (a)-(d).

Packaging cells useful for production of the viral vectors described herein include, e.g., animal cells permissive for the virus, or cells modified so as to be permissive for the virus; or the packaging cell construct, for example, with the use of a transformation agent such as calcium phosphate. Non-limiting examples of packaging cell lines useful for producing viral vectors described herein include, e.g., human embryonic kidney 293 (HEK-293) cells (e.g., American Type Culture Collection [ATCC] No. CRL-1573), HEK-293 cells that contain the SV40 Large T-antigen (HEK-293T or 293T), HEK293T/17 cells, human sarcoma cell line HT-1080 (CCL-121), lymphoblast-like cell line Raji (CCL-86), glioblastoma-astrocytoma epithelial-like cell line U87-MG (HTB-14), T-lymphoma cell line HuT78 (TIB-161), NIH/3T3 cells, Chinese Hamster Ovary cells (CHO) (e.g., ATCC Nos. CRL9618, CCL61, CRL9096), HeLa cells (e.g., ATCC No. CCL-2), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RATI cells, mouse L cells (ATCC No. CCLI.3), HLHepG2 cells, CAP cells, CAP-T cells, and the like.

L929 cells, the FLY viral packaging cell system outlined in Cosset et al (1995) J Virol 69,7430-7436, NS0 (murine myeloma) cells, human amniocytic cells (e.g., CAP, CAP-T), yeast cells (including, but not limited to, S. cerevisiae, Pichia pastoris), plant cells (including, but not limited to, Tobacco NT1 , BY-2), insect cells (including but not limited to SF9, S2, SF21, Tni (e.g. High 5)) or bacterial cells (including, but not limited to, E. coli).

For additional packaging cells and systems, packaging techniques and vectors for packaging the nucleic acid genome into the pseudotyped viral vector see, for example, Polo, et al, Proc Natl Acad Sci USA, (1999) 96:4598-4603. Methods of packaging include using packaging cells that permanently express the viral components, or by transiently transfecting cells with plasmids.

Further embodiments include methods of redirecting a virus and/or delivering a reporter or therapeutic gene to a target cell, the method comprising a method for transducing cells *in vitro* or *in vivo,* the method comprising the steps of: contacting the target cell with a viral vector comprising a capsid described herein, wherein the capsid comprises a targeting ligand that specifically binds a receptor expressed by the target cell. In some embodiments, the target cell is *in vitro.* In other embodiments, the target cell is *in vivo* in a subject, e.g., a human.

### Target Cells

A wide variety of cells may be targeted in order to deliver a nucleotide of interest using a recombinant viral vector as disclosed herein. The target cells will generally be chosen based upon the nucleotide of interest and the desired effect.

In some embodiments, a nucleotide of interest may be delivered to enable a target cell to produce a protein that makes up for a deficiency in an organism, such as an enzymatic deficiency, or immune deficiency, such as X-linked severe combined immunodeficiency. Thus, in some embodiments, cells that would normally produce the protein in the animal are targeted. In other embodiments, cells in the area in which a protein would be most beneficial are targeted.

In other embodiments, a nucleotide of interest, such as a gene encoding an siRNA, may inhibit expression of a particular gene in a target cell. The nucleotide of interest may, for example, inhibit expression of a gene involved in a pathogen life cycle. Thus cells susceptible to infection from the pathogen or infected with the pathogen may be targeted. In other embodiments, a nucleotide of interest may inhibit expression of a gene that is responsible for production of a toxin in a target cell.

In other embodiments a nucleotide of interest may encode a toxic protein that kills cells in which it is expressed. In this case, tumor cells or other unwanted cells may be targeted.

In still other embodiments a nucleotide of interest that encodes a therapeutic protein.

Once a particular population of target cells is identified in which expression of a nucleotide of interest is desired, a target receptor is selected that is specifically expressed on that population of target cells. The target receptor may be expressed exclusively on that population of cells or to a greater extent on that population of cells than on other populations of cells. The more specific the expression, the more specifically delivery can be directed to the target cells. Depending on the context, the desired amount of specificity of the marker (and thus of the gene delivery) may vary. For example, for introduction of a toxic gene, a high specificity is most preferred to avoid killing non-targeted cells. For expression of a protein for harvest, or expression of a secreted product where a global impact is desired, less marker specificity may be needed.

As discussed above, the target receptor may be any receptor for which a targeting ligand can be identified or created. Preferably the target receptor is a peptide or polypeptide, such as a receptor. However, in other embodiments the target receptor may be a carbohydrate or other molecule that can be recognized by a binding partner. If a binding partner, e.g., ligand, for the target receptor is already known, it may be used as the affinity molecule. However, if a binding molecule is not known, antibodies to the target receptor may be generated using standard procedures. The antibodies can then be used as a targeting ligand.

Thus, target cells may be chosen based on a variety of factors, including, for example, (1) the particular application (e.g., therapy, expression of a protein to be collected, and conferring disease resistance) and (2) expression of a marker with the desired amount of specificity.

Target cells are not limited in any way and include both germline cells and cell lines and somatic cells and cell lines. Target cells can be stem cells derived from either origin. When the target cells are germline cells, the target cells are preferably selected from the group consisting of single-cell embryos and embryonic stem cells (ES).

### Pharmaceutical compositions, dosage forms and administration

A further embodiment provides a medicament comprising at least one recombinant viral capsid protein and appropriate targeting ligand according to this invention and/or a nucleic acid according to this invention. Preferably such medicament is useful a gene transfer particle.

Also disclosed herein are pharmaceutical compositions comprising the viral particles described herein and a pharmaceutically acceptable carrier and/or excipient. In addition, disclosed herein are pharmaceutical dosage forms comprising the viral particle described herein.

As discussed herein, the viral particles described herein can be used for various therapeutic applications (in vivo and ex vivo) and as research tools.

Pharmaceutical compositions based on the viral particles disclosed herein can be formulated in any conventional manner using one or more physiologically acceptable carriers and/or excipients. The viral particles may be formulated for administration by, for example, injection, inhalation or insulation (either through the mouth or the nose) or by oral, buccal, parenteral or rectal administration, or by administration directly to a tumor.

The pharmaceutical compositions can be formulated for a variety of modes of administration, including systemic, topical or localized administration. Techniques and formulations can be found in, for example, Remrnington's Pharmaceutical Sciences, Meade Publishing Co., Easton, Pa. For systemic administration, injection is preferred, including intramuscular, intravenous, intraperitoneal, and subcutaneous. For the purposes of injection, the pharmaceutical compositions can be formulated in liquid solutions, preferably in physiologically compatible buffers, such as Hank's solution or Ringer's solution. In addition, the pharmaceutical compositions may be formulated in solid form and redissolved or suspended immediately prior to use. Lyophilized forms of the pharmaceutical composition are also suitable.

For oral administration, the pharmaceutical compositions may take the form of, for example, tablets or capsules prepared by conventional means with pharmaceutically acceptable excipients such as binding agents (e.g. pregelatinized maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulfate). The tablets can also be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. ationd oil, oily esters, ethyl alcohol or fractionated vegetable oils); and preservatives (e.g. methyl or propyl-p-hydroxybenzoates or sorbic acid). The preparations can also contain buffer salts, flavoring, coloring and sweetening agents as appropriate.

The pharmaceutical compositions can be formulated for parenteral administration by injection, e.g. by bolus injection or continuous infusion. Formulations for injection can be presented in a unit dosage form, e.g. in ampoules or in multi-dose containers, with an optionally added preservative. The pharmaceutical compositions can further be formulated as suspensions, solutions or emulsions in oily or aqueous vehicles, and may contain other agents including suspending, stabilizing and/or dispersing agents.

Additionally, the pharmaceutical compositions can also be formulated as a depot preparation. These long acting formulations can be administered by implantation (e.g. subcutaneously or intramuscularly) or by intramuscular injection. Thus, for example, the compounds may be formulated with suitable polymeric or hydrophobic materials (e.g. as an emulsion in an acceptable oil) or ion exchange resins, or as sparingly soluble derivatives, for example, as a sparingly soluble salt. Other suitable delivery systems include microspheres, which offer the possibility of local noninvasive delivery of drugs over an extended period of time. This technology can include microspheres having a precapillary size, which can be injected via a coronary catheter into any selected part of an organ without causing inflammation or ischemia. The administered therapeutic is men slowly released from the microspheres and absorbed by the surrounding cells present in the selected tissue.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts, and fusidic acid derivatives. In addition, detergents may be used to facilitate permeation. Transmucosal administration can occur using nasal sprays or suppositories. For topical administration, the viral particles described herein can be formulated into ointments, salves, gels, or creams as generally known in the art. A wash solution can also be used locally to treat an injury or inflammation in order to accelerate healing.

Pharmaceutical forms suitable for injectable use can include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid. It must be stable under the conditions of manufacture and certain storage parameters (e.g. refrigeration and freezing) and must be preserved against the contaminating action of microorganisms, such as bacteria and fungi.

If formulations disclosed herein are used as a therapeutic to boost an immune response in a subject, a therapeutic agent can be formulated into a composition in a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

A carrier can also be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents known in the art. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions can be prepared by incorporating the active compounds or constructs in the required amount in the appropriate solvent with various of the other ingredients enumerated above, as required, followed by filtered sterilization.

Upon formulation, solutions can be administered in a manner compatible with the dosage formulation and in such amount as is therapeutically effective. The formulations are easily administered in a variety of dosage forms, such as the type of injectable solutions described above, but slow release capsules or microparticles and microspheres and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intratumorally, intramuscular, subcutaneous and intraperitoneal administration. In this context, sterile aqueous media that can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion.

The person responsible for administration will, in any event, determine the appropriate dose for the individual subject. For example, a subject may be administered viral particles described herein on a daily or weekly basis for a time period or on a monthly, bi-yearly or yearly basis depending on need or exposure to a pathogenic organism or to a condition in the subject (e.g. cancer).

In addition to the compounds formulated for parenteral administration, such as intravenous, intratumorally, intradermal or intramuscular injection, other pharmaceutically acceptable forms include, e.g., tablets or other solids for oral administration; liposomal formulations; time release capsules; biodegradable and any other form currently used.

One may also use intranasal or inhalable solutions or sprays, aerosols or inhalants. Nasal solutions can be aqueous solutions designed to be administered to the nasal passages in drops or sprays. Nasal solutions can be prepared so that they are similar in many respects to nasal secretions. Thus, the aqueous nasal solutions usually are isotonic and slightly buffered to maintain a pH of 5.5 to 7.5. In addition, antimicrobial preservatives, similar to those used in ophthalmic preparations, and appropriate drug stabilizers, if required, may be included in the formulation. Various commercial nasal preparations are known and can include, for example, antibiotics and antihistamines and are used for asthma prophylaxis.

Oral formulations can include excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders. In certain defined embodiments, oral pharmaceutical compositions will include an inert diluent or assimilable edible carrier, or they may be enclosed in hard or soft shell gelatin capsule, or they may be compressed into tablets, or they may be incorporated directly with the food of the diet. For oral therapeutic administration, the active compounds may be incorporated with excipients and used in the form of ingestible tablets, buccal tablets, troches, capsules, elixirs, suspensions, syrups, wafers, and the like.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabens as preservatives, a dye and flavoring, such as cherry or orange flavor.

Further embodiments disclosed herein can concern kits for use with methods and compositions. Kits can also include a suitable container, for example, vials, tubes, mini- or microfuge tubes, test tube, flask, bottle, syringe or other container. Where an additional component or agent is provided, the kit can contain one or more additional containers into which this agent or component may be placed. Kits herein will also typically include a means for containing the viral particles and any other reagent containers in close confinement for commercial sale. Such containers may include injection or blow-molded plastic containers into which the desired vials are retained. Optionally, one or more additional active agents such as, e.g., anti-inflammatory agents, anti-viral agents, anti-fungal or anti-bacterial agents or anti-tumor agents may be needed for compositions described.

Compositions disclosed herein may be administered by any means known in the art. For example, compositions may include administration to a subject intravenously, intratumorally, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intrathecally, subcutaneously, subconjunctival, intravesicularlly, mucosally, intrapericardially, intraumbilically, intraocularly, orally, locally, by inhalation, by injection, by infusion, by continuous infusion, by localized perfusion, via a catheter, via a lavage, in a cream, or in a lipid composition.

Any method known to one skilled in the art maybe used for large scale production of viral particles, packaging cells and particle constructs described herein. For example, master and working seed stocks may be prepared under GMP conditions in qualified primary CEFs or by other methods. Packaging cells may be plated on large surface area flasks, grown to near confluence and viral particles purified. Cells may be harvested and viral particles released into the culture media isolated and purified, or intracellular viral particles released by mechanical disruption (cell debris can be removed by large-pore depth filtration and host cell DNA digested with endonuclease). Virus particles may be subsequently purified and concentrated by tangential-flow filtration, followed by diafiltration. The resulting concentrated bulk maybe formulated by dilution with a buffer containing stabilizers, filled into vials, and lyophilized. Compositions and formulations may be stored for later use. For use, lyophilized viral particles may be reconstituted by addition of diluent.

Certain additional agents used in the combination therapies can be formulated and administered by any means known in the art.

Compositions as disclosed herein can also include adjuvants such as aluminum salts and other mineral adjuvants, tensoactive agents, bacterial derivatives, vehicles and cytokines. Adjuvants can also have antagonizing immunomodulating properties. For example, adjuvants can stimulate Th1 or Th2 immunity. Compositions and methods as disclosed herein can also include adjuvant therapy.

### EXAMPLES

The following examples are provided for illustrative purposes only and are not intended to limit the scope of the invention.

### Materials and Methods

### Cell Lines and Antibodies

All 293 cell lines were maintained in DMEM supplemented with 10% FBS, 1% Pen/Strep, and 1% L-glutamine. 293 hErbB2 and 293hASGR1/2 cell lines were generated by lentiviral transduction of the parental 293 cell line with a vector expressing the corresponding cDNA. All cell lines were obtained from the Regeneron TC core facility. The B1 antibody recognizes a linear epitope shared by AAV VP1, VP2 and VP3.

### AAV capsid protein constructs

GeneBlocks encoding the desired SpyTag insertions, flanking linker amino acids, and additional mutations were purchased from IDT and cloned into BsiWI and XcmI-digested pAAV2-CAP wt or pAAV9-CAP wt using Gibson Assembly according to the manufacturer's protocol (NEB).

### Fusion of SpyCatcher to antibodies

GeneBlocks encoding SpyCatcher were purchased from IDT, and Gibson Assembly was used to clone the coding sequence in-frame into expression plasmids for scFv or antibody heavy chains at the C terminus of each construct, separated by a flexible amino acid linker GSGESG (SEQ ID NO:48).

### Prepration of AAV viral vectors

Virus was generated by transfecting 293T packaging cells using PEI Pro with the following plasmids: pAd Helper, an AAV2 ITR-containing genome plasmid encoding a reporter protein, and a pAAV-CAP plasmid encoding AAV Rep and Cap genes, either with or without additional plasmids encoding either an scFv or the heavy and light chains of an antibody. The scFv and antibody heavy chain constructs are all fused to SpyCatcher at their C terminus as described above. Transfection was performed in OptiMEM, and media was changed to DMEM supplemented with 10% FBS, 1% Pen/Strep, and 1% L-Glut after 8 hours.

Transfected packaging cells were incubated for 3 days at 37°C, then virus was collected from cell lysates using a standard freeze-thaw protocol. In brief, packaging cells were lifted by scraping and pelleted. Supernatant was removed, and cells were resuspended in a solution of 50mM Tris-HCl; 150mM NaCl; and 2 mM MgCl2 [pH 8.0]. Intracellular virus particles were released by inducing cell lysis via three consecutive freeze-thaw cycles, consisting of shuttling cell suspension between dry ice/ethanol bath and 37°C water bath with vigorous vortexing. Viscosity was reduced by treating lysate with EMD Millipore Benzonase (50 U/ml of cell lysate) for 60 min at 37°C, with occasional mixing. Debris was then pelleted by centrifugation, and the resulting supernatant was filtered through a 0.22 µm PVDF Millex-GV Filter, directly into the upper chamber of an Amicon Ultra-15 Centrifugal Filter Unit with Ultracel-100 membrane (100 KDa MWCO) filter cartridge. The filter unit was centrifuged at 5-10 minute intervals until desired volume was reached in the upper chamber, then concentrated crude virus was pipetted into a low-protein-binding tube and stored at 4C. Titer (viral genomes per milliliter vg/mL) was determined by qPCR using a standard curve of a virus of known concentration.

### Cell Infection/Transduction and Flow Cytometric analysis

To infect cells, viral particles were added directly to the media of cells in culture, and the mixture was incubated overnight at 37°C. The media in each well was replaced 24 hours later, and cells were incubated for 5 days. On day 5 post-infection, cells were trypsinized, resuspended in PBS with 2% FBS, and the percentage of GFP+ cells was collected on a BD FACSCanto flow cytometer and analyzed using FlowJo software.

### Western blot analysis

The reaction between SpyTagged AAV proteins VP1, VP2 and VP3 and SpyCatcher-tagged antibodies or scFvs was monitored by Western blot analysis. Novex^{®} Tris-Glycine SDS Sample Buffer with Reducing Agent was added to equal volumes of crude virus preparations, and samples were heated to 85°C for 5 minutes, then cooled to room temperature and loaded onto a pre-cast 4-12% Tris-Glycine gel (Invitrogen). Proteins were separated by reducing SDS-PAGE and blotted onto PVDF via a wet transfer. Membranes were blocked with Li-Cor Odyssey TBS Blocking Buffer and probed with the mouse monocolonal B1 antibody (ARP American Research Products, Inc.) diluted 1:100 in TBST overnight at 4°C. Blots were washed in TBST, probed with an infrared-conjugated anti-mouse secondary antibody, and imaged on the Li-Cor Odyssey.

### Example 1 Conjugation of an scFv to a peptide inserted into the AAV2 capsid at residue N587 directs antigen-specific targeting

Each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pAAV2-CAP N587 SpyTag HBM WITH or WITHOUT | 4ug |
| pCMV-C6.5-SpyCatcher | 4ug |

Cells incubated with viral particles as described above were evaluated by flow cytometric analysis for infection.

AAV2 bearing the heparin binding mutations (HBM) R585A and R588A, as well as the SpyTag peptide at capsid position N587, was produced in the presence or absence of C6.5-SpyCatcher, an scFv that binds HER2 and is fused to SpyCatcher at its C-terminus. AAV2 conjugated to the HER2-targeting scFv specifically infects HER2+ cells, and displays very little background infection of HER2- cells. **Figure 1****.**

### Example 2 Conjugation of an antibody to a peptide inserted into the AAV2 capsid at residue N587 directs antigen-specific targeting

Each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pAAV2-CAP wt | 4ug |

OR

| | |
|---|---|
| pAAV2-CAP N587 SpyTag HBM WITH OR WITHOUT | 4ug |
| pAnti-HER2 hIgG4 SpyCatcher Vh | 1.5ug |
| pAnti-HER2 Vk | 3ug |

Wildtype AAV2 and AAV2 bearing the heparin binding mutations (HBM) R585A and R588A, as well as the SpyTag peptide at capsid position N587, was produced in the presence or absence of the antibody heavy and light chains encoding SpyCatcher-Herceptin, an antibody that binds HER2 and is fused to SpyCatcher at the C-terminus of the heavy chain. Cells infected with viral particles as described above were evaluated by flow cytometric analysis to monitor transduction. AAV2 conjugated to the HER2-targeting antibody specifically infects HER2+ cells, and displays very little background infection of HER2- cells. **Figure 2****.**

### Example 3 Conjugation of an antibody to a peptide inserted into the AAV2 capsid at residue G453 directs antigen-specific targeting

Residues N587 and G453 each lie on exposed regions of the AAV2 capsid that form protein spikes that extend away from the virion surface. The residues lie on two different spikes, so it was investigated whether a SpyTag inserted after residue G453 would function in the same way as the SpyTag inserted after residue N587. Each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pAAV2-CAP G453 SpyTag HBM | 0.5ug |
| pAAV2-CAP R585A R588A HBM | 3.5ug |

OR

| | |
|---|---|
| pAAV2-CAP wt WITH or WITHOUT | 4ug |
| pAnti-HER2 hIgG4 SpyCatcher Vh | 1.5ug |
| pAnti-HER2 Vk | 3ug |

Wildtype AAV2 and AAV2 bearing the heparin binding mutations (HBM) R585A and R588A as well as the SpyTag peptide at capsid position G453, was produced in the presence or absence of the antibody heavy and light chains encoding SpyCatcher-Herceptin, an antibody that binds HER2 and is fused to SpyCatcher at the C-terminus of the heavy chain. The virus was produced as a mosaic by mixing SpyTag-expressing capsids with HBM capsids. Cells infected with viral particles as described above were evaluated by flow cytometric analysis to monitor transduction. AAV2 conjugated to the HER2-targeting antibody specifically infects HER2+ cells, and displays very little background infection of HER2- cells. **Figure 3**

### Example 4 Increasing modification of AAV virions by scFvs decreases their infectivity

In an effort to optimize the efficiency of the SpyTag-SpyCatcher reaction, the accessibility of SpyTag on the surface of the virus was improved by flanking the peptide tag with flexible linker amino acids on each side. A panel of N587 SpyTag insertion mutants flanked by increasing linker lengths was generated, and prepared virus using these AAV2 Rep-Cap constructs in the presence or absence of C6.5-SpyCatcher, the scFv that binds HER2 and is fused to SpyCatcher at its C-terminus. The reaction between SpyTagged AAV2 proteins VP1, VP2 and VP3 and SpyCatcher-tagged C6.5 was monitored by Western blotting; SpyTagged capsid proteins that have reacted with SpyCatcher-tagged scFv exhibit an increase in size by SDS-PAGE. Cells infected with viral particles as described above were evaluated by flow cytometric analysis to measure transduction.

Each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pAnti-HER2 hIgG4 SpyCatcher Vh | 1.5ug |
| pAnti-HER2 Vk | 3ug |
| pAAV2-CAP N587 LinkerX SpyTag | 4ug |

pAAV2-CAP N587 Linker SpyTag constructs include:
pAAV2-CAP N587 Linker1 SpyTag HBM
pAAV2-CAP N587 Linker2 SpyTag HBM
pAAV2-CAP N587 Linker4 SpyTag HBM
pAAV2-CAP N587 Linker6 SpyTag HBM
pAAV2-CAP N587 Linker8 SpyTag HBM
pAAV2-CAP N587 Linker10 SpyTag HBM

When SpyTag was not flanked by linker amino acids, VP-SpyTag-SpyCatcher-scFv complexes were undetectable by Western blotting, and the viruses achieved low levels of specific transduction of HER2+ cells. **Figure 4****.** As the linker length increased (1-6 amino acids), VP-SpyTag-SpyCatcher-scFv complexes began to be detectable via Western blotting, and the transduction efficiency of the viruses increased. **Figure 4****.** However, when SpyTag was flanked by the two longest linkers (8-10 amino acids), nearly all of the VP proteins had reacted with SpyCatcher-Vh by Western blotting, but these fully-decorated viruses no longer transduced cells efficiently. **Figure 4****.** Therefore, it appeared that overmodification of AAV particles by scFvs is detrimental to their ability to transduce target cells, and only a small number of conjugated scFvs are required to retarget the virus to target cells.

### Example 5 Increasing modification of AAV virions by antibodies decreases their infectivity

Using the panel of N587 SpyTag insertion mutants flanked by increasing linker lengths, virus was prepared using these AAV2 Rep-Cap constructs in the presence or absence of the antibody heavy and light chains encoding SpyCatcher-Herceptin, an antibody that binds HER2 and is fused to SpyCatcher at the C-terminus of the heavy chain. The reaction between SpyTagged AAV proteins VP1, VP2 and VP3 and SpyCatcher-tagged Herceptin heavy chain (Vh) was monitored by Western blotting; SpyTagged capsid proteins that have reacted with SpyCatcher-tagged antibodies will exhibit a size shift by SDS-PAGE.

Each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pAnti-HER2 hIgG4 SpyCatcher Vh | 1.5ug |
| pAnti-HER2 Vk | 3ug |
| pAAV2-CAP N587 LinkerX SpyTag | 4ug |

pAAV2-CAP N587 Linker SpyTag constructs include:
pAAV2-CAP N587 SpyTag HBM
pAAV2-CAP N587 Linker1 SpyTag HBM
pAAV2-CAP N587 Linker2 SpyTag HBM
pAAV2-CAP N587 Linker4 SpyTag HBM
pAAV2-CAP N587 Linker6 SpyTag HBM
pAAV2-CAP N587 Linker8 SpyTag HBM
pAAV2-CAP N587 Linker10 SpyTag HBM

When SpyTag was not flanked by linker amino acids or was flanked by very short amino acids, VP-SpyTag-SpyCatcher-Vh complexes were not detected by Western blotting, but the viruses specifically infected HER2+ cells at an efficiency nearing wildtype levels. **Figure 5****.** Conversely, when SpyTag was flanked by longer linkers (6 amino acids or greater), nearly all of the VP proteins had reacted with SpyCatcher-Vh by Western blotting, but these fully-decorated viruses were no longer infectious. **Figure 5****.** Increasing modification of AAV particles by antibodies is detrimental to their ability to transduce target cells, and only a small number of conjugated antibodies are required to retarget the virus to target cells.

### Example 6 Mosaicism modulates the transduction efficiency of virus particles

Since long, flexible linkers allow efficient reaction of SpyTagged AAV capsids with SpyCatcher-fused scFvs, but overmodification of AAV particles by scFvs is detrimental to their ability to transduce target cells, the number of SpyTags on each virion was reduced while SpyTag accessibility and efficient reactivity was retained by generating mosaic AAV particles that are a mixture of different ratios of highly reactive SpyTagged constructs with non-SpyTagged capsid constructs, all bearing the R585A R588A heparin binding mutation (HBM). Each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pCMV-C6.5-SpyCatcher | 4ug |

WITH VARYING RATIOS OF:

| | |
|---|---|
| pAAV2-CAP N587 Linker10 SpyTag | HBM Xug |
| pAAV2-CAP R585A R588A | Xug |

or

| | |
|---|---|
| pAd Helper | 8ug |
| pAAV-CMV-hrGFP | 4ug |
| pAnti-HER2 hIgG4 SpyCatcher Vh | 1.5ug |
| pAnti-HER2 Vk | 3ug |

WITH VARYING RATIOS OF:

| | |
|---|---|
| pAAV2-CAP N587 Linker10 SpyTag HBM | Xug |
| pAAV2-CAP R585A R588A | Xug |

or

| | |
|---|---|
| pAd Helper | 8ug |
| pAAV-CMV-hrGFP | 4ug |
| pAnti-HER2 hIgG4 SpyCatcher Vh | 1.5ug |
| pAnti-HER2 Vk | 3ug |

WITH VARYING RATIOS OF:

| | |
|---|---|
| pAAV2-CAP G453 LinkerX SpyTag HBM | Xug |
| pAAV2-CAP R585A R588A | Xug |

The ratio between pAAV2-CAP N587 Linker10 SpyTag HBM and pAAV2-CAP R585A R588A plasmids was either 1:0 (4ug:0ug) which represents pure pAAV2-CAP N587 Linker10 SpyTag HBM virions, 3:1 (3ug: lug), 1:1 (2ug:2ug), or 1:3 (1ug:3ug) in the transfection mix. The ratio between pAAV2-CAP G453 Linker10 SpyTag HBM and pAAV2-CAP R585A R588A plasmids was either 1:0 (4ug:0ug) which represents pure pAAV2-CAP G453 Linker10 SpyTag HBM virions, 1:3 (1ug:3ug), or 1:7 (0.5ug:3.5ug) in the transfection mix. The reaction between SpyTagged AAV proteins VP1, VP2 and VP3 and SpyCatcher-tagged anti-HER scFv or SpyCatcher-tagged Herceptin heavy chain (Vh) was monitored by Western blotting; SpyTagged capsid proteins that have reacted with SpyCatcher-tagged scFvs or antibodies will exhibit a size shift by SDS-PAGE. The reaction of the N587 SpyTag linker panel with SpyCatcher-anti-HER2 scFv is shown in **Figure 6****.** The reaction of the N587 SpyTag linker panel with SpyCatcher-anti-HER2 antibody is shown in **Figure 7****.** Cells infected with the mosaic viral particles described above were evaluated by flow cytometric analysis to measure transduction **Figure 6-7****.** As the amount of highly reactive Linker10-flanked SpyTag capsids was decreased, and the number of non-SpyTagged capsids increased, a decrease in the amount of VP-SpyTag-SpyCatcher-scFv complexes was observed by Western blotting, coupled with an increase in the transduction efficiency of the virus **Figures 6-7****.** An inverse relationship between the number of antibodies decorating the virion and the efficiency of transduction with the retargeted virus was demonstrated.

The reaction of G453 SpyTag HBM and G453 Linker10 SpyTag HBM with SpyCatcher-anti-HER2 antibody is shown in **Figure 8****.** Both SpyTag insertions at G453, either SpyTag alone or SpyTag flanked by Linker10, reacted very efficiently with SpyCatcher-tagged Herceptin as measured by Western blotting, suggesting that the G453 insertion site is naturally more accessible than N587, which does not readily react unless flanked by linker amino acids. As observed with the N587 linker panel, when the viruses were heavily modified by SpyCatcher-Herceptin antibody, the viruses were no longer infectious. Therefore, it appears that high levels of modification of AAV particles by antibodies is detrimental to their ability to transduce target cells, and conclude that a SpyTag inserted after G453 is naturally more accessible than a SpyTag inserted at N587.

### Example 7 The SpyTag-SpyCatcher system can be used with additional antibody-target pairs to achieve specific retargeting in vitro

The ability of the SpyTag-SpyCatcher approach to retarget AAV to targets other than HER2 was examined. SpyCatcher-tagged antibodies targeting additional cell-surface proteins were cloned, and the ability of these antibodies to retarget SpyTagged AAV to cell types expressing these additional targets was examined. For experiments targeting ASGR1 and CD63, each virus was generated as described above by transfecting one 15cm plate of 293T packaging cells with the following plasmids and quantities:

| | |
|---|---|
| pAd Helper | 8ug |
| pAAV-CMV-hrGFP | 4ug |
| pAAV2-CAP N587 Linker10 SpyTag HBM | 0.5ug |
| pAAV2-CAP R585A R588A | 3.5ug |

WITH OR WITHOUT

| | |
|---|---|
| SpyCatcher-fused Vh heavy chain plasmid | 1.5ug |
| Vk light chain plasmid | 3ug |

For experiments targeting PTPRN, each virus was generated as described above by transfecting one 15cm plate of 293T packaging cells with the following plasmids and quantities:

| | |
|---|---|
| pAd Helper | 8ug |
| pAAV-CMV-eGFP | 4ug |
| pAAV2-CAP G453 Linker 10 SpyTag HBMx5 | 0.5ug |
| pAAV2-CAP N587 Myc | 3.5ug |

WITH OR WITHOUT

| | |
|---|---|
| SpyCatcher-fused Vh heavy chain plasmid | 1.5ug |
| Vk light chain plasmid | 3ug |

For experiments targeting ENTPD3 and CD20, each virus was generated as described above by transfecting one 15cm plate of 293T packaging cells with the following plasmids and quantities:

| | |
|---|---|
| pAd Helper | 8ug |
| pAAV-CMV-Firefly Luciferase | 4ug |
| pAAV2-CAP N587 SpyTag HBM | 4ug |

WITH OR WITHOUT

| | |
|---|---|
| SpyCatcher-fused Vh heavy chain plasmid | 1.5ug |
| Vk light chain plasmid | 3ug |

SpyCatcher-Vh and Vk plasmids that encode antibody heavy and light chains that recognize the human proteins ASGR1, CD63, PTPRN, ENTPD3, and CD20 were tested. To generate mosaic AAV particles with a low number of exposed SpyTags, the SpyTag and non-SpyTagged plasmids were present at a ratio of 1:7 in the transfection mix, which was previously determined to be the ideal ratio of SpyTag to non-SpyTag capsids for retargeting AAV using antibodies. Cells expressing ASGR1, CD63 or PTPRN were infected with the mosaic AAV2 particles described above, and transduction was measured by flow cytometry. AAV2 conjugated to the ASGR1, CD63, and PTPRN-specific antibodies, was able to specifically infect the cognate target cells expressing ASGR1, CD63, and PTPRN, respectively, and displayed very low background infection in the absence of the antibody. Cells expressing ENTPD3 or CD20 were infected with the AAV2 particles described above, and transduction was measured by a Luciferase assay using standard protocols. AAV2 conjugated to the ENTPD3 and CD20-specific antibodies, was able to specifically infect the cognate target cells expressing ENTPD3 and CD20, respectively, and displayed very low background infection in the absence of the antibody.

### Figure 9

### Example 8 The SpyTag-SpyCatcher system can be adapted for retargeting AAV9

The adaptability of the SpyTag-SpyCatcher system to other AAV serotypes was examined. AAV9 is a widely used serotype that generates high titer virus and is very efficient in transducing mouse tissues. The residues important for receptor binding differ between AAV2 and AAV9, since AAV2 binds Heparin Sulfate Proteoglycans and AAV9 binds Galactose. Residues known to be important in receptor binding were determined from available literature (Bell, C. L., Gurda, B. L., Van Vliet, K., Agbandje-McKenna, M., & Wilson, J. M. (2012). Identification of the galactose binding domain of the adeno-associated virus serotype 9 capsid. Journal of Virology, 86(13), 7326-7333. http://doi.org/10.1128/JVI.00448-12), and included N470, D271, N272, Y446, and W503. The W503A mutation was selected as the receptor binding mutation to use in generating mutant constructs, since this single amino acid mutation strongly reduced receptor binding. Regions of the AAV9 capsid that are orthologous to the two projections (variable loops) within which AAV2 N587 and G453 lie were also identified; the corresponding residues in AAV9 are A589 and G453. SpyTag was inserted into these two sites within the AAV9 capsid, with and without flanking linker amino acids, and in combination with receptor binding mutation W503A.

Each virus was generated as described above by transfecting one 15cm plate of 293T packaging cells with the following plasmids and quantities:

| | |
|---|---|
| pAd Helper | 8ug |
| pAAV-CMV-eGFP | 4ug |
| pAnti-HER2 hIgG4 SpyCatcher Vh | 1.5ug |
| pAnti-HER2 Vk | 3ug |
| pAAV9 RC plasmid | 4ug total DNA |

For AAV9 wt, AAV9-CAP A589SpyT _W503A, AAV9-CAP A589Linker10SpyT_W503A, and AAV9-CAP G453Linker10SpyT_W503A, 4ug of each plasmid was used for each transfection.

For mosaic viruses, 3.5ug of pAAV9-CAP W503A and 0.5ug of either pAAV9-CAP A589Linker10SpyT _W5503A or pAAV9-CAP G453Linker10SpyT_W503A was used for each transfection to achieve a 1:7 ratio of SpyTag to non-SpyTagged Rep-Cap plasmids.

Cell transduction, flow cytometric analysis, and Western blot analysis were performed as described above.

AAV9 RC A589 and G453 SpyTag insertions supported a reaction with SpyCatcher-Herceptin and mediated specific transduction of HER2+ cells. **Figure 10****.** Similar to AAV2, a SpyTag inserted at AAV9 RC A589 without flanking linkers was not very accessible and reacted poorly with SpyCatcher. **Figure 10****.** Conversely, the addition of amino acid linkers on either side of SpyTag allowed a more robust reactivity to SpyCatcher, and mosaic particles with a few highly reactive SpyTags were very efficient at transducing target cells. **Figure 10****.**

### Example 9: Retargeting of SpyTagged AAV particle- SpyCatcher-Vh complexes in vivo

To determine whether the VP-SpyTag-SpyCatcher-Vh could be retargeted to liver cells expressing hASGR1 *in vivo,* mice genetically modified such that their liver cells express hASGR1 on C57BL/6 background, and control wild-type mice were injected intravascularly with wild-type AAV alone or VP-SpyTag-SpyCatcher-Vh viral particles (as pure or mosaic particles, and with or without an amino acid linker) carrying a reporter gene, e.g., green fluorescent protein or firefly luciferase. Controls include mice that were injected with phosphate buffered saline (PBS). To determine whether VP-SpyTag-SpyCatcher-Vh could be detargeted from liver and retargeted to other organs, wildtype mice were injected intravascularly with wildtype AAV alone or VP-SpyTag-SpyCatcher-Vh viral particles (as mosaic particles) carrying a reporter gene, e.g., green fluorescent protein. To demonstrate detargeting from liver and retargeting to another organ, the protein ENTPD3 was chosen, since it is not known to be expressed in liver but is expressed in other organs, such as pancreatic islet cells (Syed et al. 2013, Am J Physiol Endocrinol Metab 305: E1319-E1326, 2013) and tongue, according to publically available databases (data extracted from GenePaint.org http://www.informatics.jax.org/assay/MGI:5423021 and Riken FANTOM5 project, adult mouse dataset).

SpyCatcher-tagged antibodies targeting human CD3, human CD63, human ASGR1 (none of which are expressed in wildtype mice) or human ENTPD3 (which also recognizes the mouse protein) were cloned and the ability of these antibodies to retarget SpyTagged AAV2 carrying either eGFP or firefly luciferase *in vivo* was examined. Each virus was generated as described above by transfecting 15cm plates of 293T packaging cells with the following plasmids and quantities:
Anti-Human CD3/ anti-Human ASGR1 GFP

| | |
|---|---|
| pAd Helper | 8ug |
| pAAV-CAGG eGFP | 4ug |
| pAAV2-CAP N587 SpyTag HBM | 4ug |

WITH OR WITHOUT

| | |
|---|---|
| pAnti-CD3 or Anti-ASGR1 SpyCatcher Vh | 1.5ug |
| pAnti-CD3 or Anti-ASGR1 Vk | 3ug |

Anti-Human CD63/ anti-Human ASGR1 Luciferase

| | |
|---|---|
| pAd Helper | 8ug |
| pAAV-UbC- Firefly Luciferase | 4ug |
| pAAV2-CAP G453 Linker 10 SpyTag HBMx5 | 0.5ug |
| pAAV2-CAP N587 Myc | 3.5ug |

WITH or WITHOUT

| | |
|---|---|
| pAnti-CD63 or Anti-ASGR1 SpyCatcher Vh | 1.5ug |
| pAnti-CD63 or Anti-ASGR1 Vk | 3ug |

Anti-Human ENTPD3/ anti-Human ASGR1 GFP

| | |
|---|---|
| pAd Helper | 8ug |
| pAAV-CMV-eGFP | 4ug |
| pAAV2-CAP G453 Linker 10 SpyTag HBMx5 | 0.5ug |
| pAAV2-CAP N587 Myc | 3.5ug |

WITH or WITHOUT

| | |
|---|---|
| pAnti-ENTPD3 or Anti-ASGR1 SpyCatcher Vh | 1.5ug |
| pAnti-ENTPD3 or Anti-ASGR1 Vk | 3ug |

SpyCatcher-Vh and Vk plasmids that encode antibody heavy and light chains that recognize the human proteins ASGR1, CD3, or CD63, were tested in mice genetically modified such that their liver cells express hASGR1 on C57BL/6 background. SpyCatcher-Vh and Vk plasmids that encode antibody that recognizes the human protein ASGR1 (as a non-targeting control) or mouse and human protein ENTPD3 were tested in wildtype mice. Ten days post infection, mice were sacrificed and expression of the reporter gene was examined; livers, spleens, and kidneys were fixed and stained with chicken anti-EGFP antibody (Jackson ImmunoResearch Labs, Inc. West Grove, PA) and Alexa-488 conjugated anti-chicken secondary antibody (Jackson ImmunoResearch Labs, Inc. West Grove, PA). To test luminescence of animals, 14-days post-infection, live animals were anesthetized using isoflurane, injected with a Luciferin substrate and imaged 10 minutes later using the IVIS Spectrum In Vivo Imaging System (PerkinElmer). **Figures 11** **and** **12** show that infection with the AAV2 -SpyTag-SpyCatcher-Vh complexes was detected only in the liver of hASGR1-expressing mice injected with hASGR1-retargeted AAV and was not detected in the liver of wildtype mice, which do not express hASGR1. No positive EGF was detected in other organs (data not shown).

**Figure 13** shows immunohistochemistry staining for eGFP expression in liver and pancreas of wildtype mice following injection of AAVs conjugated to antibodies targeting ENTPD3 or hASGR1 as a non-targeting control, since hASGR1 is not expressed in wildtype mice. Four weeks post-infection, organs were harvested from infected animals and fixed in 10% neutroal buffered formalin for 48 hours, then stained for eGFP via immunohistochemistry. Figure 13 shows that the AAV2-SpyTag-SpyCatcher-Vh complexes were detargeted from the wildtype mouse liver; all mice injected with AAVs conjugated to antibodies targeting ENTPD3 and hASGR1 showed similar lack of eGFP expression in liver. A mouse injected with AAVs conjugated to antibodies targeting ENTPD3 had cells expressing eGFP in pancreatic islets, where ENTPD3 is thought to be expressed.

**Figure 14** shows immunohistochemistry staining for eGFP expression in liver and tongue of wildtype mice following injection of AAVs conjugated to antibodies targeting ENTPD3 or hASGR1 as a non-targeting control, since hASGR1 is not expressed in wildtype mice. 14 days post-infection, organs were harvested from infected animals and fixed in 10% neutroal buffered formalin for 48 hours, then stained for eGFP via immunohistochemistry. Figure 14 shows that the AAV2-SpyTag-SpyCatcher-Vh complexes were detargeted from the wildtype mouse liver; all mice injected with AAVs conjugated to antibodies targeting ENTPD3 and hASGR1 showed similar lack of eGFP expression in liver. All three mice injected with AAVs conjugated to a non-targeting irrelevant antibody (anti-ASGR1) showed no staining in the tongue, while all three mice injected with AAVs conjugated to anti-ENTPD3 showed eGFP expressing cells in the tongue, where ENTPD3 is thought to be expressed.

### Example 10: Delivery of a suicide gene to cells expressing a targeted ligand

Also described is the ability of VP-SpyTag-SpyCatcher-Vh complexes to specifically deliver therapeutic cargo, such as one or more suicide genes, a biological therapeutic (e.g., antibody), a CRISPR/Cas gene editing system, shRNA, etc., to a targeted cell type.

To test the ability of VP-SpyTag-SpyCatcher-Vh complexes to deliver a suicide gene to a specific cell, a xenograft nude mouse model of HER2+ breast cancer as described by Wang et al. ((2010) Cancer Gene Therapy 17:559-570) is used.

VP-SpyTag-SpyCatcher-Vh complexes carrying a suicide gene (SG) are generated similarly to those described in the Materials and Methods.

*Cell Lines:* BT474 breast cancer, SK-BR-3 breast cancer and Calu-3 lung cancer cell lines are HER2 positive human tumor cell lines (Bunn P.A. et al., (2001) Clin Cancer Res. 7:3239-3250; Pegram M, et al. (1999) Oncogene 18:2241-2251; Spiridon CI, et al., (2002) Clin Cancer Res. 8:1720-1730). A-673 rhabdomyosarcoma and HeLa cervical cancer are HER2 negative human tumor cell lines and BEAS-2b is an immortalized bronchial epithelial HER2 negative cell line (Jia LT et al. (2003) Cancer Res. 63:3257-3262; Kern JA, et al., (1993) Am J Respir Cell Mol Biol 9:448-454; Martinez-Ramirez A, et al., (2003) Cancer Genet Cytogenet. 2003;141:138-142). All of these cell lines are obtained from American Type Culture Collection (ATCC, Manassas, VA) and maintained in medium recommended by the ATCC.

*Mice:* Female nude mice, 6 to 8 weeks of age are obtained and housed under specific pathogen-free conditions. On day 0, mice are simultaneously (1) injected subcutaneously in the right flank with 10⁷ BT474, SK-BR-3, Calu-3, A-673 or HeLa tumor cells and (2) treated intravenously VP-SpyTag-SpyCatcher-Vh complexes carrying a reporter (e.g., EGFP) or suicide gene. Serving as controls are untreated animals (animals injected with tumor cells only), animals injected with wildtype AAV particles carrying a reporter or suicide gene, animals injected with SpyTag-virus particle only, etc. All animals are treated with an appropriate pro-drug 1 day after injection and treatment. The size of each tumor is measured using calipers 2 times weekly, and tumor volume is calculated as length×width²×0.52. Upon morbidity, tumor ulceration, a tumor diameter of 15 mm, or tumor volume of 1000 mm³, the mice are sacrificed, and the sacrifice date recorded as the date of death. Livers, spleens, kidneys and tumors of animals injected with virus particles carrying a reporter gene are fixed and reporter gene expression visualized.

Although the targeted delivery of suicide inducing genes has been described (Zarogoulidis P., et al. (2013) J. Genet. Syndr. Gene Ther. 4:16849), this example describes the delivery of a suicide gene to a cell expressing the targeted HER2 ligand using the viral particles described herein. In additional experiments, a suicide gene is delivered to another cell type expressing one or more other target ligands using a viral particle described herein Exemplary and non-limiting examples of receptors suitable for targeting include those receptors that mediate endocytosis of the viral particle, e.g., carcino-embryonic antigen (CEA) (Qiu Y, et al. (2012) Cancer Lett. 316:31-38) and vascular endothelial growth factor receptor (VEGFR) (Leng A, et al. (2013) Tumour Biol. 32:1103-1111; Liu T, et al. (2011) Exp Mol Pathol. 91:745-752). Additional receptors that may be targeted include: epidermal growth factor receptor (EGFR) (Heimberger AB, et al. (2009) Expert Opin Biol Ther. 9:1087-1098), cluster of differentiation 44s (CD44s) (Heider KH, et al. (2004) Cancer Immunol Immunother. 53:567-579), cluster of differentiation 133 (CD133 aka AC133) (Zhang SS, et al. (2012) BMC Med. ;10:85), folate receptor (FR) (Duarte S, et al., (2011) J Control Release 149(3):264-72), transferrin receptor (TfR) or cluster differentiation 71 (CD71) (Habashy HO, et al., Breast Cancer Res Treat. 119(2):283-93), mucins (Torres MP, et al., (2012) Curr Pharm Des. 2012; 18(17):2472-81), stage specific embryonic antigen 4 (SSEA-4) (Malecki M., et al., (2012) J Stem Cell Res Ther. 2(5)), tumor resistance antigen 1-60 (TRA-1-60) (Malecki M., et al., (2013) J Stem Cell Res Ther. 3:134).

While the invention has been particularly shown and described with reference to a number of embodiments, it would be understood by those skilled in the art that changes in the form and details may be made to the various embodiments disclosed herein without departing from the spirit and scope of the invention and that the various embodiments disclosed herein are not intended to act as limitations on the scope of the claims.

Although any methods and materials similar or equivalent to those described herein can be used in the practice or testing of the present invention, some preferred methods and materials are now described. All publications cited herein are incorporated herein by reference to describe in their entirety. Unless defined otherwise, all technical and scientific terms used herein have the same meaning as commonly understood by one of ordinary skill in the art to which this invention belongs.

Aspects of the invention
1. A recombinant viral capsid protein comprising a first member of a protein:protein binding pair operably linked to the capsid protein, optionally wherein the first member is a peptide tag, and optionally wherein the first member and/or the mutation reduces or abolishes the natural tropism of the capsid protein.
2. The recombinant viral capsid protein of aspect 1, further comprising a second cognate member of the protein:protein binding pair, wherein the first and second members are bound by a covalent bond.
3. The recombinant viral capsid protein of aspect 2, wherein the covalent bond is an isopeptide bond.
4. The recombinant viral capsid protein of any one of aspects 2-3, wherein the second member is operably linked to a targeting ligand, optionally wherein the targeting ligand is a binding moiety.
5. The recombinant viral capsid protein of any one of aspects 1-4, wherein the first member is flanked by a first and/or second linker that link(s) the first member to the capsid protein, and wherein the first and/or second linker is each independently at least one amino acid in length.
6. The recombinant viral capsid protein of aspect 5, wherein the first and second linkers are not identical.
7. The recombinant viral capsid protein of aspect 5, wherein the first and second linkers are identical and are 10 amino acids in length.
8. The recombinant viral capsid protein of any one of aspects 1-7, further comprising a mutation at an amino acid position involved with binding of the viral capsid protein to its natural receptor, wherein the mutation comprises an insertion of a heterologous peptide into the capsid protein, substitution of one or more amino acids of the capsid protein with a heterologous peptide, deletion of one or more amino acids of the capsid protein, or a combination thereof.
9. The recombinant viral capsid protein of any one of aspects 1-8, wherein the transduction efficiency of the recombinant viral capsid protein is:
   (i) reduced by 10%,
   (ii) reduced by 20%,
   (iii) reduced by 30%,
   (iv) reduced by 40%,
   (v) reduced by 50%,
   (vi) reduced by 60%,
   (vii) reduced by 70%,
   (viii) reduced by 80%,
   (ix) reduced by 90%, or
   (x) abolished
10. The recombinant viral capsid protein of any one of aspects 1-9, wherein the viral capsid protein is derived from a capsid gene of an adeno-associated virus (AAV) , wherein the capsid gene encodes an AAV VP1, VP2, and/or VP3 capsid protein, and optionally further comprising a mutation at an amino acid position involved with binding of the capsid .
11. The recombinant viral capsid protein of aspect 10, wherein the AAV is selected from the group consisting of AAV1, AAV2, AAV3, AAV4, AAV5, AAV6, AAV7, AAV8 and AAV9.
12. The recombinant viral capsid protein of aspect 10, wherein the adeno-associated virus is AAV2.
13. The recombinant viral capsid protein of aspect 10, wherein the adeno-associated virus is AAV9.
14. The recombinant viral capsid protein of any one of aspects 1-13, wherein the protein:protein binding pair is:
   (i) SpyTag:SpyCatcher,
   (ii) SpyTag:KTag,
   (iii) Isopeptag:pilin-C,
   (iv) SnoopTag:SnoopCatcher, or
   (v) SpyTag002:SpyCatcher002.
15. The recombinant viral capsid protein of any one of aspects 1-14, wherein the first member and any linker together are no more than about 50 amino acids in length.
16. The recombinant viral capsid protein of any one of aspects 1-15, wherein the first member is SpyTag.
17. The recombinant viral capsid protein of any one of aspects 2-16, wherein the second cognate member is SpyCatcher.
18. The recombinant viral capsid protein of any one of aspects 2-16, wherein the second cognate member is KTag.
19. The recombinant viral capsid protein of any one of aspects 2-15, wherein the first member is KTag and the second cognate member is SpyTag.
20. The recombinant viral capsid protein of any one of aspects 2-15, wherein the first member is SnoopTag and the second cognate member is SnoopCatcher.
21. The recombinant viral capsid protein of any one of aspects 2-15, wherein the first member is isopeptag and the second cognate member is Pilin-C
22. The recombinant viral capsid protein of any one of aspects 2-15, wherein the first member is SpyTag002 and the second cognate member is SpyCatcher002.
23. The recombinant viral capsid protein of any one of aspects 1-22, wherein the recombinant viral capsid protein or a viral capsid comprising the recombinant viral capsid protein has a reduced to abolished to infect a target cell in the absence of an appropriate targeting ligand compared to the viral capsid protein or capsid comprising the appropriate targeting ligand.
24. The recombinant viral capsid protein of any one of aspects 4-23, wherein the binding moiety is an antibody, or a portion thereof.
25. The recombinant viral capsid protein of aspect 24, wherein the antibody, or portion thereof, is fused to SpyCatcher.
26. The recombinant viral capsid protein of aspect 25, wherein the antibody, or portion thereof, is fused to a linker at the C-terminus, and the linker is fused to SpyCatcher at the linker's C-terminus.
27. The recombinant viral capsid protein of aspect 26, wherein the linker comprises a sequence set forth as SEQ ID NO:48 (GSGESG).
28. The recombinant viral capsid protein of any one of aspects 4-27, wherein the targeting ligand comprises an amino acid sequence set forth as SEQ ID NO:46.
29. The recombinant viral capsid protein of any one of aspects 4-28, wherein the targeting ligand specifically binds a cell surface molecule, optionally wherein the cell surface marker is
   (i) asialoglycoprotein 1 (ASGR1),
   (ii) ENTPD3,
   (iii) PTPRN,
   (iv) CD20,
   (v) CD63, or
   (vi) Her2.
30. The recombinant viral capsid protein of aspect 29, wherein the cell surface molecule is asialoglycoprotein 1 (ASGR1).
31. The recombinant viral capsid protein of aspect 29, wherein the cell surface molecule is CD63.
32. The recombinant viral capsid protein of aspect 29, wherein the cell surface molecule is ENTDP3.
33. A recombinant viral capsid comprising the recombinant viral capsid protein of any one of aspects 1-32.
34. The recombinant viral capsid of aspect 33, further comprising a reference viral capsid protein lacking any member of the specific binding pair.
35. The recombinant viral capsid of aspect 34, wherein the recombinant viral capsid protein and the reference viral capsid protein each comprise a mutation of at least one residue involved with the binding of the viral particle with its natural ligand.
36. The recombinant viral capsid of any one of aspects 34-35, comprising the recombinant viral capsid protein and the reference viral capsid protein at a ratio between 1:1 and 1:15.
37. A recombinant viral vector comprising a nucleotide of interest encapsulated by the recombinant viral capsid of any one of aspects 33-36.
38. The recombinant viral particle of aspect 37, wherein the nucleotide of interest is under the control of a promoter selected from the group consisting of a viral promoter, a bacterial promoter, a mammalian promoter, an avian promoter, a fish promoter, an insect promoter, and any combination thereof.
39. The recombinant viral particle of aspect 37, wherein the nucleotide of interest is under the control of a human promoter.
40. The recombinant viral particle of aspect 37, wherein the nucleotide of interest is under the control of a non-human promoter.
41. The recombinant viral particle of any one of aspects 37-40, wherein the nucleotide of interest is a reporter gene
42. The recombinant viral particle of aspect 41, wherein the reporter gene encodesgreen fluorescent protein.
43. The recombinant viral particle of aspect 41, wherein the reporter gene encodes green fluorescent protein, β-galactosidase (encoded lacZ gene), enhanced Green Fluorescent Protein (eGFP), MmGFP, blue fluorescent protein (BFP), enhanced blue fluorescent protein (eBFP), mPlum, mCherry, tdTomato, mStrawberry, J-Red, DsRed, mOrange, mKO, mCitrine, Venus, YPet, yellow fluorescent protein (YFP), enhanced yellow fluorescent protein (eYFP), Emerald, CyPet, cyan fluorescent protein (CFP), Cerulean, T-Sapphire, luciferase, alkaline phosphatase, or a combination thereof.
44. The recombinant viral particle of any one of aspects 37-40, wherein the nucleotide of interest is selected from the group consisting of a gene encoding a therapeutic protein, a suicide gene, a nucleotide encoding an antibody or fragment thereof, a nucleotide encoding a CRISPR/Cas system or portion(s) thereof, a nucleotide encoding antisense RNA, and a nucleotide encoding shRNA.
45. A composition comprising (a) the viral capsid of any one of aspects 33-36 or the viral vector of any one of aspects 37-44 and (b) a pharmaceutically acceptable carrier.
46. A method of delivering a nucleotide of interest to a target cell comprising contacting the target cell with the viral particle of any one of aspects 37-44 or the composition of aspect 45, and wherein the viral capsid comprises a targeting ligand that specifically binds a protein expressed on the surface the target cell.
47. The method of aspect 46, wherein the target cell is *in vitro.*
48. The method of aspect 46, wherein the target cell is *in vivo* in a subject.
49. The method of aspect 48, wherein the subject is a human.
50. The method of any one of aspects 46-49, wherein the target cell is a human target cell.
51. The method of aspect 50, wherein the target cell is a human liver cell, and wherein the targeting ligand binds human asialoglycoprotein receptor (ASGR1).
52. The method of aspect 50, wherein the target cell is a human neuronal cell, and wherein the targeting ligand binds GABA.
53. The method of aspect 50, wherein the target cell is a human T cell, and wherein the targeting ligand binds CD3, optionally CD3ε.
54. The method of aspect 50, wherein the targeting ligand binds PTPRN.
55. The method of aspect 50, wherein the target cell is a human hematopoietic cell, and wherein the targeting ligand binds CD34.
56. The method of aspect 50, wherein the target cell is a human kidney cell.
57. The method of aspect 50, wherein the target cell is a human cancer cell, and wherein the targeting ligand binds a tumor associated antigen.
58. The method of aspect 57, wherein the tumor antigen is E6, E7 or Her2
59. The method of aspect 50, wherein the targeting ligand binds CD20.
60. The method of aspect 50, wherein the targeting ligand binds human glucagon receptor.
61. The method of any one of aspects 46-50, wherein the targeting ligand specifically binds CD63.
62. The method of any one of aspects 46-50, wherein the targeting ligand specifically binds human ectonucleoside triphosphate diphosphohydrolase 3 (hENTPD3).
63. A method of providing a viral capsid protein with a scaffold and/or adaptor comprising
   (a) inserting a nucleic acid encoding a first member of a specific protein:protein binding pair, and optionally a linker, into a nucleic acid sequence encoding an viral capsid protein to form a nucleotide sequence encoding a genetically modified capsid protein comprising the first member of the specific binding pair, and optionally the linker, and
   (b) culturing a packaging cell in conditions sufficient for the production of viral particles, wherein the packaging cell comprises the nucleic acid.
64. A method of producing a viral particle comprising culturing a packaging cell in conditions sufficient for the production of viral particles, wherein the packaging cell comprises a nucleotide sequence encoding a genetically modified capsid protein comprising a first member of a specific protein:protein binding pair, and optionally an amino acid linker that links the first member to the capsid protein.
65. The method of aspect 63 or aspect 64, wherein the packaging cell further comprises a helper plasmid and/or a transfer plasmid comprising a nucleotide of interest.
66. The method of any one of aspects 63-65, wherein the packaging cell further comprises a plasmid encoding a reference mosaic capsid.
67. The method of any one of aspects 63-66, further comprising lysing the packaging cell and isolating adeno-associated viral vectors from the cell lysate.
68. The method of any one of aspects 63-67, further comprising
   a. clearing cell debris,
   b. treating the supernatant containing viral particles with nucleases,
   c. concentrating viral particles,
   d. purifying the viral particles, and
   e. any combination of a.-d.
69. The method of any one of aspects 63-68, wherein the nucleotide sequence encoding a genetically modified capsid protein further comprises a mutation of an amino acid at a position involved with the natural tropism of the capsid protein, wherein the mutation comprises an insertion of a heterologous peptide into the capsid protein, substitution of one or more amino acids of the capsid protein with a heterologous peptide, deletion of one or more amino acids of the capsid protein, or a combination thereof.
70. A viral particle made according to the method of any one of aspects 63-69.
71. A packaging cell for producing a viral particle comprising a plasmid encoding the recombinant viral capsid protein according to any one of aspects 1-32.
72. A recombinant vector encoding the recombinant viral capsid protein of any one of aspects 1-32.

## Claims

1. A recombinant viral vector comprising a nucleotide of interest encapsulated by a recombinant viral capsid comprising a recombinant viral capsid protein that is genetically modified to display a heterologous amino acid sequence comprising a first member of a protein:protein binding pair inserted into the viral capsid protein,
wherein the viral capsid protein is derived from a *cap* gene of an adeno-associated virus (AAV) that encodes an AAV VP1, VP2, and/or VP3 capsid protein;
wherein
(a) the first member of the protein:protein binding pair is a peptide tag and the viral capsid protein further comprises the second cognate member of the protein:protein binding pair, wherein the first member and second cognate member are bound by an isopeptide bond; and
(b) the second cognate member of the protein:protein binding pair is fused to a targeting ligand which is a binding moiety, wherein the binding moiety is an antibody or a portion thereof,
wherein the protein:protein binding pair is SpyTag:SpyCatcher, and wherein the viral capsid is a mosaic capsid.

2. The recombinant viral vector of claim 1, wherein:
(a) the first member is flanked by a first and/or second linker that link(s) the first member to the viral capsid protein, and wherein the first and/or second linker is each independently at least one amino acid in length, optionally wherein:
(i) the first and second linkers are not identical; or
(ii) the first and second linkers are identical and are 10 amino acids in length;
(b) the viral capsid protein further comprises a mutation at an amino acid position involved with binding of the viral capsid protein to its natural receptor, wherein the mutation comprises an insertion of a heterologous peptide into the capsid protein, substitution of one or more amino acids of the capsid protein with a heterologous peptide, deletion of one or more amino acids of the capsid protein, or a combination thereof; and/or
(c) the AAV is selected from the group consisting of AAV1, AAV2, AAV3, AAV4, AAV5, AAV6, AAV7, AAV8 and AAV9, optionally wherein the AAV is AAV2 or AAV9.

3. The recombinant viral vector of claim 1 or claim 2, wherein the amino acid sequence is less than 50 amino acids in length, and wherein the recombinant viral capsid protein exhibits reduced to abolished natural tropism.

4. The recombinant viral vector of any one of the preceding claims, wherein the antibody, or portion thereof, is fused to SpyCatcher,
optionally wherein the antibody, or portion thereof, is fused to a linker at the C-terminus, and the linker is fused to SpyCatcher at the linker's C-terminus, such as wherein the linker comprises a sequence set forth as SEQ ID NO:48 (GSGESG).

5. The recombinant viral vector of any one of the preceding claims, wherein the targeting ligand specifically binds a cell surface molecule, optionally wherein the cell surface molecule is:
(a) asialoglycoprotein 1 (ASGR1), or
(b) CD63.

6. The recombinant viral vector of any one of the preceding claims, wherein the recombinant viral capsid further comprises a reference viral capsid protein lacking any member of the specific binding pair, optionally wherein the recombinant viral capsid protein and the reference viral capsid protein each comprise a mutation of at least one residue involved with the binding of the viral particle with its natural ligand.

7. The recombinant viral vector of any one of the preceding claims, wherein the nucleotide of interest is:
(a) under the control of a promoter selected from the group consisting of a viral promoter, a bacterial promoter, a mammalian promoter, an avian promoter, a fish promoter, an insect promoter, and any combination thereof; or
(b) under the control of a human promoter.

8. The recombinant viral vector of any one of the preceding claims, wherein the nucleotide of interest is:
(a) a reporter gene, optionally wherein the reporter gene encodes green fluorescent protein, β-galactosidase (encoded lacZ gene), enhanced Green Fluorescent Protein (eGFP), MmGFP, blue fluorescent protein (BFP), enhanced blue fluorescent protein (eBFP), mPlum, mCherry, tdTomato, mStrawberry, J-Red, DsRed, mOrange, mKO, mCitrine, Venus, YPet, yellow fluorescent protein (YFP), enhanced yellow fluorescent protein (eYFP), Emerald, CyPet, cyan fluorescent protein (CFP), Cerulean, T-Sapphire, luciferase, alkaline phosphatase, or a combination thereof; or
(b) selected from the group consisting of a gene encoding a therapeutic protein, a suicide gene, a nucleotide encoding an antibody or fragment thereof, a nucleotide encoding a CRISPR/Cas system or portion(s) thereof, a nucleotide encoding antisense RNA, and a nucleotide encoding shRNA.

9. A composition comprising: (a) the viral vector of any one of claims 1-8; and (b) a pharmaceutically acceptable carrier.

10. The viral vector of any one of claims 1-8 or the composition of claim 9 for use in a method of therapy by delivering a nucleotide of interest to a target cell comprising contacting the target cell with said viral vector or said composition, wherein the viral capsid protein comprises a targeting ligand that specifically binds a protein expressed on the surface the target cell, wherein the target cell is *in vivo* in a subject, optionally wherein the subject is a human.

11. The viral vector or composition for use according to claim 10, wherein the target cell is a human target cell, optionally wherein:
(a) the target cell is a human liver cell, and wherein the targeting ligand binds human asialoglycoprotein receptor (ASGR1);
(b) the target cell is a human neuronal cell, and wherein the targeting ligand binds GABA;
(c) the target cell is a human T cell, and wherein the targeting ligand binds CD3, optionally CD3ε;
(d) the target cell is a human hematopoietic cell, and wherein the targeting ligand binds CD34;
(e) the target cell is a human kidney cell;
(f) the target cell is a human cancer cell, and wherein the targeting ligand binds a tumor associated antigen, optionally wherein the tumor antigen is E6, E7 or Her2; or
(g) the targeting ligand binds human glucagon receptor.

12. A method of delivering a nucleotide of interest to a target cell comprising contacting the target cell with the viral vector of any one of claims 1-8 or the composition of claim 9, wherein the viral capsid protein comprises a targeting ligand that specifically binds a protein expressed on the surface the target cell, wherein the target cell is *in vitro.*

13. The method according to claim 12, wherein the target cell is a human target cell, optionally wherein:
(a) the target cell is a human liver cell, and wherein the targeting ligand binds human asialoglycoprotein receptor (ASGR1);
(b) the target cell is a human neuronal cell, and wherein the targeting ligand binds GABA;
(c) the target cell is a human T cell, and wherein the targeting ligand binds CD3, optionally CD3ε;
(d) the target cell is a human hematopoietic cell, and wherein the targeting ligand binds CD34;
(e) the target cell is a human kidney cell;
(f) the target cell is a human cancer cell, and wherein the targeting ligand binds a tumor associated antigen, optionally wherein the tumor antigen is E6, E7 or Her2; or
(g) the targeting ligand binds human glucagon receptor.
